# EUROPEAN PATENT APPLICATION

(11) **EP 4 019 090 A1**
(43) Date of publication of application: **29.06.2022**
(21) Application number: 20216928.0
(22) Date of filing: 23.12.2020
(51) Int. Cl.: A61P 29/00, A61P 37/06, A61P 37/08, C07K 16/24, C07K 16/28, G01N 33/68, G01N 33/564

(54) **ANTIBODY VARIABLE DOMAINS THAT BIND IL-4R**

(71) Applicant: Numab Therapeutics AG, 8820 Wädenswil (CH)
(72) Inventor: The designation of the inventor has not yet been filed
(74) Representative: Virnekäs, Bernhard

(57) **Abstract**

The present invention relates to an antibody variable domain, which specifically binds to IL-4R, to multispecific antibodies comprising one or two of said antibody variable domains and at least one further binding domain that specifically binds to a target different from IL-4R . The present invention further relates to nucleic acid sequences encoding said antibody variable domain or said multispecific antibody, vector(s) comprising said nucleic acid sequences, host cell(s) comprising said nucleic acid sequences or said vector(s), and a method of producing said antibody variable domain or said multispecific antibody. Additionally, the present invention relates to pharmaceutical compositions comprising said multispecific antibody and to methods of use thereof.

## Description

### FIELD OF THE INVENTION

The present invention relates to an antibody variable domain, which specifically binds to IL-4R, to multispecific antibodies comprising one or two of said antibody variable domains and at least one further binding domain that specifically binds to a target different from IL-4R. The present invention further relates to nucleic acid sequences encoding said antibody variable domain or said multispecific antibody, vector(s) comprising said nucleic acid sequences, host cell(s) comprising said nucleic acid sequences or said vector(s), and a method of producing said antibody variable domain or said multispecific antibody. Additionally, the present invention relates to pharmaceutical compositions comprising said multispecific antibody and to methods of use thereof.

### BACKGROUND OF THE INVENTION

The interleukin-4 receptor IL-4Rα, herein also referred to as IL-4R or IL4R, is a type I receptor. It binds interleukin 4 (IL-4) either upon direct interaction with the common cytokine receptor gamma chain (γ_{c}) or by forming receptor complexes with type II receptors, such as in particular with the type II receptor IL-13Rα1. This IL-4R/IL-13Rα1 receptor complex can bind interleukin 4 (IL-4) as well as interleukin 13 (IL-13) to regulate IgE antibody production in B cells. IL-4 binding to IL-4R is further known to activate macrophages and to promote differentiation of type 2 helper T-cells (Th2-cells), leading to Th2-driven inflammation. IL-4 and/or IL-13 binding to IL-4Rα/γ_{c} and/or IL-4R/IL-13Rα1 leads to the activation of Janus kinase (JAK)1, JAK2, signal transducer and activator of transcription (STAT)1, STAT3, STAT6, and STAT dimerization, which in turn induces the transcription of specific genes.

IL-4R signaling has been shown to be associated with a number of human diseases, such as inflammatory and autoimmune disorders, including atopic dermatitis, prurigo nodularis, nasal polyposis, chronic urticaria, asthma and chronic obstructive pulmonary disease.

Several monoclonal antibodies, which reduce or block IL4R-mediated signaling by either binding the cytokines IL-4 and/or IL-13 or to the IL-4R, have been described in the prior art for the treatment of such diseases.

For example, dupilumab (Dupixent^{®}) developed by Regeneron Pharmaceuticals and Sanofi Genzyme, which antagonizes IL-4R by binding to the alpha subunit of IL-4R, has received approval from the United States Food and Drug Administration for moderate-to-severe atopic dermatitis in 2017 and for the treatment of chronic rhinosinusitis with nasal polyposis (CRSwNP). It has further been evaluated for treatment of persistent asthma in adults and adolescents. In 2018 dupilumab also received approval from the United States Food and Drug Administration for asthma.

Furthermore, WO 2014/039461 describes anti-IL4R antagonistic antibodies for use in the treatment of atopic dermatitis.

Atopic dermatitis (AD) is a chronic inflammatory skin disease characterized by intense pruritus (*i*. *e*. severe itch) and by scaly and dry eczematous lesions. Severe disease can be extremely disabling due to major psychological problems, significant sleep loss, and impaired quality of life, leading to high socioeconomic costs. AD often begins in childhood before age 5 and may persist into adulthood.

The pathophysiology of AD is influenced by a complex interplay between immunoglobulin E (IgE)-mediated sensitization, the immune system, and environmental factors. The primary skin defect may be an immunological disturbance that causes IgE-mediated sensitization, with epithelial-barrier dysfunction that is the consequence of both genetic mutations and local inflammation.

However, the anti-IL-4R therapies discussed above have significant limitations. Their efficacy is often limited and/or the response rates are low to moderate for many allergic, inflammatory and autoimmune disorders, even for those disorders that have been associated with an imbalanced IL-4R signaling. One explanation for this suboptimal performance of the presently available anti IL-4R therapies could be that imbalanced IL-4R signaling is not the sole causes of these diseases. Thus, in order to increase the response rates and/or efficacy of these therapies, other signaling pathways have to be addressed as well. Apart from this, many of these types of diseases are associated with disruptive symptoms that should be treated as well, such as for example itching. Anti-IL-4R therapies do not directly address itching. Itching, however, is a common symptom in skin-associated inflammatory and autoimmune disorders, such as in the case of AD. Itch-associated scratching generally promotes inflammation and worsening of the disease-related symptoms. Thus, there is a critical need for additional IL-4R-based treatment options for patients living with such allergic, inflammatory and autoimmune disorders.

More specifically, it is desirable to have a stable and potent anti-IL-4R antibody building block at hand that can be readily incorporated into multispecific antibody formats, which additionally interfere in other signaling pathways. Said anti-IL-4R building blocks should have a high potency in inhibiting IL-4- and/or IL-13-mediated signaling. Furthermore, said building blocks should have superior biophysical properties, such as in particular a high stability, in order to facilitate their efficient incorporation into multispecific antibodies being suitable for pharmaceutical develoment.

In particular, said anti-IL-4R building block, *i*. *e*. antibody binding domain, should exhibit the following minimum features:
- it should bind to human IL-4R with a monovalent dissociation constant (K_{D}) of 200 pM or less, as measured by surface plasmon resonance (SPR);
- it should be cross-reactive with *Macaca fascicularis* (Cynomolgus) IL-4R, in particular it should bind to Cynomolgus IL-4R with a monovalent K_{D} of 5 nM or less, as measured by surface plasmon resonance (SPR);
- it should have a melting temperature (Tₘ), determined by differential scanning fluorimetry (DSF), of 63°C or higher, in particular when formulated in 50 mM phosphate citrate buffer with 150 mM NaCl at pH 6.4.

Furthermore, it is desirable that said anti-IL-4R building block has the ability to neutralize the human IL-4 induced signaling with an IC₅₀ of 5 ng/ml or less and the human IL-13 induced signaling with an IC₅₀ of 10 ng/ml or less, as measured in a stat-6 reporter gene assay in HEK-Blue cells. Further desirable features are listed in item 7 below.

While strategies are known in the prior art how to identify anti-IL-4R antibody variable domains that fulfill one or two of the abovementioned criteria, the identification of such antibody variable domains that fulfill all of these criteria, let alone most or all of the criteria mentioned in item 7 below, is challenging and the outcome is unpredictable.

### SUMMARY OF THE INVENTION

It is an object of the present invention to provide novel antibody variable domains, which specifically bind IL-4R, that are able to efficiently reduce or eliminate IL-4- and IL-13-mediated activity and that are at the same time highly stable so that they can be readily incorporated into multispecific antibody formats.

The inventors have now surprisingly found that scFvs based on the monoclonal rabbit antibody clone 44-34-C10 are able to bind to human IL-4R as well as to cynomolgus IL-4R with high affinity and to potently block IL-4R signaling while exhibiting excellent stability. This antibody clone is one of a limited number of monoclonal rabbit antibody clones from a broad immunization campaign that have been identified to bind to IL-4R with high affinity. In particular, the scFvs derived from clone 44-34-C10 bind to human IL-4R with a dissociation constant (K_{D}) in the lower pM range and to cynomolgus IL-4R with a K_{D} of well below 1 nM, can neutralize the IL-4- and IL-13-induced signaling with an IC₅₀ of below 1.5 ng/ml and below 2.5 ng/ml, respectively, have a melting temperature (Tₘ), as determined by DSF, of 63 °C or higher and can be stored at a concentration of 10 mg/ml over a period of 4 weeks at 4°C without significant loss in protein content and monomeric content. This is particularly surprising in view of the fact that none of the scFvs produced from the other clones exhibit a good pharmacological activity and at the same time a high stability. Optimization of the pharmacological activity of these scFvs almost exclusively resulted in a worsening of the stability and *vice versa.*

Thus, the scFvs that are based on clone 44-34-C10 represent suitable building blocks that can readily be incorporated into multi-specific antibody formats such as Morrison formats.

Accordingly, in a first aspect, the present invention relates to an antibody variable domain, which specifically binds to IL-4R, comprising:
a) a VH sequence selected from SEQ ID NOs: 4, 5, 6, 7, 8 and 9, and
b) a VL sequence selected from SEQ ID NOs: 14, 15, 16, 17 and 18.

In a second aspect, the present invention relates to a multispecific antibody comprising:
a) one or two antibody variable domains as defined herein;
b) at least one binding domain, which specifically binds to a target different from IL-4R.

In a third aspect, the present invention relates to a nucleic acid sequence or two nucleic acid sequences encoding the antibody variable domain or the multispecific antibody of the resent invention.

In a fourth aspect, the present invention relates to a vector or two vectors comprising the nucleic acid sequence or the two nucleic acid sequences of the present invention.

In a fifth aspect, the present invention relates to a host cell or host cells comprising the vector or the two vectors of the present invention.

In a sixth aspect, the present invention relates to a method for producing the multispecific antibody of the present invention, comprising (i) providing the nucleic acid sequence or the two nucleic acid sequences of the present invention, or the vector or the two vectors of the present invention, expressing said nucleic acid sequence or said two nucleic acid sequences, or said vector or vectors, and collecting said multispecific antibody from the expression system, or (ii) providing a host cell or host cells of the present invention, culturing said host cell or said host cells; and collecting said multispecific antibody from the cell culture.

In a seventh aspect, the present invention relates to a pharmaceutical composition comprising the multispecific antibody of the present invention and a pharmaceutically acceptable carrier.

In an eighth aspect, the present invention relates to a multispecific antibody of the present invention for use as a medicament.

In a ninth aspect, the present invention relates to a multispecific antibody of the present invention for use in the treatment of a disease, particularly a human disease, more particularly a human disease selected from allergic, inflammatory and autoimmune diseases, particularly from inflammatory and autoimmune diseases.

In a tenth aspect, the present invention relates to a method for the treatment of a disease, particularly a human disease, more particularly a human disease selected from allergic, inflammatory and autoimmune diseases, particularly from inflammatory and autoimmune diseases, comprising the step of administering the multispecific antibody of the present invention to a patient in need thereof.

The aspects, advantageous features and preferred embodiments of the present invention summarized in the following items, respectively alone or in combination, further contribute to solving the object of the invention:
1. An antibody variable domain, which specifically binds to IL-4R, comprising:
   a) variable heavy chain (VH),
      wherein the variable heavy chain comprises, from N-terminus to C-terminus, the regions HFW1-HCDR1-HFW2-HCDR2-HFW3-HCDR3-HFW4, wherein each HFW designates a heavy chain framework region, and each HCDR designates a heavy chain complementarity-determining region, and wherein
      said HCDR1 is selected from SEQ ID NO: 1;
      said HCDR2 is selected from SEQ ID NO: 2; and
      said HCDR3 is selected from SEQ ID NO: 3;
   b) variable light chain (VL),
      wherein the variable light chain comprises, from N-terminus to C-terminus, the regions LFW1-LCDR1-LFW2-LCDR2-LFW3-LCDR3-LFW4, wherein each LFW designates a light chain framework region, and each LCDR designates a light chain complementarity-determining region, and wherein
      said LCDR1 is selected from SEQ ID NO: 10;
      said LCDR2 is selected from SEQ ID NO: 11 or 12; and
      said LCDR3 is selected from SEQ ID NO: 13.
2. The antibody variable domain of item 1, wherein said variable heavy chain is a VH3 chain, and/or wherein said variable light chain is a Vκ1 light chain.
3. The antibody variable domain of item 1, wherein
   - said variable heavy chain is a VH3 chain,
   - the LFW1, LFW2 and LFW3 are of the Vκ1 light chain subtype, and
   - the LFW4 has a human Vλ sequence selected from SEQ ID NOs: 22 to 29.
4. The antibody variable domain of any one of the preceding items, wherein said antibody variable domain is selected from a Fab, an Fv, an scFv, dsFv, and an scAB, preferably from a Fab, an Fv, an scFv and a dsFv, in particular from a Fab, an scFv and a dsFv.
5. The antibody variable domain of any one of the preceding items, wherein said antibody variable domain comprises:
   a) a VH sequence at least 90, 91, 92, 93, 94, 95, 96, 97, 98 or 99 percent identical to any one of the amino acid sequences selected from SEQ ID NOs: 4, 5, 6, 7, 8 and 9; and
   b) a VL sequence at least 90, 91, 92, 93, 94, 95, 96, 97, 98 or 99 percent identical to any one of the amino acid sequences selected from SEQ ID NOs: 14, 15, 16, 17 and 18.
6. The antibody variable domain of any one of the items 1 to 5, wherein said antibody variable domain blocks the binding of IL4 and IL13 to IL4R.
7. The antibody variable domain of any one of the items 1 to 5, wherein said antibody variable domain, when being in an scFv format, exhibits at least two of the following features a. to f.:
   a. binds to human IL-4R with a monovalent dissociation constant (K_{D}) of 0.1 to 200 pM, particularly with a K_{D} of 0.1 to 100 pM, particularly of 0.1 to 50 pM, as measured by surface plasmon resonance (SPR);
   b. is cross-reactive with *Macaca fascicularis* (Cynomolgus) IL4R, in particular binds to Cynomolgus IL-4R with a monovalent K_{D} of 1 pM to 5 nM, particularly of 2 pM to 3 nM, particularly of 3 pM to 2 nM, as measured by SPR;
   c. is cross-reactive with *Callithrix jacchus* (Marmoset) IL-4R, in particular binds to Marmoset IL-4R with a monovalent K_{D} of 1 pM to 5 nM, particularly of 2 pM to 3 nM, particularly of 3 pM to 2 nM, as measured by SPR;
   d. neutralizes the human IL-4-induced signaling with an IC₅₀ of 0.01 to 5 ng/ml, particularly with an IC₅₀ of 0.01 to 3 ng/ml, particularly with an IC₅₀ of 0.01 to 1.5 ng/ml, as measured in a stat6 reporter gene assay in HEK-Blue cells;
   e. neutralizes the human IL-13-induced signaling with an IC₅₀ of 0.01 to 10 ng/ml, particularly with an IC₅₀ of 0.01 to 5 ng/ml, particularly with an IC₅₀ of 0.01 to 2.5 ng/ml, as measured in a stat-6 reporter gene assay in HEK-Blue cells;
   f. inhibits the binding of human IL-4 to human IL-4R with an IC₅₀ of 0.01 to 10 ng/ml, particularly with an IC₅₀ of 0.01 to 5 ng/ml, particularly with an IC₅₀ of 0.01 to 2 ng/ml, as measured in a competition ELISA;
   and wherein said antibody variable domain, when being in an scFv format, further exhibits at least one of the following features g. to j.:
   g. has a melting temperature (Tₘ), determined by differential scanning fluorimetry (DSF), of at least 63°C, particularly of at least 65°C, particularly of at least 67°C, particularly of at least 69°C, particularly of at least 70°C, particularly of at least 71°C, particularly of at least 72°C, in particular wherein said scFv is formulated in 50 mM phosphate citrate buffer with 150 mM NaCl at pH 6.4;
   h. has a loss in monomer content, after storage for at least four weeks at 4°C of less than 5 %, when said scFv is at a starting concentration of 10 mg/ml, and in particular wherein said scFv is formulated in 50 mM phosphate citrate buffer with 150 mM NaCl at pH 6.4;
   i. has a loss in protein content, after storage for at least four weeks at 40°C of less than 5 %, particularly of less than 4%, particularly of less than 3%, particularly of less than 2%, particularly of less than 1%, when said scFv is at a starting concentration of 10 mg/ml, and in particular wherein said scFv is formulated in 50 mM phosphate citrate buffer with 150 mM NaCl at pH 6.4;
   j. has a loss in monomer content after 5 freeze-thawing cycles of less than 2 %, particularly of less than 1%, when said scFv is at a starting concentration of 10 mg/ml, and in particular wherein said scFv is formulated in 50 mM phosphate citrate buffer with 150 mM NaCl at pH 6.4.
8. The antibody variable domain of item 7, wherein said antibody variable domain, when being in an scFv format, exhibits at least the features a., b. and g.
9. The antibody variable domain of item 7, wherein said antibody variable domain, when being in an scFv format, exhibits at least the features a., b., d., e., g. and h., in particular at least the features a., b., d., e., f., g., h. and i.
10. The antibody variable domain of item 7, wherein said antibody variable domain, when being in an scFv format, exhibits all features a. to j.
11. The antibody variable domain of any one of the preceding items, wherein said antibody variable domain comprises:
   a) a VH sequence selected from SEQ ID NOs: 4, 5, 6, 7, 8 and 9, and
   b) a VL sequence selected from SEQ ID NOs: 14, 15, 16, 17 and 18.
12. The antibody variable domain of any one of the preceding items, wherein said antibody variable domain comprises:
   a) a VH sequence of SEQ ID NO: 4 and a VL sequence of SEQ ID NO: 14; or
   b) a VH sequence of SEQ ID NO: 4 and a VL sequence of SEQ ID NO: 17; or
   c) a VH sequence of SEQ ID NO: 4 and a VL sequence of SEQ ID NO: 18; or
   d) a VH sequence of SEQ ID NO: 5 and a VL sequence of SEQ ID NO: 15; or
   e) a VH sequence of SEQ ID NO: 6 and a VL sequence of SEQ ID NO: 16; or
   f) a VH sequence of SEQ ID NO: 7 and a VL sequence of SEQ ID NO: 14; or
   g) a VH sequence of SEQ ID NO: 8 and a VL sequence of SEQ ID NO: 18; or
   h) a VH sequence of SEQ ID NO: 9 and a VL sequence of SEQ ID NO: 14.
13. The antibody variable domain of any one of the preceding items, which is selected from the scFv antibodies of SEQ ID NOs: 33 to 39.
14. A multispecific antibody comprising:
   a) one or two antibody variable domains as defined in any one of items 1 to 13;
   b) at least one binding domain, which specifically binds to a target different from IL-4R.
15. The multispecific antibody of item 14, wherein the multispecific antibody does not comprise an immunoglobulin Fc region.
16. The multispecific antibody of item 15, wherein the multispecific antibody is in a format selected from the group consisting of: a tandem scDb (Tandab), a linear dimeric scDb (LD-scDb), a circular dimeric scDb (CD-scDb), a tandem tri-scFv, a tribody (Fab-(scFv)₂), a Fab-Fv₂, a triabody, an scDb-scFv, a tetrabody, a di-diabody, a tandem-di-scFv and a MATCH.
17. The multispecific antibody of item 15, wherein said antibody does not comprise CH1 and/or CL regions.
18. The multispecific antibody of item 17, wherein said antibody is in a scDb-scFv, a triabody, a tetrabody or a MATCH format, in particular wherein said multispecific antibody is in a MATCH or scDb-scFv format, more particularly wherein said multispecific antibody is in a MATCH format, more particularly a MATCH3 or a MATCH4 format.
19. The multispecific antibody of item 14, wherein the multispecific antibody comprises an immunoglobulin Fc region.
20. The multispecific antibody of item 19, wherein the immunoglobulin Fc region is selected from an IgG subclass, particularly from IgG subclasses IgG1 and IgG4, particularly from IgG4.
21. The multispecific antibody of item 20, wherein the format of said multispecific antibody is selected from bivalent bispecific IgG formats, trivalent bispecific IgG formats and tetravalent bispecific IgG formats;
   more particularly wherein the format of said multispecific antibody is selected from KiH-based IgGs; DVD-Ig; CODV-IgG and Morrison (IgG CH₃-scFv fusion (Morrison-H) or IgG CL-scFv fusion (Morrison-L)), even more particularly from DVD-Ig and Morrison (IgG CH₃-scFv fusion (Morrison-H) or IgG CL-scFv fusion (Morrison-L)).
22. The multispecific antibody of item 21, wherein the format of said multispecific antibody is selected from a Morrison-H and Morrison-L format.
23. The multispecific antibody of any one of items 14 to 22, wherein said multispecific antibody comprises two antibody variable domains as defined in any one of items 1 to 13 and two binding domains, which specifically bind to a second target different from IL-4R.
24. A nucleic acid sequence or two nucleic acid sequences encoding the antibody variable domain of any one of items 1 to 13 or the multispecific antibody of any one of items 14 to 23.
25. A vector or two vectors comprising the nucleic acid sequence or the two nucleic acid sequences of item 24.
26. A host cell or host cells comprising the vector or the two vectors of item 25.
27. A method for producing the antibody variable domain of items 1 to 13 or the multispecific antibody of items 14 to 23, comprising (i) providing the nucleic acid sequence or the two nucleic acid sequences of item 24, or the vector or the two vectors of item 25, expressing said nucleic acid sequence or said two nucleic acid sequences, or said vector or said two vectors, and collecting said antibody variable domain or said multispecific antibody from the expression system, or (ii) providing a host cell or host cells according to item 26, culturing said host cell or said host cells; and collecting said antibody variable domain or said multispecific antibody from the cell culture.
28. A pharmaceutical composition comprising the multispecific antibody of any one of items 14 to 23 and a pharmaceutically acceptable carrier.
29. The multispecific antibody of any one of items 14 to 23 for use as a medicament.
30. The multispecific antibody of any one of items 14 to 23 for use in the treatment of a disease, particularly a human disease, more particularly a human disease selected from allergic, inflammatory and autoimmune diseases, particularly from inflammatory and autoimmune diseases.
31. The multispecific antibody of item 30, wherein said disease is selected from atopic dermatitis, acute allergic contact dermatitis, chronic spontaneous urticaria, bullous pemphigoid, alopecia areata, dermatomyositis, prurigo nodularis, psoriasis and atopic asthma; particularly wherein said disease is atopic dermatitis.
32. A method for the treatment of a disease, particularly a human disease, more particularly a human disease selected from allergic, inflammatory and autoimmune diseases, particularly from inflammatory and autoimmune diseases, comprising the step of administering the multispecific antibody of any one of items 14 to 23 to a patient in need thereof.
33. The method of item 32, wherein said disease is selected from atopic dermatitis, acute allergic contact dermatitis, chronic spontaneous urticaria, bullous pemphigoid, alopecia areata, dermatomyositis, prurigo nodularis, psoriasis and atopic asthma; particularly wherein said disease is atopic dermatitis.

### BRIEF DESCRIPTION OF THE DRAWINGS

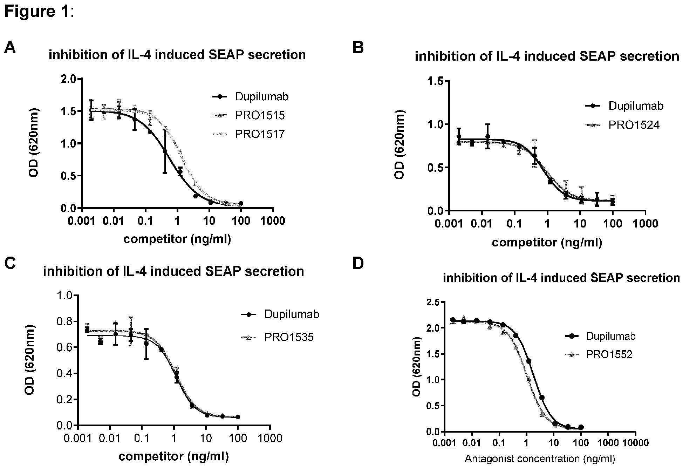

**FIG. 1** shows the potency of the best ranking scFvs to neutralize IL-4-induced signaling in the stat-6 reporter gene assay. In comparison to dupilumab, the best ranking scFvs (A) PRO1515 (44-32-F04 sc01) and PRO1517 (44-34-C10 sc01), (B) PRO1524 (44-03-A03-sc04), (C) PRO1535 (44-34-C10 sc04) and (D) PRO1552 (44-18-C11-sc08) showed IC₅₀ values that are comparable to dupilumab for neutralization of IL-4-induced signaling.

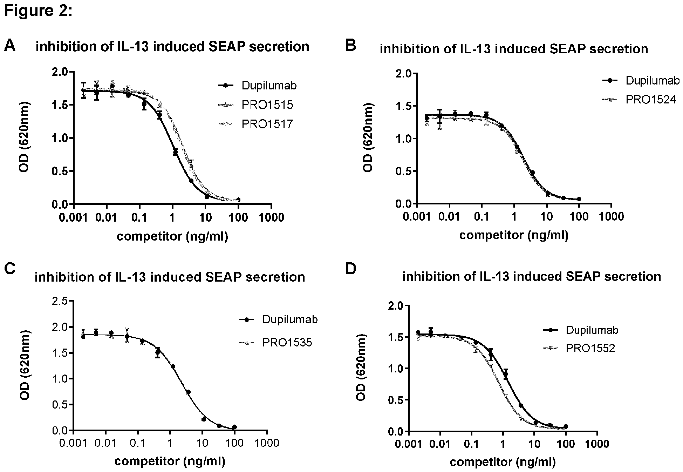

**FIG. 2** shows the potency of the best ranking scFvs to neutralize IL-13-induced signaling in the stat-6 reporter gene assay. In comparison to dupilumab, the best ranking scFvs (A) PRO1515 (44-32-F04 sc01) and PRO1517 (44-34-C10 sc01), (B) PRO1524 (44-03-A03-sc04), (C) PRO1535 (44-34-C10 sc04) and (D) PRO1552 (44-18-C11-sc08) showed IC₅₀ values that are comparable to dupilumab for neutralization of IL-13-induced signaling.

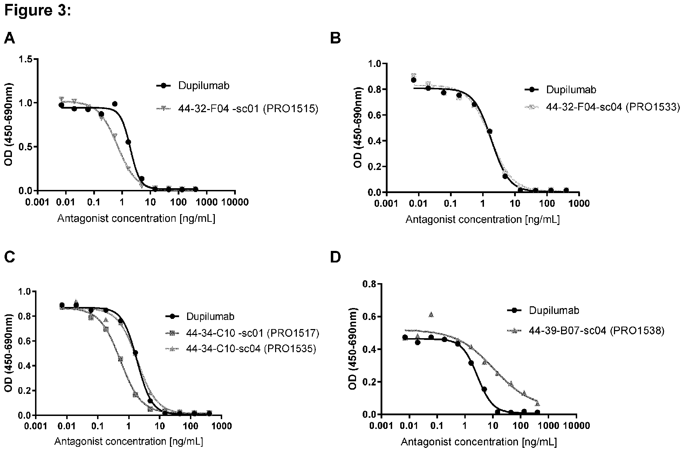

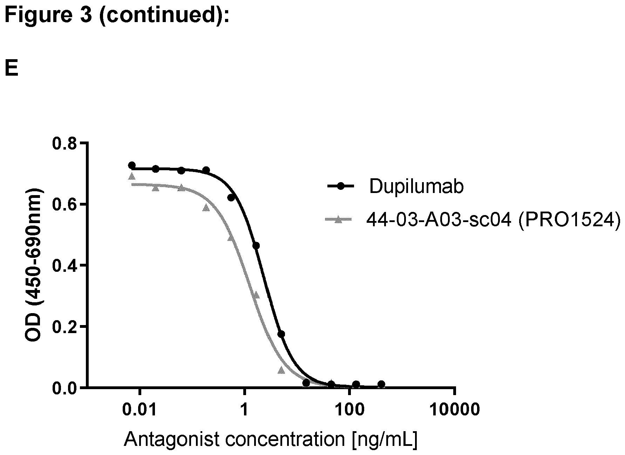

**FIG. 3** shows the potencies of the scFvs (A) PRO1515, (B) PRO1533, (C) PRO1517 and PRO1535 and (D) PRO1538 to inhibit the interaction between IL-4 and IL-4R. PRO1515 (44-32-F04 sc01), PRO1517 (44-34-C10 sc01), PRO1533 (44-32-F04 sc04) and PRO1535 (44-34-C10 sc04) showed IC₅₀ values that are comparable to dupilumab.

### DETAILED DESCRIPTION OF THE INVENTION

Known therapeutic anti-IL-4R antibodies are often limited by a lack of efficacy and by low to moderate response rates in patients suffering from allergic, inflammatory and autoimmune disorders, even in cases where these disorders have been associated with an imbalanced IL-4R signaling. These therapies also do not directly address the disruptive symptoms that are often associated with these diseases, such as for example itching. Thus, there is a critical need for additional IL-4R-based treatment options for patients living with said allergic, inflammatory and autoimmune disorders.

The present invention provides novel anti-IL-4R antibody variable domains comprising specific VL and VH sequences. Said variable domains are based on the monoclonal rabbit antibody clone 44-34-C10. This clone was selected from a limited number of monoclonal rabbit antibodies that were identified in a broad immunization campaign and were found to bind to IL-4R with high affinity. The scFvs derived from said monoclonal rabbit antibody clone 44-34-C10 bind to human IL-4R with a dissociation constant (K_{D}) in the lower pM range and to cynomolgus IL-4R with a K_{D} of well below 1 nM, can neutralize the IL-4- and IL-13-induced signaling with an IC₅₀ of below 1.5 ng/ml and below 2.5 ng/ml, respectively, have a melting temperature (Tₘ), as determined by DSF, of 63 °C or higher and can be stored at a concentration of 10 mg/ml over a period of 4 weeks at 4°C without significant loss in protein content and monomeric content.

To the best knowledge of the inventors, there exist no anti-IL-4R antibody variable domains in the prior art that have such advantageous properties.

The antibody variable domain of the present invention could be successfully incorporated in a multispecific antibody format that additionally targets IL-31. These anti-IL-4R x IL-31 multispecific antibodies are able to bind to IL-4R with high affinity and to potently inhibit IL-4R-mediated signaling, while exhibiting very advantageous biophysical properties, in particular an outstanding formulation and storage stability at antibody concentrations well above 100 mg/ml. This demonstrates that the antibody variable domains of the present invention also provide advantageous biological and biophysical properties when incorporated in multispecific antibody formats.

Unless defined otherwise, all technical and scientific terms used herein have the same meaning as commonly understood by those of ordinary skill in the art to which this invention pertains.

The terms "comprising" and "including" are used herein in their open-ended and non-limiting sense unless otherwise noted. With respect to such latter embodiments, the term "comprising" thus includes the narrower term "consisting of".

The terms "a" and "an" and "the" and similar references in the context of describing the invention (especially in the context of the following claims) are to be construed to cover both the singular and the plural, unless otherwise indicated herein or clearly contradicted by context. For example, the term "a cell" includes a plurality of cells, including mixtures thereof. Where the plural form is used for compounds, salts, and the like, this is taken to mean also a single compound, salt, or the like.

In one aspect, the present invention relates to an antibody variable domain, which specifically binds to IL-4R, comprising:
a) a VH sequence selected from SEQ ID NOs: 4, 5, 6, 7, 8 and 9, and
b) a VL sequence selected from SEQ ID NOs: 14, 15, 16, 17 and 18.

The term "antibody" and the like, as used herein, includes whole antibodies or single chains thereof; and any antigen-binding variable domain (*i*. *e*., "antigen-binding portion") or single chains thereof; and molecules comprising antibody CDRs, VH regions or VL regions (including without limitation multispecific antibodies). A naturally occurring "whole antibody" is a glycoprotein comprising at least two heavy (H) chains and two light (L) chains inter-connected by disulfide bonds. Each heavy chain is comprised of a heavy chain variable region (abbreviated herein as VH) and a heavy chain constant region. The heavy chain constant region is comprised of three domains, CH1, CH2 and CH3. Each light chain is comprised of a light chain variable region (abbreviated herein as VL) and a light chain constant region. The light chain constant region is comprised of one domain, CL. The VH and VL regions can be further subdivided into regions of hypervariability, termed complementarity determining regions (CDRs), flanked by regions that are more conserved, termed framework regions (FRs). Each VH and VL is composed of three CDRs and four FRs arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e. g., effector cells) and the first component (Clq) of the classical complement system.

The term "antibody variable domain", as used herein, refers to one or more parts of an intact antibody that have the ability to specifically bind to a given antigen (e. g., IL-4R). This can be any antigen-binding fragment (*i*. *e*., "antigen-binding portion") of an intact antibody or single chains thereof; and molecules comprising antibody CDRs, VH regions or VL regions. Specifically, in case of the multispecific antibodies of the present invention, the term "antibody variable domain", as used herein, refers to a Fab fragment, *i*. *e*. a monovalent fragment consisting of the VL, VH, CL and CH1 domains; an Fv fragment consisting of the VL and VH domains of a single arm of an antibody (Fv); a disulfide stabilized Fv fragment (dsFv); a single chain Fv fragment (scFv); and a single chain Fv fragment having an additional light chain constant domain (C_{L}) fused to it (scAB). Preferably, the antibody variable domain of the present invention is selected from a Fab fragment, an Fv fragment, a disulfide stabilized Fv fragment (dsFv) and an scFv fragment. More preferably, the antibody variable domain of the present invention is selected from a Fab fragment, a disulfide stabilized Fv fragment (dsFv) and an scFv fragment. In particular embodiments, the antibody variable domain of the present invention is a Fab fragment. In other particular embodiments, the antibody variable domain of the present invention is a single-chain Fv fragment (scFv). In other particular embodiments, the VL and VH domains of the scFv fragment are stabilized by an interdomain disulfide bond, in particular said VH domain comprises a single cysteine residue in position 51 (AHo numbering) and said VL domain comprises a single cysteine residue in position 141 (AHo numbering).

The term "Complementarity Determining Regions" ("CDRs") refers to amino acid sequences with boundaries determined using any of a number of well-known schemes, including those described by Kabat et al. (1991), "Sequences of Proteins of Immunological Interest," 5th Ed. Public Health Service, National Institutes of Health, Bethesda, MD ("Kabat" numbering scheme); Al-Lazikani et al., (1997) JMB 273, 927-948 ("Chothia" numbering scheme); ImMunoGenTics (IMGT) numbering (Lefranc, M.-P., The Immunologist, 7, 132-136 (1999); Lefranc, M.-P. et al., Dev. Comp. Immunol., 27, 55-77 (2003)) ("IMGT" numbering scheme); and the numbering scheme described in Honegger & Pluckthun, J. Mol. Biol. 309 (2001) 657-670 ("AHo" numbering). For example, for classic formats, under Kabat, the CDR amino acid residues in the heavy chain variable domain (VH) are numbered 31-35 (HCDR1), 50-65 (HCDR2), and 95-102 (HCDR3); and the CDR amino acid residues in the light chain variable domain (VL) are numbered 24-34 (LCDR1), 50-56 (LCDR2), and 89-97 (LCDR3). Under Chothia the CDR amino acids in the VH are numbered 26-32 (HCDR1), 52-56 (HCDR2), and 95-102 (HCDR3); and the amino acid residues in VL are numbered 24-34 (LCDR1), 50-56 (LCDR2), and 89-97 (LCDR3). By combining the CDR definitions of both Kabat and Chothia, the CDRs consist of amino acid residues 26-35 (HCDR1), 50-65 (HCDR2), and 95-102 (HCDR3) in human VH and amino acid residues 24-34 (LCDR1), 50-56 (LCDR2), and 89-97 (LCDR3) in human VL. Under IMGT the CDR amino acid residues in the VH are numbered approximately 26-35 (HCDR1), 51-57 (HCDR2) and 93-102 (HCDR3), and the CDR amino acid residues in the VL are numbered approximately 27-32 (LCDR1), 50-52 (LCDR2), and 89-97 (LCDR3) (numbering according to "Kabat"). Under IMGT, the CDRs of an antibody can be determined using the program IMGT/DomainGap Align.

In the context of the present invention, the numbering system suggested by Honegger & Plückthun ("AHo") is used (Honegger & Pluckthun, J. Mol. Biol. 309 (2001) 657-670), unless specifically mentioned otherwise. In particular, the following residues are defined as CDRs according to AHo numbering scheme: LCDR1 (also referred to as CDR-L1): L24-L42; LCDR2 (also referred to as CDR-L2): L58-L72; LCDR3 (also referred to as CDR-L3): L107-L138; HCDR1 (also referred to as CDR-H1): H27-H42; HCDR2 (also referred to as CDR-H2): H57-H76; HCDR3 (also referred to as CDR-H3): H108-H138. For the sake of clarity, the numbering system according to Honegger & Plückthun takes the length diversity into account that is found in naturally occurring antibodies, both in the different VH and VL subfamilies and, in particular, in the CDRs, and provides for gaps in the sequences. Thus, in a given antibody variable domain usually not all positions 1 to 149 will be occupied by an amino acid residue.

The term "binding specificity" as used herein refers to the ability of an individual antibody to react with one antigenic determinant and not with a different antigenic determinant. As used herein, the term "specifically binds to" or is "specific for" refers to measurable and reproducible interactions such as binding between a target and an antibody, which is determinative of the presence of the target in the presence of a heterogeneous population of molecules including biological molecules. For example, an antibody that specifically binds to a target (which can be an epitope) is an antibody that binds this target with greater affinity, avidity, more readily, and/or with greater duration than it binds to other targets. In its most general form (and when no defined reference is mentioned), "specific binding" is referring to the ability of the antibody to discriminate between the target of interest and an unrelated molecule, as determined, for example, in accordance with specificity assay methods known in the art. Such methods comprise, but are not limited to Western blots, ELISA, RIA, ECL, IRMA, SPR (Surface plasmon resonance) tests and peptide scans. For example, a standard ELISA assay can be carried out. The scoring may be carried out by standard color development (e. g. secondary antibody with horseradish peroxide and tetramethyl benzidine with hydrogen peroxide). The reaction in certain wells is scored by the optical density, for example, at 450 nm. Typical background (= negative reaction) may be about 0.1 OD; typical positive reaction may be about 1 OD. This means the ratio between a positive and a negative score can be 10-fold or higher. In a further example, an SPR assay can be carried out, wherein at least 10-fold, particularly at least 100-fold difference between a background and signal indicates on specific binding. Typically, determination of binding specificity is performed by using not a single reference molecule, but a set of about three to five unrelated molecules, such as milk powder, transferrin or the like.

In a further aspect, the present invention relates to a multispecific antibody comprising:
a) one or two antibody variable domains as defined herein;
b) at least one binding domain, which specifically binds to a target different from IL-4R.

In particular embodiments, the multispecific antibodies of the invention do not comprise an immunoglobulin Fc region.

In these particular embodiments, the multispecific antibody is preferably in a format selected from the group consisting of: a tandem scDb (Tandab), a linear dimeric scDb (LD-scDb), a circular dimeric scDb (CD-scDb), a tandem tri-scFv, a tribody (Fab-(scFv)₂), a Fab-Fv₂, a triabody, an scDb-scFv, a tetrabody, a di-diabody, a tandem-di-scFv and a MATCH (described in WO 2016/0202457; Egan T., et al., MABS 9 (2017) 68-84). In particular, the multispecific antibody of the invention is in a MATCH format. More particularly, the multispecific antibody of the invention is in a MATCH3 or a MATCH4 format.

The term "immunoglobulin Fc region" or "Fc region", as used herein, is used to define a C-terminal region of an immunoglobulin heavy chain, *i*. *e*. the CH2 and CH3 domains of the heavy chain constant regions. The term "Fc region" includes native-sequence Fc regions and variant Fc regions, *i*. *e*. Fc regions that are engineered to exhibit certain desired properties, such as for example altered Fc receptor binding function and/or reduced or suppressed Fab arm exchange. An example of such an engineered Fc region is the knob-into-hole (KiH) technology (see for example Ridgway et al., Protein Eng. 9:617-21 (1996) and Spiess et al., J Biol Chem. 288(37):26583-93 (2013)). Native-sequence Fc regions include human IgG1, IgG2 (IgG2A, IgG2B), IgG3 and IgG4. "Fc receptor" or "FcR" describes a receptor that binds to the Fc region of an antibody. Particularly, the FcR is a native sequence human FcR, which binds an IgG antibody (a gamma receptor) and includes receptors of the FcγRI, FcγRII, and FcγRIII subclasses, including allelic variants and alternatively spliced forms of these receptors, FcγRII receptors including FcγRIIA (an "activating receptor") and FcγRI IB (an "inhibiting receptor"), which have similar amino acid sequences that differ primarily in the cytoplasmic domains thereof. Activating receptor FcyRIIA contains an immunoreceptor tyrosine-based activation motif (ITAM) in its cytoplasmic domain. Inhibiting receptor FcγRIIB contains an immunoreceptor tyrosine-based inhibition motif (ITIM) in its cytoplasmic domain, (see M. Daeron, Annu. Rev. Immunol. 5:203-234 (1997). FcRs are reviewed in Ravetch and Kinet, Annu. Rev. Immunol. 9: 457-92 (1991); Capet et al, Immunomethods 4: 25-34 (1994); and de Haas et al, J. Lab. Clin. Med. 126: 330-41 (1995). Other FcRs, including those to be identified in the future, are encompassed by the term "FcR" herein. The term "Fc receptor" or "FcR" also includes the neonatal receptor, FcRn, which is responsible for the transfer of maternal IgGs to the fetus. Guyer et al., J. Immunol. 117: 587 (1976) and Kim et al., J. Immunol. 24: 249 (1994). Methods of measuring binding to FcRn are known (see, *e. g*., Ghetie and Ward, Immunol. Today 18: (12): 592-8 (1997); Ghetie et al., Nature Biotechnology 15 (7): 637-40 (1997); Hinton et al., J. Biol. Chem. TJI (8): 6213-6 (2004); WO 2004/92219 (Hinton et al). Binding to FcRn *in vivo* and serum half-life of human FcRn high-affinity binding polypeptides can be assayed, *e. g*., in transgenic mice or transfected human cell lines expressing human FcRn, or in primates to which the polypeptides having a variant Fc region are administered. WO 2004/42072 (Presta) describes antibody variants which improved or diminished binding to FcRs. See also, *e. g*., Shields et al., J. Biol. Chem. 9(2): 6591-6604 (2001).

In other particular embodiments, the multispecific antibodies of the invention comprise an immunoglobulin Fc region.

In further particular embodiments, the multispecific antibodies of the invention comprise an IgG region.

The term "IgG region", as used herein, refers to the heavy and light chain of an immunoglobulin G, *i*. *e*. the Fc region, as defined above, and the Fab region, consisting of the VL, VH, CL and CH1 domains. The term "IgG region" includes native-sequence IgG regions, such as human IgG1, IgG2 (IgG2A, IgG2B), IgG3 and IgG4, as well as engineered IgG regions, which exhibit certain desired properties, as for example the properties defined above for the Fc region.

In particular embodiments, the multispecific antibodies of the invention comprise an IgG region, wherein the IgG region is selected from the IgG subclasses IgG1 and IgG4, in particular from IgG4.

The terms "binding domain", "antigen-binding fragment thereof", "antigen-binding portion" of an antibody, and the like, as used herein, refer to one or more parts of an intact antibody that have the ability to specifically bind to a given antigen. Antigen-binding functions of an antibody can be performed by fragments of an intact antibody. Specifically, in case of the multispecific antibodies of the present invention, the terms "binding domain", "antigen-binding fragment thereof", "antigen-binding portion", and the like, as used herein, refer to a Fab fragment, *i*. *e*. a monovalent fragment consisting of the VL, VH, CL and CH1 domains; an Fv fragment consisting of the VL and VH domains of a single arm of an antibody; a disulfide stabilized Fv fragment (dsFv); and a single chain Fv fragment (scFv). Preferably, the binding domains of the multispecific antibodies of the present invention are independently of each other selected from a Fab fragment, an Fv fragment, an scFv fragment and a single-chain Fv fragment (scFv). In particular embodiments, the binding domains of the antibodies of the present invention are independently of each other selected from a Fab fragment and a single-chain Fv fragment (scFv). In other particular embodiments, the VL and VH domains of the scFv fragment are stabilized by an interdomain disulfide bond, in particular said VH domain comprises a single cysteine residue in position 51 (AHo numbering) and said VL domain comprises a single cysteine residue in position 141 (AHo numbering).

Suitably, the antibody variable domain of the invention is an isolated variable domain. Likewise, the multispecific antibodies of the invention are isolated antibodies. The term "isolated variable domain" or "isolated antibody", as used herein, refers to an variable domain or an antibody that is substantially free of other variable domains or other antibodies having different antigenic specificities (*e. g*., an isolated antibody variable domain that specifically binds IL-4R is substantially free of antibody variable domains that specifically bind antigens other than IL-4R). Moreover, an isolated antibody variable domain or isolated antibody may be substantially free of other cellular material and/or chemicals.

Suitably, the antibody variable domains and multispecific antibodies of the invention are monoclonal antiboy variable domains and antibodies. The term "monoclonal antibody variable domains" or "monoclonal antibody" as used herein refers to variable domains or antibodies that have substantially identical amino acid sequences or are derived from the same genetic source. A monoclonal variable domain or antibody displays a binding specificity and affinity for a particular epitope, or binding specificities and affinities for specific epitopes.

The antibody variable domains and multispecific antibodies of the invention include, but are not limited to, chimeric, human and humanized antibody variable domains and antibodies.

The term "chimeric antibody" or "chimeric antibody variable domain", as used herein, refers to an antibody molecule or antibody variable domain in which (a) the constant region, or a portion thereof, is altered, replaced or exchanged so that the antigen-binding site (variable region) is linked to a constant region of a different or altered class, effector function and/or species; or (b) the variable region, or a portion thereof, is altered, replaced or exchanged with a variable region having a different or altered antigen specificity. For example, a mouse antibody can be modified by replacing its constant region with the constant region from a human immunoglobulin. Due to the replacement with a human constant region, the chimeric antibody can retain its specificity in recognizing the antigen while having reduced antigenicity in human as compared to the original mouse antibody.

The term "human antibody" or "human antibody variable domain" , as used herein, is intended to include antibodies or antibody variable domains having variable regions in which both the framework and CDR regions are derived from sequences of human origin. Furthermore, if the antibody or antibody variable domain contains a constant region, the constant region also is derived from such human sequences, e. g., human germline sequences, or mutated versions of human germline sequences. The human antibodies and antibody variable domains of the invention may include amino acid residues not encoded by human sequences (*e. g*., mutations introduced by random or site-specific mutagenesis *in vitro* or by somatic mutation *in vivo*)*.* This definition of a human antibody or antibody variable domain specifically excludes a humanized antibody or antibody variable domain comprising non-human antigen-binding residues. Human antibodies and antibody variable domains can be produced using various techniques known in the art, including phage-display libraries (Hoogenboom and Winter, J. Mol. Biol, 227:381 (1992); Marks et al, J. Mol. Biol, 222:581 (1991)). Also available for the preparation of human monoclonal antibodies and human monoclonal antibody variable domains are methods described in Cole et al, Monoclonal Antibodies and Cancer Therapy, Alan R. Liss, p. 77 (1985); Boemer et al, J. Immunol, 147(I):86-95 (1991). See also van Dijk and van de Winkel, Curr. Opin. Pharmacol, 5: 368-74 (2001). Human antibodies and human antibody variable domains can be prepared by administering the antigen to a transgenic animal that has been modified to produce such antibodies and antibody variable domains in response to antigenic challenge, but whose endogenous loci have been disabled, *e. g*., immunized xenomice (see, *e. g*., U.S. Pat. Nos. 6,075,181 and 6,150,584 regarding XENOMOUSE^{™} technology). See also, for example, Li et al, Proc. Natl. Acad. Sci. USA, 103:3557- 3562 (2006) regarding human antibodies generated via a human B-cell hybridoma technology.

The term "humanized" antibody or "humanized" antibody variable domain, as used herein, refers to an antibody or antibody variable domain that retains the reactivity of a non-human antibody or antibody variable domain while being less immunogenic in humans. This can be achieved, for instance, by retaining the non-human CDR regions and replacing the remaining parts of the antibody or antibody variable domain with their human counterparts (*i*. *e*., the constant region as well as the framework portions of the variable region). Additional framework region modifications may be made within the human framework sequences as well as within the CDR sequences derived from the germline of another mammalian species. The humanized antibodies and antibody variable domains of the invention may include amino acid residues not encoded by human sequences (*e. g*., mutations introduced by random or site-specific mutagenesis *in vitro* or by somatic mutation *in vivo,* or a conservative substitution to promote stability or manufacturing). See, *e. g*., Morrison et al., Proc. Natl. Acad. Sci. USA, 81:6851-6855, 1984; Morrison and Oi, Adv. Immunol., 44:65-92, 1988; Verhoeyen et al., Science, 239: 1534-1536, 1988; Padlan, Molec. Immun., 28:489-498, 1991; and Padlan, Molec. Immun., 31: 169-217, 1994. Other examples of human engineering technology include but are not limited to the Xoma technology disclosed in U.S. Pat. No. 5,766,886.

The term "recombinant humanized antibody" or "recombinant humanized antibody variable domain" as used herein, includes all human antibodies and human antibody variable domains that are prepared, expressed, created or isolated by recombinant means, such as antibodies and antibody variable domains isolated from a host cell transformed to express the humanized antibody or humanized antibody variable domain, e. g., from a transfectoma, and antibodies and antibody variable domains prepared, expressed, created or isolated by any other means that involve splicing of all or a portion of a human immunoglobulin gene, sequences to other DNA sequences.

Preferably, the antibody variable domains and multispecific antibodies of the invention are humanized. More preferably, the antibody variable domains and multispecific antibodies of the invention are humanized and comprise rabbit derived CDRs.

The term "multispecific antibody" as used herein, refers to an antibody that binds to two or more different epitopes on at least two or more different targets (*e*. *g.,* IL-4R and IL-31). Preferably, the multispecific antibodies of the invention are bispecific. The term "bispecific antibody" as used herein, refers to an antibody that binds to at least two different epitopes on two different targets (*e. g.*, IL-4R and IL-31).

The term "epitope" means a protein determinant capable of specific binding to an antibody. Epitopes usually consist of chemically active surface groupings of molecules such as amino acids or sugar side chains and usually have specific three-dimensional structural characteristics, as well as specific charge characteristics. "Conformational" and "linear" epitopes are distinguished in that the binding to the former but not the latter is lost in the presence of denaturing solvents.

The term "conformational epitope" as used herein refers to amino acid residues of an antigen that come together on the surface when the polypeptide chain folds to form the native protein.

The term "linear epitope" refers to an epitope, wherein all points of interaction between the protein and the interacting molecule (such as an antibody) occurring linearly along the primary amino acid sequence of the protein (continuous).

The term "recognize" as used herein refers to an antibody or antibody variable domain that finds and interacts (*e. g*., binds) with its conformational epitope.

Suitably, the multispecific antibodies of the invention comprise one or two antibody variable domains that specifically bind IL-4R, as defined herein. Particularly, the multispecific antibodies of the invention comprise two antibody variable domains that specifically bind IL-4R, as defined herein.

The term "IL-4R" refers in particular to *human* IL-4R with UniProt ID number P24394. The antibody variable domains of the present invention target human IL-4R. Particularly, the antibody variable domains of the invention target *human* and Cynomolgus (*Macaca fascicularis*) IL-4R. More particularly, the antibody variable domains of the invention target *human,* Cynomolgus (*Macaca fascicularis*) and Marmoset *(Callithrix jacchus*) IL-4R.

The antibody variable domains of the invention, when being in scFv format, are characterized by the following parameters:
a. bind to human IL-4R with a monovalent dissociation constant (K_{D}) of 0.1 to 200 pM, particularly with a K_{D} of 0.1 to 100 pM, particularly of 0.1 to 50 pM, as measured by surface plasmon resonance (SPR);
b. are cross-reactive with *Macaca fascicularis* (Cynomolgus) IL4R, in particular bind to Cynomolgus IL-4R with a monovalent K_{D} of 1 pM to 5 nM, particularly of 2 pM to 3 nM, particularly of 3 pM to 2 nM, as measured by SPR; and
g. have a melting temperature (Tm), determined by differential scanning fluorimetry (DSF), of at least 63°C, particularly of at least 65°C, particularly of at least 67°C, particularly of at least 69°C, particularly of at least 70°C, particularly of at least 71°C, particularly of at least 72°C, in particular wherein said scFvs are formulated in 50 mM phosphate citrate buffer with 150 mM NaCl at pH 6.4.

In specific embodiments, the antibody variable domains of the invention, when being in scFv format, are characterized by the following parameters:
a. bind to human IL-4R with a monovalent dissociation constant (K_{D}) of 0.1 to 200 pM, particularly with a K_{D} of 0.1 to 100 pM, particularly of 0.1 to 50 pM, as measured by surface plasmon resonance (SPR);
b. are cross-reactive with *Macaca fascicularis* (Cynomolgus) IL4R, in particular bind to Cynomolgus IL-4R with a monovalent K_{D} of 1 pM to 5 nM, particularly of 2 pM to 3 nM, particularly of 3 pM to 2 nM, as measured by SPR;
d. neutralize the human IL-4 induced signaling with an IC₅₀ of 0.01 to 5 ng/ml, particularly with an IC₅₀ of 0.01 to 3 ng/ml, particularly with an IC₅₀ of 0.01 to 1.5 ng/ml, as measured in a stat6 reporter gene assay in HEK-Blue cells;
e. neutralize the human IL-13 induced signaling with an IC₅₀ of 0.01 to 10 ng/ml, particularly with an IC₅₀ of 0.01 to 5 ng/ml, particularly with an IC₅₀ of 0.01 to 2.5 ng/ml, as measured in a stat-6 reporter gene assay in HEK-Blue cells;
g. have a melting temperature (Tm), determined by differential scanning fluorimetry (DSF), of at least 63°C, particularly of at least 65°C, particularly of at least 67°C, particularly of at least 69°C, particularly of at least 70°C, particularly of at least 71°C, particularly of at least 72°C, in particular wherein said scFvs are formulated in 50 mM phosphate citrate buffer with 150 mM NaCl at pH 6.4; and
h. have a loss in monomer content, after storage for at least four weeks, at 4°C, of less than 5 %, when said scFv is at a starting concentration of 10 mg/ml, and in particular wherein said scFvs are formulated in 50 mM phosphate citrate buffer with 150 mM NaCl at pH 6.4.

In more specific embodiments, the antibody variable domains of the invention, when being in scFv format, are characterized by the following parameters:
a. bind to human IL-4R with a monovalent dissociation constant (K_{D}) of 0.1 to 200 pM, particularly with a K_{D} of 0.1 to 100 pM, particularly of 0.1 to 50 pM,as measured by surface plasmon resonance (SPR);
b. are cross-reactive with *Macaca fascicularis* (Cynomolgus) IL4R, in particular bind to Cynomolgus IL-4R with a monovalent K_{D} of 1 pM to 5 nM, particularly of 2 pM to 3 nM, particularly of 3 pM to 2 nM, as measured by SPR;
d. neutralize the human IL-4 induced signaling with an IC₅₀ of 0.01 to 5 ng/ml, particularly with an IC₅₀ of 0.01 to 3 ng/ml, particularly with an IC₅₀ of 0.01 to 1.5 ng/ml, as measured in a stat6 reporter gene assay in HEK-Blue cells;
e. neutralize the human IL-13 induced signaling with an IC₅₀ of 0.01 to 10 ng/ml, particularly with an IC₅₀ of 0.01 to 5 ng/ml, particularly with an IC₅₀ of 0.01 to 2.5 ng/ml, as measured in a stat-6 reporter gene assay in HEK-Blue cells;
f. inhibit the binding of human IL-4 to human IL-4R with an IC₅₀ of 0.01 to 10 ng/ml, particularly with an IC₅₀ of 0.01 to 5 ng/ml, particularly with an IC₅₀ of 0.01 to 2 ng/ml, as measured in a competition ELISA;
g. have a melting temperature (Tₘ), determined by differential scanning fluorimetry (DSF), of at least 63°C, particularly of at least 65°C, particularly of at least 67°C, particularly of at least 69°C, particularly of at least 70°C, particularly of at least 71°C, particularly of at least 72°C, in particular wherein said scFvs are formulated in 50 mM phosphate citrate buffer with 150 mM NaCl at pH 6.4;
h. have a loss in monomer content, after storage for at least four weeks, at 4°C, of less than 5 %, when said scFvs are at a starting concentration of 10 mg/ml, and in particular wherein said scFvs are formulated in 50 mM phosphate citrate buffer with 150 mM NaCl at pH 6.4; and
i. have a loss in protein content, after storage for at least four weeks, at 40°C, of less than 5 %, particularly of less than 4%, particularly of less than 3%, particularly of less than 2%, particularly of less than 1%, when said scFvs are at a starting concentration of 10 mg/ml, and in particular wherein said scFvs are formulated in 50 mM phosphate citrate buffer with 150 mM NaCl at pH 6.4.

In more specific embodiments, the antibody variable domains of the invention, when being in scFv format, are characterized by the following parameters:
a. bind to human IL-4R with a monovalent dissociation constant (K_{D}) of 0.1 to 200 pM, particularly with a K_{D} of 0.1 to 100 pM, particularly of 0.1 to 50 pM, as measured by surface plasmon resonance (SPR);
b. are cross-reactive with *Macaca fascicularis* (Cynomolgus) IL4R, in particular bind to Cynomolgus IL-4R with a monovalent K_{D} of 1 pM to 5 nM, particularly of 2 pM to 3 nM, particularly of 3 pM to 2 nM, as measured by SPR;
c. are cross-reactive with *Callithrix jacchus* (Marmoset) IL-4R, in particular bind to Marmoset IL-4R with a monovalent K_{D} of 1 pM to 5 nM, particularly of 2 pM to 3 nM, particularly of 3 pM to 2 nM, as measured by SPR;
d. neutralize the human IL-4 induced signaling with an IC₅₀ of 0.01 to 5 ng/ml, particularly with an IC₅₀ of 0.01 to 3 ng/ml, particularly with an IC₅₀ of 0.01 to 1.5 ng/ml, as measured in a stat6 reporter gene assay in HEK-Blue cells;
e. neutralize the human IL-13 induced signaling with an IC₅₀ of 0.01 to 10 ng/ml, particularly with an IC₅₀ of 0.01 to 5 ng/ml, particularly with an IC₅₀ of 0.01 to 2.5 ng/ml, as measured in a stat-6 reporter gene assay in HEK-Blue cells;
f. inhibit the binding of human IL-4 to human IL-4R with an IC₅₀ of 0.01 to 10 ng/ml, particularly with an IC₅₀ of 0.01 to 5 ng/ml, particularly with an IC₅₀ of 0.01 to 2 ng/ml, as measured in a competition ELISA;
g. have a melting temperature (Tm), determined by differential scanning fluorimetry (DSF), of at least 63°C, particularly of at least 65°C, particularly of at least 67°C, particularly of at least 69°C, particularly of at least 70°C, particularly of at least 71°C, particularly of at least 72°C, in particular wherein said scFvs are formulated in 50 mM phosphate citrate buffer with 150 mM NaCl at pH 6.4;
h. have a loss in monomer content, after storage for at least four weeks, at 4°C, of less than 5 %, when said scFvs are at a starting concentration of 10 mg/ml, and in particular wherein said scFvs are formulated in 50 mM phosphate citrate buffer with 150 mM NaCl at pH 6.4;
i. have a loss in protein content, after storage for at least four weeks, at 40°C, of less than 5 %, particularly of less than 4%, particularly of less than 3%, particularly of less than 2%, particularly of less than 1%, when said scFvs are at a starting concentration of 10 mg/ml, and in particular wherein said scFvs are formulated in 50 mM phosphate citrate buffer with 150 mM NaCl at pH 6.4; and
j. have a loss in monomer content after 5 freeze-thawing cycles of less than 2 %, particularly of less than 1%, when said scFvs are at a starting concentration of 10 mg/ml, and in particular wherein said scFvs are formulated in 50 mM phosphate citrate buffer with 150 mM NaCl at pH 6.4.

The term "HEK-Blue cells" or "HEK-Blue", as used herein, refers to commercially available human embryonic kidney cells that are transfected with and stably express an optimized secreted embryonic alkaline phosphatase (SEAP) reporter gene under the control of a promoter inducible by NF-κB transcription factor. The level of SEAP protein released into the culture media is typically used as a measure of NF-κB activation.

As used herein, the term "affinity" refers to the strength of interaction between the antibody or the antibody variable domain and the antigen at single antigenic sites. Within each antigenic site, the variable region of the antibody variable domain or the antibody "arm" interacts through weak non-covalent forces with antigen at numerous sites; the more interactions, the stronger the affinity.

"Binding affinity" generally refers to the strength of the total sum of non-covalent interactions between a single binding site of a molecule (*e. g*., of an antibody or an antibody variable domain) and its binding partner (*e. g*., an antigen or, more specifically, an epitope on an antigen). Unless indicated otherwise, as used herein, "binding affinity", "bind to", "binds to" or "binding to" refers to intrinsic binding affinity that reflects a 1:1 interaction between members of a binding pair (*e. g*., an antibody variable domain and an antigen). The affinity of a molecule X for its partner Y can generally be represented by the dissociation constant (K_{D}). Affinity can be measured by common methods known in the art, including those described herein. Low-affinity antibodies and antibody variable domains generally bind antigens slowly and tend to dissociate readily, whereas high-affinity antibodies generally bind antigens faster and tend to remain bound longer. A variety of methods of measuring binding affinity are known in the art, any of which can be used for purposes of the present invention. Specific illustrative and exemplary embodiments for measuring binding affinity, *i*. *e*. binding strength are described in the following.

The term "K_{assoc}", "Ka" or "Kₒₙ", as used herein, are intended to refer to the association rate of a particular antibody-antigen interaction, whereas the term "K_{dis}", "K_{d}" or "K_{off}", as used herein, is intended to refer to the dissociation rate of a particular antibody-antigen interaction. In one embodiment, the term "K_{D}", as used herein, is intended to refer to the dissociation constant, which is obtained from the ratio of K_{d} to Kₐ (*i*. *e*. K_{d}/Kₐ) and is expressed as a molar concentration (M). The "K_{D}" or "K_{D} value" or "KD" or "KD value" according to this invention is in one embodiment measured by using surface plasmon resonance assays.

Affinity to recombinant *human* IL-4R, recombinant *Cynomolgus* IL-4R and recombinant *Marmoset* IL-4R was determined by surface plasmon resonance (SPR) measurements, as described in the paragraphs [0192] and [0194] (scFvs) and [0227] (multispecific molecules).

The antibody variable domain of the invention acts as an antagonist of IL-4R. In other words, the antibody variable domain of the invention is an inhibitor of IL-4R signaling. The term "blocker" or "inhibitor" or "antagonist", as used herein, refers to an antibody or antibody variable domain that inhibits or reduces a biological activity of the antigen it binds to. The antibody variable domains of the invention bind to the IL-4R, thereby blocking the binding of IL-4 to IL-4R, in particular the binding of both IL-4 and IL-13 to IL-4R, which leads to reduced IL-4R function.

DSF is described earlier (Egan, et al., MAbs, 9(1) (2017), 68-84; Niesen, et al., Nature Protocols, 2(9) (2007) 2212-2221). The midpoint of transition for the thermal unfolding of the scFv constructs is determined by Differential Scanning Fluorimetry using the fluorescence dye SYPRO^{®} Orange (see Wong & Raleigh, Protein Science 25 (2016) 1834-1840). Samples in phosphate-citrate buffer at pH 6.4 are prepared at a final protein concentration of 50 µg/ml and containing a final concentration of 5x SYPRO^{®} Orange in a total volume of 100 µl. Twenty-five microliters of prepared samples are added in triplicate to white-walled AB gene PCR plates. The assay is performed in a qPCR machine used as a thermal cycler, and the fluorescence emission is detected using the software's custom dye calibration routine. The PCR plate containing the test samples is subjected to a temperature ramp from 25°C to 96°C in increments of 1°C with 30 s pauses after each temperature increment. The total assay time is about 2 h. The Tm is calculated by the software GraphPad Prism using a mathematical second derivative method to calculate the inflection point of the curve. The reported Tm is an average of three measurements.

The loss in monomer content is as determined by area under the curve calculation of SE-HPLC chromatograms. SE-HPLC is a separation technique based on a solid stationary phase and a liquid mobile phase as outlined by the US Pharmacopeia (USP), chapter 621. This method separates molecules based on their size and shape utilizing a hydrophobic stationary phase and aqueous mobile phase. The separation of molecules is occurring between the void volume (V₀) and the total permeation volume (V_{T}) of a specific column. Measurements by SE-HPLC are performed on a Chromaster HPLC system (Hitachi High-Technologies Corporation) equipped with automated sample injection and a UV detector set to the detection wavelength of 280 nm. The equipment is controlled by the software EZChrom Elite (Agilent Technologies, Version 3.3.2 SP2) which also supports analysis of resulting chromatograms. Protein samples are cleared by centrifugation and kept at a temperature of 4-6°C in the autosampler prior to injection. For the analysis of scFv samples the column Shodex KW403-4F (Showa Denko Inc., #F6989202) is employed with a standardized buffered saline mobile phase (50 mM sodium-phosphate pH 6.5, 300 mM sodium chloride) at the recommended flow rate of 0.35 ml/min. The target sample load per injection was 5 µg. Samples are detected by an UV detector at a wavelength of 280 nm and the data recorded by a suitable software suite. The resulting chromatograms are analyzed in the range of Vo to V_{T} thereby excluding matrix associated peaks with >10 min elution time.

Suitably, the antibody variable domains of the invention are binding domains provided in the present disclosure. They are derived from the rabbit antibody clone 44-34-C10, and include, but are not limited to, the humanized antibody variable domains whose sequences are listed in Table 1.

The term "multivalent antibody" refers to a single binding molecule with more than one valency, where "valency" is described as the number of antigen-binding moieties that binds to epitopes on target molecules. As such, the single binding molecule can bind to more than one binding site on a target molecule and/or to more than one target molecule due to the presence of more than one copy of the corresponding antigen-binding moieties. Examples of multivalent antibodies include, but are not limited to bivalent antibodies, trivalent antibodies, tetravalent antibodies, pentavalent antibodies, hexavalent antibodies, and the like.

The term "monovalent antibody", as used herein, refers to an antibody that binds to a single target molecule, and more specifically to a single epitope on a target molecule. Also, the term "binding domain" or "monovalent binding domain", as used herein, refers to a binding domain that binds to a single epitope on a target molecule.

In particular embodiments, the multispecific antibodies of the invention comprise one antibody variable domain that specifically binds IL-4R, as defined herein, and one binding domain, which binds to a target different from IL-4R, *i*. *e*. the multispecific antibodies of the invention are monovalent for both IL-4R and the target different from IL-4R.

In further particular embodiments, the multispecific antibodies of the invention comprise one antibody variable domain that specifically binds IL-4R, as defined herein, and two binding domains, which have the same binding specificity and specifically bind to a target different from IL-4R, *i*. *e*. the multispecific antibodies of the invention are monovalent for IL-4R specificity and bivalent for the target different from IL-4R.

In further particular embodiments, the multispecific antibodies of the invention comprise two antibody variable domains that specifically bind IL-4R, as defined herein, and one binding domain, which binds to a target different from IL-4R, *i*. *e*. the multispecific antibodies of the invention are bivalent for IL-4R specificity and monovalent for the target different from IL-4R.

In preferred embodiments, the multispecific antibodies of the invention comprise two antibody variable domains that specifically bind IL-4R, as defined herein, and two binding domains, which have the same binding specificity and specifically bind to a target different from IL-4R, *i*. *e*. the multispecific antibodies of the invention are bivalent for IL-4R specificity and bivalent for the target different from IL-4R.

In case the multispecific antibodies of the invention comprise two binding domains, which have the same binding specificity and specifically bind to a target different from IL-4R, said two binding domains either bind the same epitope or different epitopes on the target molecules. Preferably, the two binding domains bind the same epitope on the target molecule.

The term "same epitope", as used herein, refers to an individual protein determinant on the protein capable of specific binding to more than one antibody, where that individual protein determinant is identical, *i*. *e*. consist of identical chemically active surface groupings of molecules such as amino acids or sugar side chains having identical three-dimensional structural characteristics, as well as identical charge characteristics for each of said antibodies.

The term "different epitope", as used herein in connection with a specific protein target, refers to individual protein determinants on the protein, each capable of specific binding to a different antibody, where these individual protein determinants are not identical for the different antibodies, *i*. *e*. consist of non-identical chemically active surface groupings of molecules such as amino acids or sugar side chains having different three-dimensional structural characteristics, as well as different charge characteristics. These different epitopes can be overlapping or nonoverlapping.

In particular embodiments, the multispecific antibodies of the invention are bispecific and bivalent.

In further particular embodiments, the multispecific antibodies of the invention are bispecific and trivalent.

Preferably, the multispecific antibodies of the invention are bispecific and tetravalent, *i*. *e*. bivalent for IL-4R and bivalent for a target different from IL-4R.

In a particular aspect, the present invention relates to a multispecific antibody comprising:
a) two antibody variable domains as defined herein;
b) two binding domains, which have the same binding specificity and specifically binds to a target different from IL-4R,
wherein said multispecific antibody comprises an IgG region.

Other variable domains used in the invention include amino acid sequences that have been mutated, yet have at least 90, 91, 92, 93, 94, 95, 96, 97, 98 or 99 percent identity in the CDR regions with the CDR regions depicted in the sequences described in Table 1, provided that such other variable domains exhibit the functional features of section [0068], and optionally additionally of section [0069] to [0071]. Other variable domains used in the invention include mutant amino acid sequences wherein no more than 1, 2, 3, 4 or 5 amino acids have been mutated in the CDR regions when compared with the CDR regions depicted in the sequence described in Table 1, provided that such other variable domains exhibit the functional features of section [0068], and optionally additionally of section [0069] to [0071].

Suitably, the VH domains of the binding domains of the invention belong to a VH3 or VH4 family. In one embodiment, a binding domain used in the invention comprises a VH domain belonging to the VH3 family. In the context of the present invention, the term "belonging to VHx family (or VLx family)" means that the framework sequences FR1 to FR3 show the highest degree of homology to said VHx family (or VLx, respectively). Examples of VH and VL families are given in Knappik et al., J. Mol. Biol. 296 (2000) 57-86, or in WO 2019/057787. A specific example of a VH domain belonging to VH3 family is represented by SEQ ID NO: 19, and a specific example of a VH domain belonging to VH4 family is represented by SEQ ID NO: 20. In particular, framework regions FR1 to FR3 taken from SEQ ID NO: 19 belong to VH3 family (Table 2, regions marked in non-bold). Suitably, a VH belonging to VH3 family, as used herein, is a VH comprising FR1 to FR3 having at least 90 %, more particularly at least 95 %, at least 96%, at least 97%, at least 98%, at least 99%, sequence identity to FR1 to FR3 of SEQ ID NO: 19. Alternative examples of VH3 and VH4 sequences, and examples of other VHx sequences, may be found in Knappik et al., J. Mol. Biol. 296 (2000) 57-86 or in WO 2019/057787.

Suitably, the VL domains of the binding domains used in the invention comprise: Vκ frameworks FR1, FR2 and FR3, particularly Vκ1 or Vκ3 frameworks, particularly Vκ1 frameworks FR1 to FR3, and a framework FR4, which is selected from a Vκ FR4. In the case where said binding domains are in an scFv-format, said binding domains comprise: Vκ frameworks FR1, FR2 and FR3, particularly Vκ1 or Vκ3 frameworks, particularly Vκ1 frameworks FR1 to FR3, and a framework FR4, which is selected from a Vκ FR4 and a Vλ FR4, particularly a Vλ FR4.

Suitable Vκ1 frameworks FR1 to FR3 as well as an exemplary Vλ FR4 are set forth in SEQ ID NO: 21 (Table 2, FR regions are marked in non-bold). Alternative examples of Vκ1 sequences, and examples of Vκ2, Vκ3 or Vκ4 sequences, may be found in Knappik et al., J. Mol. Biol. 296 (2000) 57-86. Suitable Vκ1 frameworks FR1 to FR3 comprise the amino acid sequences having at least 80, 90, 95 percent identity to amino acid sequences corresponding to FR1 to FR3 and taken from SEQ ID NO: 21 (Table 2, FR regions are marked in non-bold). Suitable Vλ FR4 are as set forth in SEQ ID NO: 22 to SEQ ID NO: 28 and in SEQ ID NO: 29 comprising a single cysteine residue, particular in a case where a second single cysteine is present in the corresponding VH chain, particularly in position 51 (AHo numbering) of VH, for the formation of an inter-domain disulfide bond. In one embodiment, the VL domains of the binding domains of the invention, when being in scFv-format, comprises Vλ FR4 comprising the amino acid sequence having at least 80, 90, 95 percent identity to an amino acid sequence selected from any of SEQ ID NO: 22 to SEQ ID NO: 29, particularly to SEQ ID NO: 22 or 29.

The antibody variable domains of the invention comprise a VH domain listed in Table 1. Suitably, the antibody variable domains of the invention comprise a VH amino acid sequence listed in Table 1, wherein no more than 5 amino acids, particularly no more than 4 amino acids, particularly no more than 3 amino acids, particularly no more than 2 amino acids, particularly no more than 1 amino acid in the framework sequences (*i*. *e*., the sequence which is not CDR sequences) have been mutated (wherein a mutation is, as various non-limiting examples, an addition, substitution or deletion). Other binding domains used in the invention include amino acids that have been mutated, yet have at least 90, 91, 92, 93, 94, 95, 96, 97, 98 or 99 percent identity in the VH regions with the VH regions depicted in the corresponding sequences described in Table 1, including VH domains comprising at least positions 5 to 140 (AHo numbering), particularly at least positions 3 to 145 of one of the sequences shown in Table 1, provided that such other variable domains exhibit the functional features of section [0068], and optionally additionally of section [0069] to [0071].

In particular, the antibody variable domains of the invention comprise a VL domain listed in Table 1. Suitably, the antibody variable domains of the invention comprise a VL amino acid sequence listed in Table 1, wherein no more than 5 amino acids, particularly no more than 4 amino acids, particularly no more than 3 amino acids, particularly no more than 2 amino acids, particularly no more than 1 amino acid in the framework sequences (*i*. *e*., the sequence which is not CDR sequences) have been mutated (wherein a mutation is, as various non-limiting examples, an addition, substitution or deletion). Other binding domains used in the invention include amino acids that have been mutated, yet have at least 90, 91, 92, 93, 94, 95, 96, 97, 98 or 99 percent identity in the VL regions with a VL region depicted in the sequences described in Table 1, including VL domains comprising at least positions 5 to 140 (AHo numbering), particularly at least positions 3 to 145 of one of the sequences shown in Table 1, provided that such other variable domains exhibit the functional features of section [0068], and optionally additionally of section [0069] to [0071].

Specific but non-limiting examples of the antibody variable domains of the invention are the scFvs PRO1517, PRO1535, PRO1747, PRO1896, PRO1897, PRO1898 and PRO1899, whose sequences are listed in Table 3.

Suitably, the at least one binding domains of the multispecific antibodies of the invention are selected from the group consisting of: a Fab, an Fv, a dsFv and an scFv.

The antibody variable domains and the binding domains comprised in the multispecific antibodies of the invention are capable of binding to their respective antigens or receptors simultaneously. The term "simultaneously", as used in this connection refers to the simultaneous binding of at least one of the antibody variable domains that specifically bind to IL-4R and at least one of the binding domains that have specificity for a target different from IL-4R.

Suitably, the antibody variable domains and the binding domains comprised in the multispecific antibodies of the invention are operably linked.

The term "operably linked", as used herein, indicates that two molecules (e. g., polypeptides, domains, binding domains) are attached in a way that each molecule retains functional activity. Two molecules can be "operably linked" whether they are attached directly or indirectly (e. g., via a linker, via a moiety, via a linker to a moiety). The term "linker" refers to a peptide or other moiety that is optionally located between binding domains or antibody variable domains used in the invention. A number of strategies may be used to covalently link molecules together. These include, but are not limited to, polypeptide linkages between N- and C-termini of proteins or protein domains, linkage via disulfide bonds, and linkage via chemical cross-linking reagents. In one aspect of this embodiment, the linker is a peptide bond, generated by recombinant techniques or peptide synthesis. Choosing a suitable linker for a specific case where two polypeptide chains are to be connected depends on various parameters, including but not limited to the nature of the two polypeptide chains (*e. g*., whether they naturally oligomerize), the distance between the N- and the C-termini to be connected if known, and/or the stability of the linker towards proteolysis and oxidation. Furthermore, the linker may contain amino acid residues that provide flexibility.

In the context of the present invention, the term "polypeptide linker" refers to a linker consisting of a chain of amino acid residues linked by peptide bonds that is connecting two domains, each being attached to one end of the linker. The polypeptide linker should have a length that is adequate to link two molecules in such a way that they assume the correct conformation relative to one another so that they retain the desired activity. In particular embodiments, the polypeptide linker has a continuous chain of between 2 and 30 amino acid residues (*e*. *g.,* 2, 3, 4, 5, 6, 7, 8, 9, 10, 11, 12, 13, 14, 15, 16, 17, 18, 19, 20, 21, 22, 23, 24, 25, 26, 27, 28, 29, or 30 amino acid residues). In addition, the amino acid residues selected for inclusion in the polypeptide linker should exhibit properties that do not interfere significantly with the activity of the polypeptide. Thus, the linker peptide on the whole should not exhibit a charge that would be inconsistent with the activity of the polypeptide, or interfere with internal folding, or form bonds or other interactions with amino acid residues in one or more of the monomers that would seriously impede the binding of receptor monomer domains. In particular embodiments, the polypeptide linker is non-structured polypeptide. Useful linkers include glycine-serine, or GS linkers. By "Gly-Ser" or "GS" linkers is meant a polymer of glycines and serines in series (including, for example, (Gly-Ser)ₙ, (GSGGS)ₙ (GGGGS)ₙ and (GGGS)ₙ, where n is an integer of at least one), glycine-alanine polymers, alanine-serine polymers, and other flexible linkers such as the tether for the shaker potassium channel, and a large variety of other flexible linkers, as will be appreciated by those in the art. Glycine-serine polymers are preferred since oligopeptides comprising these amino acids are relatively unstructured, and therefore may be able to serve as a neutral tether between components. Secondly, serine is hydrophilic and therefore able to solubilize what could be a globular glycine chain. Third, similar chains have been shown to be effective in joining subunits of recombinant proteins such as single-chain antibodies.

In one group of embodiments, the multispecific antibody of the invention comprises an immunoglobulin Fc region and is in a format selected from (scFv)₂-Fc-(scFv)₂ fusion (ADAPTIR); DVD-Ig; a DART^{™} and a TRIDENT^{™}. The term " DART^{™}" refers to an antibody format developed by MacroGenics that comprises an immunoglobulin Fc region polypeptide and one or two bispecific Fv binding domains fused to the N-terminus of one heavy chain of the Fc region or to both N-termini of the heavy chains of the Fc region. The term " TRIDENT^{™}" refers to an antibody format developed by MacroGenics that comprises an immunoglobulin Fc region polypeptide, one bispecific Fv binding domain and one Fab fragment. Both the bispecific Fv binding domain and the Fab fragment are fused to the respective N-termini of the two heavy chains of the Fc region polypeptide.

In another group of embodiments, the format of the multispecific antibodies of the present invention is selected from bivalent bispecific IgG formats, trivalent bispecific IgG formats and tetravalent bispecific IgG formats. In particular, the format of said multispecific antibodies is selected from KiH-based IgGs, such as DuoBodies (bispecific IgGs prepared by the Duobody technology) (MAbs. 2017 Feb/Mar;9(2): 182-212. doi: 10.1080/19420862.2016.1268307); DVD-Ig; IgG-scFv fusions, such as CODV-IgG, Morrison (IgG CH₃-scFv fusion (Morrison-H) or IgG CL-scFv fusion (Morrison-L)), bsAb (scFv linked to C-terminus of light chain), Bs1Ab (scFv linked to N-terminus of light chain), Bs2Ab (scFv linked to N-terminus of heavy chain), Bs3Ab (scFv linked to C-terminus of heavy chain), Ts1Ab (scFv linked to N-terminus of both heavy chain and light chain) and Ts2Ab (dsscFv linked to C-terminus of heavy chain). More particularly, the format of said multispecific antibody is selected from KiH-based IgGs, such as DuoBodies; DVD-Ig; CODV-IgG and Morrison (IgG CH₃-scFv fusion (Morrison-H) or IgG CL-scFv fusion (Morrison-L)), even more particularly from DVD-Ig and Morrison (IgG CH₃-scFv fusion (Morrison-H) or IgG CL-scFv fusion (Morrison-L)).

In particular embodiments of the invention, the format of said multispecific antibodies is selected from a Morrison format, *i*. *e*. a Morrison-L and a Morrison-H format. The Morrison-L and Morrison-H format used in the present invention are tetravalent and bispecific molecular formats bearing an IgG Fc region, in particular an IgG4 Fc region. Two highly stable scFv binding domains, wherein the light chain comprises Vk FR1 to FR3 in combination with a Vλ FR4 (A-cap), herein also called λ-cap scFv, are fused via a linker L1 to the heavy chain (Morrison-H) or light chain (Morrison-L) C-termini.

The linker L1 is a peptide of 2-30 amino acids, more particularly 5-25 amino acids, and most particularly 10-20 amino acids. In particular embodiments, said linker L1 comprises one or more units of four (4) glycine amino acid residues and one (1) serine amino acid residue (GGGGS)ₙ, wherein n=1, 2, 3, 4 or 5, particularly n=2.

In a special embodiment of the invention, the multispecific antibodies have a Morrison-L format, as defined above. In another special embodiment of the invention, the multispecific antibodies have a Morrison-H format, as defined above.

In case the antibody variable domains comprised in the multispecific antibodies of the invention are in the form of scFv fragments, these scFv fragments comprise a variable heavy chain domain (VH) and a variable light chain domain (VL) connected by a linker L2.

The linker L2 is a peptide of 10-40 amino acids, more particularly 15-30 amino acids, and most particularly 20-25 amino acids. In particular embodiments, said linker L2 comprises one or more units of four (4) glycine amino acid residues and one (1) serine amino acid residue (GGGGS)ₙ, wherein n=1, 2, 3, 4, 5, 6, 7 or 8, particularly n=4.

Specific but non-limiting examples of multispecific antibodies of the invention, where the antibody variable domains comprised therein are Fab fragments, are the Morrison-H antibodies PRO2198, PRO2199, whose sequences are listed in Table 4.

The antibody variable domains and multispecific antibodies of the invention can be produced using any convenient antibody-manufacturing method known in the art (see, e. g., Fischer, N. & Leger, O., Pathobiology 74 (2007) 3-14 with regard to the production of bispecific constructs; Hornig, N. & Farber-Schwarz, A., Methods Mol. Biol. 907 (2012)713-727, and WO 99/57150 with regard to bispecific diabodies and tandem scFvs). Specific examples of suitable methods for the preparation of the bispecific construct further include, inter alia, the Genmab (see Labrijn et al., Proc. Natl. Acad. Sci. USA 110 (2013) 5145-5150) and Merus (see de Kruif et al., Biotechnol. Bioeng. 106 (2010) 741-750) technologies. Methods for production of bispecific antibodies comprising a functional antibody Fc part are also known in the art (see, e. g., Zhu et al., Cancer Lett. 86 (1994) 127-134); and Suresh et al., Methods Enzymol. 121 (1986) 210-228).

These methods typically involve the generation of monoclonal antibodies or monoclonal antibody variable domains, for example by means of fusing myeloma cells with the spleen cells from a mouse that has been immunized with the desired antigen using the hybridoma technology (see, e. g., Yokoyama et al., Curr. Protoc. Immunol. Chapter 2, Unit 2.5, 2006) or by means of recombinant antibody engineering (repertoire cloning or phage display/yeast display) (see, e. g., Chames & Baty, FEMS Microbiol. Letters 189 (2000) 1-8), and the combination of the antigen-binding domains or fragments or parts thereof of two or more different monoclonal antibodies to give a bispecific or multispecific construct using known molecular cloning techniques.

The multispecific antibodies of the invention can be prepared by conjugating the constituent binding specificities, using methods known in the art. For example, each binding specificity of the bispecific molecule can be generated separately and then conjugated to one another. When the binding specificities are proteins or peptides, a variety of coupling or cross-linking agents can be used for covalent conjugation. Examples of cross-linking agents include protein A, carbodiimide, N-succinimidyl-5-acetyl-thioacetate (SATA), 5,5'-dithiobis (2-nitrobenzoic acid) (DTNB), o-phenylenedimaleimide (oPDM), N-succinimidyl-3-(2-pyridyldithio)propionate (SPDP), and sulfosuccinimidyl 4-(N-maleimidomethyl)-cyclohaxane-1-carboxylate (sulfo-SMCC) (see *e. g*., Karpovsky et al., 1984 J. Exp. Med. 160: 1686; Liu, M A et al., 1985 Proc. Natl. Acad. Sci. USA 82:8648). Other methods include those described in Paulus, 1985 Behring Ins. Mitt. No. 78, 118-132; Brennan et al., 1985 Science 229:81-83, and Glennie et al., 1987 J. Immunol. 139: 2367-2375. Conjugating agents are SATA and sulfo-SMCC, both available from Pierce Chemical Co. (Rockford, III).

Alternatively, two or more binding specificities can be encoded in the same vector and expressed and assembled in the same host cell. This method is particularly useful where the bispecific molecule is a mAb x Fab, a mAb x scFv, a mAb x dsFv or a mAb x Fv fusion protein. Methods for preparing multispecific antibodies and molecules are described for example in U.S. Pat. No. 5,260,203; U.S. Pat. No. 5,455,030; U.S. Pat. No. 4,881,175; U.S. Pat. No. 5,132,405; U.S. Pat. No. 5,091,513; U.S. Pat. No. 5,476,786; U.S. Pat. No. 5,013,653; U.S. Pat. No. 5,258,498; and U.S. Pat. No. 5,482,858.

Binding of the antibody variable domains and multispecific antibodies to their specific targets can be confirmed by, for example, enzyme-linked immunosorbent assay (ELISA), radioimmunoassay (REA), FACS analysis, bioassay (*e. g*., growth inhibition), or Western Blot assay. Each of these assays generally detects the presence of protein-antibody complexes of particular interest by employing a labeled reagent (*e. g*., an antibody) specific for the complex of interest.

In a further aspect, the invention provides a nucleic acid sequence or two nucleic acid sequences encoding the antibody variable domain or the multispecific antibody of the invention. Such nucleic acid sequences can be optimized for expression in mammalian cells.

The term "nucleic acid" is used herein interchangeably with the term "polynucleotide(s)" and refers to one or more deoxyribonucleotides or ribonucleotides and polymers thereof in either single- or double-stranded form. The term encompasses nucleic acids containing known nucleotide analogs or modified backbone residues or linkages, which are synthetic, naturally occurring, and non-naturally occurring, which have similar binding properties as the reference nucleic acid, and which are metabolized in a manner similar to the reference nucleotides. Examples of such analogs include, without limitation, phosphorothioates, phosphoramidates, methyl phosphonates, chiral-methyl phosphorates, 2-O-methyl ribonucleotides, peptide-nucleic acids (PNAs). Unless otherwise indicated, a particular nucleic acid sequence also implicitly encompasses conservatively modified variants thereof (*e. g*., degenerate codon substitutions) and complementary sequences, as well as the sequence explicitly indicated. Specifically, as detailed below, degenerate codon substitutions may be achieved by generating sequences in which the third position of one or more selected (or all) codons is substituted with mixed-base and/or deoxyinosine residues (Batzer et al., Nucleic Acid Res. 19:5081, 1991; Ohtsuka et al., J. Biol. Chem. 260:2605-2608, 1985; and Rossolini et al., Mol. Cell. Probes 8:91-98, 1994).

The invention provides substantially purified nucleic acid molecules which encode polypeptides comprising segments or domains of the antibody variable domain or multispecific antibody described above. When expressed from appropriate expression vectors, polypeptides encoded by these nucleic acid molecules are capable of exhibiting antigen-binding capacities of the multispecific antibody of the present invention.

The polynucleotide sequences can be produced by *de novo* solid-phase DNA synthesis or by PCR mutagenesis of an existing sequence (*e. g*., sequences as described in the Examples below) encoding the multispecific antibody of the invention or variable domains thereof or binding domains thereof. Direct chemical synthesis of nucleic acids can be accomplished by methods known in the art, such as the phosphotriester method of Narang et al., 1979, Meth. Enzymol. 68:90; the phosphodiester method of Brown et al., Meth. Enzymol. 68: 109, 1979; the diethylphosphoramidite method of Beaucage et al., Tetra. Lett., 22: 1859, 1981; and the solid support method of U.S. Pat. No. 4,458,066. Introducing mutations to a polynucleotide sequence by PCR can be performed as described in, *e. g*., PCR Technology: Principles and Applications for DNA Amplification, H. A. Erlich (Ed.), Freeman Press, NY, N.Y., 1992; PCR Protocols: A Guide to Methods and Applications, Innis et al. (Ed.), Academic Press, San Diego, Calif, 1990; Mattila et al., Nucleic Acids Res. 19:967, 1991; and Eckert et al., PCR Methods and Applications 1:17, 1991.

Also provided in the invention are expression vectors and host cells for producing the antibody variable domain or multispecific antibody of the invention.

The term "vector" is intended to refer to a polynucleotide molecule capable of transporting another polynucleotide to which it has been linked. One type of vector is a "plasmid", which refers to a circular double stranded DNA loop into which additional DNA segments may be ligated. Another type of vector is a viral vector, wherein additional DNA segments may be ligated into the viral genome. Certain vectors are capable of autonomous replication in a host cell into which they are introduced (*e. g*., bacterial vectors having a bacterial origin of replication and episomal mammalian vectors). Other vectors (*e. g*., non-episomal mammalian vectors) can be integrated into the genome of a host cell upon introduction into the host cell, and thereby are replicated along with the host genome.

Moreover, certain vectors are capable of directing the expression of genes to which they are operatively linked. Such vectors are referred to herein as "recombinant expression vectors" (or simply, "expression vectors"). In general, expression vectors of utility in recombinant DNA techniques are often in the form of plasmids. In the present specification, "plasmid" and "vector" may be used interchangeably as the plasmid is the most commonly used form of vector. However, the invention is intended to include such other forms of expression vectors, such as viral vectors (*e. g.*, replication defective retroviruses, adenoviruses and adeno-associated viruses), which serve equivalent functions. In this particular context, the term "operably linked" refers to a functional relationship between two or more polynucleotide (*e. g*., DNA) segments. Typically, it refers to the functional relationship of a transcriptional regulatory sequence to a transcribed sequence. For example, a promoter or enhancer sequence is operably linked to a coding sequence if it stimulates or modulates the transcription of the coding sequence in an appropriate host cell or other expression system. Generally, promoter transcriptional regulatory sequences that are operably linked to a transcribed sequence are physically contiguous to the transcribed sequence, *i*. *e*., they are cis-acting. However, some transcriptional regulatory sequences, such as enhancers, need not be physically contiguous or located in close proximity to the coding sequences whose transcription they enhance.

Various expression vectors can be employed to express the polynucleotides encoding the antibody variable domain or multispecific antibody chain(s). Both viral-based and non-viral expression vectors can be used to produce the antibodies or antibody variable domains in a mammalian host cell. Non-viral vectors and systems include plasmids, episomal vectors, typically with an expression cassette for expressing a protein or RNA, and human artificial chromosomes (see, *e*. *g.,* Harrington et al., Nat Genet. 15:345, 1997). For example, non-viral vectors useful for expression of the IL-4R-binding polynucleotides and polypeptides in mammalian (e. g., human) cells include pThioHis A, B and C, pcDNA3.1/His, pEBVHis A, B and C, (Invitrogen, San Diego, Calif.), MPS V vectors, and numerous other vectors known in the art for expressing other proteins. Useful viral vectors include vectors based on retroviruses, adenoviruses, adeno-associated viruses, herpes viruses, vectors based on SV40, papilloma virus, HBP Epstein Barr virus, vaccinia virus vectors and Semliki Forest virus (SFV). See, Brent et al., supra; Smith, Annu. Rev. Microbiol. 49:807, 1995; and Rosenfeld et al., Cell 68: 143, 1992.

The choice of expression vector depends on the intended host cells in which the vector is to be expressed. Typically, the expression vectors contain a promoter and other regulatory sequences (*e. g*., enhancers) that are operably linked to the polynucleotides encoding a multispecific antibody chain or a variable domain. In one embodiment, an inducible promoter is employed to prevent expression of inserted sequences except under inducing conditions. Inducible promoters include, *e. g*., arabinose, lacZ, metallothionein promoter or a heat shock promoter. Cultures of transformed organisms can be expanded under non-inducing conditions without biasing the population for coding sequences whose expression products are better tolerated by the host cells. In addition to promoters, other regulatory elements may also be required or desired for efficient expression of a multispecific antibody chain or a variable domain. These elements typically include an ATG initiation codon and adjacent ribosome binding site or other sequences. In addition, the efficiency of expression may be enhanced by the inclusion of enhancers appropriate to the cell system in use (see, e. g., Scharf et al., Results Probl. Cell Differ. 20: 125, 1994; and Bittner et al., Meth. Enzymol., 153:516, 1987). For example, the SV40 enhancer or CMV enhancer may be used to increase expression in mammalian host cells.

Vectors to be used typically encode the antibody variable domain or multispecific antibody light and heavy chain including constant regionsor parts thereof, if present. Such vectors allow expression of the variable regions as fusion proteins with the constant regions thereby leading to production of intact antibodies and antibody variable domains thereof. Typically, such constant regions are human.

The term "recombinant host cell" (or simply "host cell") refers to a cell into which a recombinant expression vector has been introduced. It should be understood that such terms are intended to refer not only to the particular subject cell but to the progeny of such a cell. Because certain modifications may occur in succeeding generations due to either mutation or environmental influences, such progeny may not, in fact, be identical to the parent cell, but are still included within the scope of the term "host cell" as used herein.

The host cells for harboring and expressing the antibody variable domain or multispecific antibody of the invention can be either prokaryotic or eukaryotic. E. *coli* is one prokaryotic host useful for cloning and expressing the polynucleotides of the present invention. Other microbial hosts suitable for use include bacilli, such as *Bacillus subtilis,* and other enterobacteriaceae, such as Salmonella, Serratia, and various Pseudomonas species. In these prokaryotic hosts, one can also make expression vectors, which typically contain expression control sequences compatible with the host cell (*e. g*., an origin of replication). In addition, any number of a variety of well-known promoters will be present, such as the lactose promoter system, a tryptophan (trp) promoter system, a beta-lactamase promoter system, or a promoter system from phage lambda. The promoters typically control expression, optionally with an operator sequence, and have ribosome binding site sequences and the like, for initiating and completing transcription and translation. Other microbes, such as yeast, can also be employed to express the antibody variable domain or multispecific antibodies of the invention. Insect cells in combination with baculovirus vectors can also be used.

In one embodiment, mammalian host cells are used to express and produce the antibody variable domain or multispecific antibody of the invention. For example, they can be either a hybridoma cell line expressing endogenous immunoglobulin genes or a mammalian cell line harboring an exogenous expression vector. These include any normal mortal or normal or abnormal immortal animal or human cell. For example, a number of suitable host cell lines capable of secreting intact immunoglobulins have been developed including the CHO cell lines, various COS cell lines, HeLa cells, myeloma cell lines, transformed B-cells and hybridomas. The use of mammalian tissue cell culture to express polypeptides is discussed generally in, *e. g*., Winnacker, FROM GENES TO CLONES, VCH Publishers, N.Y., N.Y., 1987. Expression vectors for mammalian host cells can include expression control sequences, such as an origin of replication, a promoter, and an enhancer (see, e. *g.,* Queen, et al., Immunol. Rev. 89:49-68, 1986), and necessary processing information sites, such as ribosome binding sites, RNA splice sites, polyadenylation sites, and transcriptional terminator sequences. These expression vectors usually contain promoters derived from mammalian genes or from mammalian viruses. Suitable promoters may be constitutive, cell type-specific, stage-specific, and/or modulatable or regulatable. Useful promoters include, but are not limited to, the metallothionein promoter, the constitutive adenovirus major late promoter, the dexamethasone-inducible MMTV promoter, the SV40 promoter, the MRP pollll promoter, the constitutive MPS V promoter, the tetracycline-inducible CMV promoter (such as the human immediate-early CMV promoter), the constitutive CMV promoter, and promoter-enhancer combinations known in the art.

Methods for introducing expression vectors containing the polynucleotide sequences of interest vary depending on the type of cellular host. For example, calcium chloride transfection is commonly utilized for prokaryotic cells, whereas calcium phosphate treatment or electroporation may be used for other cellular hosts. (See generally Green, M. R., and Sambrook, J., Molecular Cloning: A Laboratory Manual (Fourth Edition), Cold Spring Harbor Laboratory Press (2012)). Other methods include, *e. g*., electroporation, calcium phosphate treatment, liposome-mediated transformation, injection and microinjection, ballistic methods, virosomes, immunoliposomes, polycation-nucleic acid conjugates, naked DNA, artificial virions, fusion to the herpes virus structural protein VP22 (Elliot and O'Hare, Cell 88:223, 1997), agent-enhanced uptake of DNA, and *ex vivo* transduction. For long-term, high-yield production of recombinant proteins, stable expression will often be desired. For example, cell lines which stably express the antibody variable domain or multispecific antibody of the invention can be prepared using expression vectors of the invention which contain viral origins of replication or endogenous expression elements and a selectable marker gene. Following the introduction of the vector, cells may be allowed to grow for 1 to 2 days in an enriched media before they are switched to selective media. The purpose of the selectable marker is to confer resistance to selection, and its presence allows growth of cells which successfully express the introduced sequences in selective media. Resistant, stably transfected cells can be proliferated using tissue culture techniques appropriate to the cell type. The present invention thus provides a method of producing the antibody variable domain or multispecific antibody of the invention, wherein said method comprises the step of culturing a host cell comprising a nucleic acid or a vector encoding the antibody variable domain or multispecific antibody of the invention, whereby said antibody variable domain or said multispecific antibody of the disclosure is expressed.

In one aspect, the present invention relates to a method of producing the antibody variable domain multispecific antibody of the invention, the method comprising the step of culturing a host cell expressing a nucleic acid encoding the antibody variable domain or multispecific antibody of the invention. In particular, the present invention relates to a method of producing the antibody variable domain or multispecific antibody of the invention, the method comprising (i) providing a nucleic acid sequence or two nucleic acid sequences encoding the antibody variable domain or multispecific antibody of the invention or one or two vectors encoding the antibody variable domain or multispecific antibody of the invention, expressing said nucleic acid sequence or nucleic acid sequences, or said vector or vectors, and collecting said antibody variable domain or multispecific antibody from the expression system, or (ii) providing a host cell or host cells expressing a nucleic acid sequence or two nucleic acid sequences encoding the antibody variable domain or multispecific antibody of the invention, culturing said host cell or said host cells; and collecting said antibody variable domain or said multispecific antibody from the cell culture.

In a further aspect, the present invention relates to a pharmaceutical composition comprising the multispecific antibody of the invention, and a pharmaceutically acceptable carrier. "Pharmaceutically acceptable carrier" means a medium or diluent that does not interfere with the structure of the antibodies. Pharmaceutically acceptable carriers enhance or stabilize the composition, or facilitate preparation of the composition. Pharmaceutically acceptable carriers include solvents, dispersion media, coatings, antibacterial and antifungal agents, isotonic and absorption delaying agents, and the like that are physiologically compatible.

Certain of such carriers enable pharmaceutical compositions to be formulated as, for example, tablets, pills, dragees, capsules, liquids, gels, syrups, slurries, suspension and lozenges for the oral ingestion by a subject. Certain of such carriers enable pharmaceutical compositions to be formulated for injection, infusion or topical administration. For example, a pharmaceutically acceptable carrier can be a sterile aqueous solution.

The pharmaceutical composition of the invention can be administered by a variety of methods known in the art. The route and/or mode of administration vary depending upon the desired results. Administration can be intravenous, intramuscular, intraperitoneal, or subcutaneous, or administered proximal to the site of the target. In particular embodiments, the administration is intramuscular, or subcutaneous, particularly subcutaneous. The pharmaceutically acceptable carrier should be suitable for intravenous, intramuscular, subcutaneous, parenteral, spinal or epidermal administration (*e*. *g.,* by injection or infusion), particularly for intramuscular or subcutaneous administration. Depending on the route of administration, the active compound, *i*. *e*., the multispecific antibody of the invention, may be coated in a material to protect the compound from the action of acids and other natural conditions that may inactivate the compound.

The pharmaceutical compositions of the invention can be prepared in accordance with methods well known and routinely practiced in the art. See, *e*. *g.,* Remington: The Science and Practice of Pharmacy, Mack Publishing Co., 20th ed., 2000; and Sustained and Controlled Release Drug Delivery Systems, J. R. Robinson, ed., Marcel Dekker, Inc., New York, 1978. Pharmaceutical compositions are preferably manufactured under GMP conditions. Typically, a therapeutically effective dose or efficacious dose of the multispecific antibody of the invention is employed in the pharmaceutical compositions of the invention. The multispecific antibodies of the invention are formulated into pharmaceutically acceptable dosage forms by conventional methods known to those of skill in the art. Dosage regimens are adjusted to provide the optimum desired response (*e. g*., a therapeutic response). For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the subjects to be treated; each unit contains a predetermined quantity of active compound calculated to produce the desired therapeutic effect in association with the required pharmaceutical carrier.

Actual dosage levels of the active ingredients in the pharmaceutical compositions of the invention can be varied so as to obtain an amount of the active ingredient which is effective to achieve the desired therapeutic response for a particular patient, composition, and mode of administration, without being toxic to the patient. The selected dosage level depends upon a variety of pharmacokinetic factors including the activity of the particular compositions of the present invention employed, or the ester, salt or amide thereof, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors.

The multispecific antibody of the invention is usually administered on multiple occasions. Intervals between single dosages can be weekly, monthly or yearly. Intervals can also be irregular as indicated by measuring blood levels of the multispecific antibody of the invention in the patient. Alternatively, the multispecific antibody of the invention can be administered as a sustained release formulation, in which case less frequent administration is required. Dosage and frequency vary depending on the half-life of the antibody in the patient. In general, humanized antibodies show longer half-life than that of chimeric antibodies and nonhuman antibodies. The dosage and frequency of administration can vary depending on whether the treatment is prophylactic or therapeutic. In prophylactic applications, a relatively low dosage is administered at relatively infrequent intervals over a long period of time. Some patients continue to receive treatment for the rest of their lives. In therapeutic applications, a relatively high dosage at relatively short intervals is sometimes required until progression of the disease is reduced or terminated, and preferably until the patient shows partial or complete amelioration of symptoms of disease. Thereafter, the patient can be administered a prophylactic regime.

In one aspect, the present invention relates to the multispecific antibody of the invention or the pharmaceutical composition of the invention for use as a medicament. In a suitable embodiment, the present invention provides the multispecific antibody or the pharmaceutical composition for use in the treatment of a disease selected from allergic, inflammatory and autoimmune diseases, particularly from inflammatory and autoimmune diseases.

In another aspect, the present invention provides the pharmaceutical composition for use in the manufacture of a medicament for the treatment of an allergic, inflammatory or autoimmune disease, particularly of an inflammatory or autoimmune disease.

In another aspect, the present invention relates to the use of the multispecific antibody or the pharmaceutical composition for treating an allergic, inflammatory or autoimmune disease, particularly for treating an inflammatory or autoimmune disease, in a subject in need thereof.

In another aspect, the present invention relates to a method of treating a subject comprising administering to the subject a therapeutically effective amount of the multispecific antibody of the present invention. In a suitable embodiment, the present invention relates to a method for the treatment of an allergic, inflammatory or autoimmune disease, particularly for treating an inflammatory or autoimmune disease, in a subject comprising administering to the subject a therapeutically effective amount of the multispecific antibody of the present invention.

The term "subject" includes human and non-human animals.

The term "animals" include all vertebrates, *e. g*., non-human mammals and non-mammals, such as non-human primates, sheep, dog, cow, chickens, amphibians, and reptiles. Except when noted, the terms "patient" or "subject" are used herein interchangeably.

The terms "treatment", "treating", "treat", "treated", and the like, as used herein, refer to obtaining a desired pharmacologic and/or physiologic effect. The effect may be therapeutic in terms of a partial or complete cure for a disease and/or adverse effect attributable to the disease or delaying the disease progression. "Treatment", as used herein, covers any treatment of a disease in a mammal, *e*. *g.,* in a human, and includes: (a) inhibiting the disease, *i*. *e*., arresting its development; and (b) relieving the disease, *i*. *e*., causing regression of the disease.

The term "therapeutically effective amount" or "efficacious amount" refers to the amount of an agent that, when administered to a mammal or other subject for treating a disease, is sufficient to affect such treatment for the disease. The "therapeutically effective amount" will vary depending on the agent, the disease and its severity and the age, weight, etc., of the subject to be treated.

In one embodiment, the allergic, inflammatory and autoimmune diseases are selected from pruritus-causing allergic diseases, pruritus-causing inflammatory diseases and pruritus-causing autoimmune diseases, particularly from pruritus-causing inflammatory diseases and pruritus-causing autoimmune diseases. The terms "pruritus" and "itch", which are herein used synonymously, refers to a sensation that causes the desire or reflex to scratch. Scratching can be problematic particularly in the case of chronic skin diseases that induce itching, such as atopic dermatitis, dermatomycoses, psoriasis or urticaria, as the permanent itching stimulates patients to scratch the affected skin areas constantly and/or excessively, which often leads to skin injuries and further deterioration of the skin surface.

The term "allergic diseases" or "allergies" as used herein refers to a large number of conditions caused by hypersensitivity of the immune system to typically harmless substances in the environment.

The term "inflammatory diseases" as used herein refers to a vast number of inflammatory disorders, *i*. *e*. inflammatory abnormalities, which are often characterized by prolonged inflammation, known as chronic inflammation. The term "inflammation" refers to the complex biological response of body tissues to harmful stimuli, such as pathogens, damaged cells, or irritants. It is a protective response involving immune cells, blood vessels, and molecular mediators. While regular inflammation reactions are essential for the body to eliminate the initial cause of cell injury, clear out necrotic cells and tissues damaged from the original insult and the inflammatory process, and to initiate tissue repair, inflammatory disorders are generally characterized by persistent inflammation in the absence of harmful stimuli.

The term "autoimmune diseases" as used herein refers to a condition arising from an abnormal immune response to a functioning body part. It is the result of autoimmunity, *i*. *e*. the presence of self-reactive immune response (e. g., auto-antibodies, self-reactive T cells), with or without damage or pathology resulting from it, which is typically restricted to certain organs or involve a particular tissue in different places.

In particular embodiments, the pruritus-causing inflammatory or autoimmune disease is selected from atopic dermatitis, acute allergic contact dermatitis, chronic spontaneous urticaria, bullous pemphigoid, alopecia areata, dermatomyositis, prurigo nodularis, psoriasis and atopic asthma, in particular from atopic dermatitis.

In another aspect, the allergic, inflammatory and autoimmune diseases are selected from atopic dermatitis, acute allergic contact dermatitis, chronic spontaneous urticaria, bullous pemphigoid, alopecia areata, dermatomyositis, prurigo nodularis, psoriasis and atopic asthma, in particular from atopic dermatitis.

In another embodiment, the allergic, inflammatory and autoimmune diseases are selected from allergic asthma, allergic rhinitis, inflammatory airway disease, recurrent airway obstruction, airway hyperresponsiveness, chronic obstruction pulmonary disease, Crohn disease, chronic non-histamine related urticaria, antihistamine-unresponsive mastocytosis, lichen simplex chronicus, seborrhoeic dermatitis, xeroderma, Dermatitis herpetiformis, lichen planus and ulcerative colitis.

In another embodiment, the present invention provides the multispecific antibody or the pharmaceutical composition, as defined herein, for use in the treatment of a disease, which is a neuropathic pruritus disease selected from post-herpetic neuralgia, post-herpetic itch, notalgia paresthetica, multiple sclerosis and brachioradial pruritus.

In another embodiment, the present invention provides the multispecific antibody or the pharmaceutical composition for use in an antipruritic agent for the treatment of an systemic disease with itching, which disease is selected from Cholestasis, chronic kidney disease, Hodgkin's disease, cutaneous T-cell lymphoma and other lymphomas or leukemias associated with chronic itch, polycythemia vera, hyperthyroidism, chronic post-arthropod itch (Id reaction), pregnancy-induced chronic itch (e.g. PUPPP), eosinophilic pustular folliculitis, drug hypersensitivity reactions, chronic pruritus of the elderly or dry skin itch (local, generalized), and pruritus at the scar portion after burn (post-burn itch), genetic or nevoid chronic itches (e.g. Netherton syndrome, Darier's disease (Morbus Darier), Hailey-Hailey disease, inflammatory linear verrucous epidermal nevus (ILVEN), familial primary cutaneous amyloidosis, Olmsted syndrome), aquagenic pruritus, fiberglass dermatitis, mucous chronic itch, chemotherapy-induced itch and HIV.

### Sequence listing (mutations designated according to AHo numbering scheme; the CDRs defined according to Numab CDR definition, unless specified otherwise)

**Table 1. Examples of IL-4R binding domains as used in the present invention CDR residues shown in bold and italic letters).**

| **SEQ ID NUMBER** | **Ab region** | **Sequence** |
|---|---|---|
| 44-34-C10 | | |
| SEQ ID NO: 1 | **HCDR1** (H27-H42; AHo numbering) | ***GFSSSSAHYMC*** |
| SEQ ID NO: 2 | **HCDR2** (H57-H76; AHo numbering) | ***CIYAGSRGSTYYASWAKG*** |
| SEQ ID NO: 3 | **HCDR3** (H108-H138; AHo numbering) | ***RAAFVNRGVSWIWPYYFSL*** |
| SEQ ID NO: 4 | **VH** (44-34-C10-sc01/44-34-C10-sc07/44-34-C10-sc08) | EVQLVESGGGLVQPGGSLRLSCAASGFSSSSAHYMCWVRQAPGKGLEWIGCIY AGSRGSTYYASWAKGRFTISRDNSKNTVYLQMNSLRAEDTAVYYCARAAFVN RGVSWIWPYYFSLWGQGTLVTVSS |
| SEQ ID NO: 5 | **VH** (44-34-C10-sc04) | ESQLVESGGGLVQPGGSLRLSCAASGFSSSSAHYMCWVRQAPGKGLEWIACIY AGSRGSTYYASWAKGRFTISKDNSKNTVYLQMNSLRAEDTAVYFCARAAFVNR GVSWIWPYYFSLWGQGTLVTVSS |
| SEQ ID NO: 6 | **VH** (44-34-C10-sc05) mutation G51C | EVQLVESGGGLVQPGGSLRLSCAASGFSSSSAHYMCWVRQAPGKCLEWIGCIY AGSRGSTYYASWAKGRFTISRDNSKNTVYLQMNSLRAEDTAVYYCARAAFVN RGVSWIWPYYFSLWGQGTLVTVSS |
| SEQ ID NO: 7 | **VH** (44-34-C10-sc06) | EVQLVESGGGRVQPGGSLRLSCAASGFSSSSAHYMCWVRQAPGKGLEWIGCIY AGSRGSTYYASWAKGRFTISRDNSKNTVYLQMNSLRAEDTATYYCARAAFVNR GVSWIWPYYFSLWGQGTQVTVSS |
| SEQ ID NO: 8 | **VH** (44-34-C10-sc09) | EVQLVESGGGRVQPGGSLRLSCAASGFSSSSAHYMCWVRQAPGKGLEWIGCIY AGSRGSTYYASWAKGRFTISKASSTTVYLQMNSLRAEDTATYYCARAAFVNRG VSWIWPYYFSLWGQGTQVTVSS |
| SEQ ID NO: 9 | **VH** (44-34-C10-sc09) mutations R12L, T103V, Q144L | EVQLVESGGGLVQPGGSLRLSCAASGFSSSSAHYMCWVRQAPGKGLEWIGCIY AGSRGSTYYASWAKGRFTISKASSTTVYLQMNSLRAEDTAVYYCARAAFVNRG VSWIWPYYFSLWGQGTLVTVSS |
| SEQ ID NO: 10 | **LCDR1** (L24-L42; AHo numbering) | ***QASEDIESYLA*** |
| SEQ ID NO: 11 | **LCDR2** (L58-L72; AHo numbering) (present in 44-34-C10-sc06, 44-34-C10-sc08 and 44-34-C10-sc09) | ***RASTLA** Y* |
| SEQ ID NO: 12 | **LCDR2** (L58-L72; AHo numbering) (present in 44-34-C 10-sc07) | ***RASTLAS*** |
| SEQ ID NO: 13 | **LCDR3** (L107-L138; AHo numbering) | ***LGSSYSTGSNI*** |
| SEQ ID NO: 14 | **VL** (44-34-C10-sc01/44-34-C10-sc06/44-34-C10-sc09) | DIQMTQSPSSLSASVGDRVTITCQASEDIESYLAWYQQKPGKAPKLLIYRASTL AYGVPSRFSGSGSGTDFTLTISSLQPEDFATYYCLGSSYSTGSNI |
| SEQ ID NO: 15 | **VL** (44-34-C10-sc04) | DIQMTQSPSSLSASVGDRVTITCQASEDIESYLAWYQQKPGKPPKLLIYRASTLA YGVPSRFSGSGSGTDFTLTISSLQPEDFATYYCLGSSYSTGSNIFGTGTKVTVL |
| SEQ ID NO: 16 | **VL** (44-34-C10-sc05) mutation G141C | DIQMTQSPSSLSASVGDRVTITCQASEDIESYLAWYQQKPGKAPKLLIYRASTL AYGVPSRFSGSGSGTDFTLTISSLQPEDFATYYCLGSSYSTGSNI |
| SEQ ID NO: 17 | **VL** (44-34-C10-sc07) | DIQMTQSPSSLSASVGDRVTITCQASEDIESYLAWYQQKPGKAPKLLIYRASTL ASGVPSRFSGSGSGTDFTLTISSLQPEDFATYYCLGSSYSTGSNI |
| SEQ ID NO: 18 | **VL** (44-34-C10-sc08) | DIQMTQSPSSLSARVGDRVTITCQASEDIESYLAWYQQKPGKAPKLLIYRASTL AYGVPSRFSGSGSGRDFTLTISSLQPEDFATYYCLGSSYSTGSNI |

**Table 2. Other sequences related to the present invention.**

| **SEQ ID NUMBER** | **Ab region** | **Sequence** |
|---|---|---|
| SEQ ID NO: 19 | VH3 | EVQLVESGGGLVQPGGSLRLSCAASGFSFSANYYPCWVRQAPGKGLEWIGCIY GGSSDITYDANWTKGRFTISRDNSKNTVYLQMNSLRAEDTAVYYCARSAWYS GWGGDLWGQGTLVTVSS |
| SEQ ID NO: 20 | VH4 | QVQLQESGPGLVKPSETLSLTCKVSGFSFSNSYWICWIRQPPGKGLEWIGCTFV GSSDSTYYANWAKGRVTISVDSSKNQFSLKLSSVTAADTAVYYCARHPSDAVY GYANNLWGQGTLVTVSS |
| SEQ ID NO: 21 | Vkappal | DIQMTQSPSSLSASVGDRVTITCQASQSINNVLAWYQQKPGKAPKLLIYRASTL ASGVPSRFSGSGSGTDFTLTISSLQPEDFATYYCQSSYGNYGDFGTGTKVTVLG |
| SEQ ID NO: 22 | Vλ germline-based FR4 (Sk17) | FGTGTKVTVLG |
| SEQ ID NO: 23 | Vλ germline-based FR4 (Sk12) | FGGGTKLTVLG |
| SEQ ID NO: 24 | Vλ germline-based FR4 | FGGGTQLIILG |
| SEQ ID NO: 25 | Vλ germline-based FR4 | FGEGTELTVLG |
| SEQ ID NO: 26 | Vλ germline-based FR4 | FGSGTKVTVLG |
| SEQ ID NO: 27 | Vλ germline-based FR4 | FGGGTQLTVLG |
| SEQ ID NO: 28 | Vλ germline-based FR4 | FGGGTQLTALG |
| SEQ ID NO: 29 | Vλ germline-based FR4 G141C | FGCGTKVTVLG |
| SEQ ID NO: 30 | **Linker** | GGGGSGGGGSGGGGSGGGGS |
| SEQ ID NO: 31 | **Linker** | GGGGS |
| SEQ ID NO: 32 | **Linker** | GGGGSGGGGS |

**Table 3. Examples of scFv antibody variable domains of the present invention (Linkers are shown in bold).**

| **SEQ ID NUMBER** | **Ab name and description** | **Sequence** |
|---|---|---|
| SEQ ID NO: 33 | **PRO1517; scFv** (VL-linker- VH) (44-34-C10-sc01) | DIQMTQSPSSLSASVGDRVTITCQASEDIESYLAWYQQKPGKAPKLLIYRASTLAYGVPS RFSGSGSGTDFTLTISSLQPEDFATYYCLGSSYSTGSNIFGTGTKVTVLGGGGGSGGGG SGGGGSGGGGSEVQLVESGGGLVQPGGSLRLSCAASGFSSSSAHYMCWVRQAPGKG LEWIGCIYAGSRGSTYYASWAKGRFTISRDNSKNTVYLQMNSLRAEDTAVYYCARAAF VNRGVSWIWPYYFSLWGQGTLVTVSS |
| SEQ ID NO: 34 | **PRO1535; scFv** (VL-linker- VH) (44-34-C10-sc04) | DIQMTQSPSSLSASVGDRVTITCQASEDIESYLAWYQQKPGKPPKLLIYRASTLAYGVPS RFSGSGSGTDFTLTISSLQPEDFATYYCLGSSYSTGSNIFGTGTKVTVLGGGGGSGGGG SGGGGSGGGGSESQLVESGGGLVQPGGSLRLSCAASGFSSSSAHYMCWVRQAPGKGL EWIACIYAGSRGSTYYASWAKGRFTISKDNSKNTVYLQMNSLRAEDTAVYFCARAAFV NRGVSWIWPYYFSLWGQGTLVTVSS |
| SEQ ID NO: 35 | **PRO1747; scFv** (VL-linker- VH) (44-34-C10-sc05) | DIQMTQSPSSLSASVGDRVTITCQASEDIESYLAWYQQKPGKAPKLLIYRASTLAYGVPS RFSGSGSGTDFTLTISSLQPEDFATYYCLGSSYSTGSNIFGCGTKVTVLGGGGGSGGGG SGGGGSGGGGSEVQLVESGGGLVQPGGSLRLSCAASGFSSSSAHYMCWVRQAPGKCL EWIGCIYAGSRGSTYYASWAKGRFTISRDNSKNTVYLQMNSLRAEDTAVYYCARAAF VNRGVSWIWPYYFSLWGQGTLVTVSS |
| SEQ ID NO: 36 | **PRO1896; scFv** (VL-linker- VH) (44-34-C10-sc06) | DIQMTQSPSSLSASVGDRVTITCQASEDIESYLAWYQQKPGKAPKLLIYRASTLAYGVPS RFSGSGSGTDFTLTISSLQPEDFATYYCLGSSYSTGSNIFGTGTKVTVLGGGGGSGGGG SGGGGSGGGGSEVQLVESGGGRVQPGGSLRLSCAASGFSSSSAHYMCWVRQAPGKG LEWIGCIYAGSRGSTYYASWAKGRFTISRDNSKNTVYLQMNSLRAEDTATYYCARAAF VNRGVSWIWPYYFSLWGQGTQVTVSS |
| SEQ ID NO: 37 | **PRO1897; scFv** (VL-linker- VH) (44-34-C10-sc07) | DIQMTQSPSSLSASVGDRVTITCQASEDIESYLAWYQQKPGKAPKLLIYRASTLASGVPS RFSGSGSGTDFTLTISSLQPEDFATYYCLGSSYSTGSNIFGTGTKVTVLGGGGGSGGGG SGGGGSGGGGSEVQLVESGGGLVQPGGSLRLSCAASGFSSSSAHYMCWVRQAPGKG LEWIGCIYAGSRGSTYYASWAKGRFTISRDNSKNTVYLQMNSLRAEDTAVYYCARAAF VNRGVSWIWPYYFSLWGQGTLVTVSS |
| SEQ ID NO: 38 | **PRO1898; scFv** (VL-linker- VH) (44-34-C10-sc08) | DIQMTQSPSSLSARVGDRVTITCQASEDIESYLAWYQQKPGKAPKLLIYRASTLAYGVP SRFSGSGSGRDFTLTISSLQPEDFATYYCLGSSYSTGSNIFGTGTKVTVLGGGGGSGGG GSGGGGSGGGGSEVQLVESGGGLVQPGGSLRLSCAASGFSSSSAHYMCWVRQAPGK GLEWIGCIYAGSRGSTYYASWAKGRFTISRDNSKNTVYLQMNSLRAEDTAVYYCARA AFVNRGVSWIWPYYFSLWGQGTLVTVSS |
| SEQ ID NO: 39 | **PRO1899; scFv** (VL-linker- VH) (44-34-C10-sc09) | DIQMTQSPSSLSASVGDRVTITCQASEDIESYLAWYQQKPGKAPKLLIYRASTLAYGVPS RFSGSGSGTDFTLTISSLQPEDFATYYCLGSSYSTGSNIFGTGTKVTVLGGGGGSGGGG SGGGGSGGGGSEVQLVESGGGRVQPGGSLRLSCAASGFSSSSAHYMCWVRQAPGKG LEWIGCIYAGSRGSTYYASWAKGRFTISKASSTTVYLQMNSLRAEDTATYYCARAAFV NRGVSWIWPYYFSLWGQGTQVTVSS |

**Table 4. Examples of multispecific antibodies of the present invention (Linkers are shown in bold).**

| **SEQ ID NUMBER** | **Ab Format** | **Sequence** |
|---|---|---|
| **PRO2198** | | |
| SEQ ID NO: 40 | Morrison-H, LC | DIQMTQSPSSLSARVGDRVTITCQASEDIESYLAWYQQKPGKAPKLLIYRASTLAYGVP SRFSGSGSGRDFTLTISSLQPEDFATYYCLGSSYSTGSNIFGQGTKVEIKRTVAAPSVFIFP PSDEQLKSGTASVVCLLNNFYPREAKVQWKVDNALQSGNSQESVTEQDSKDSTYSLSS TLTLSKADYEKHKVYACEVTHQGLSSPVTKSFNRGEC |
| SEQ ID NO: 41 | Morrison-H, HC | EVQLVESGGGLVQPGGSLRLSCAASGFSSSAHYMCWVRQAPGKGLEWIGCIYAGSRG STYYASWAKGRFTISRDNSKNTVYLQMNSLRAEDTAVYYCARAAFVNRGVSWIWPYY FSLWGQGTLVTVSSASTKGPSVFPLAPCSRSTSESTAALGCLVKDYFPEPVTVSWNSGA LTSGVHTFPAVLQSSGLYSLSSVVTVPSSSLGTKTYTCNVDHKPSNTKVDKRVESKYGP PCPPCPAPEFLGGPSVFLFPPKPKDTLMISRTPEVTCVVVDVSQEDPEVQFNWYVDGVE VHNAKTKPREEQFNSTYRVVSVLTVLHQDWLNGKEYKCKVSNKGLPSSIEKTISKAKG QPREPQVYTLPPSQEEMTKNQVSLTCLVKGFYPSDIAVEWESNGQPENNYKTTPPVLDS DGSFFLYSRLTVDKSRWQEGNVFSCSVMHEALHNHYTQKSLSLSLGGGGGSGGGGSA FQLTQSPSSLSASVGDRVTITCQASQSIDSWLAWYQQKPGKPPKLLIYQASKLASGVPSR FSGSGSGTDYTLTISSLQPEDFATYYCQTYYGGGHIGWGFGTGTKVTVLGGGGGSGGG GSGGGGSGGGGSQSQLVESGGGRVQPGGSLRLSCAASGFSFSANYWICWVRQAPGK GLEWIVCIYIGSRADTYYAPWAKGRFTISKDNSKNTVYLQMNSLRAEDTATYFCARFSS GIYDLDRFFLWGQGTQVTVSS |

| **PRO2199** | | |
|---|---|---|
| SEQ ID NO: 42 | Morrison-H, LC | DIQMTQSPSSLSASVGDRVTITCQASEDIESYLAWYQQKPGKAPKLLIYRASTLAYGVPS RFSGSGSGTDFTLTISSLQPEDFATYYCLGSSYSTGSNIFGQGTKVEIKRTVAAPSVFIFPP SDEQLKSGTASVVCLLNNFYPREAKVQWKVDNALQSGNSQESVTEQDSKDSTYSLSST LTLSKADYEKHKVYACEVTHQGLSSPVTKSFNRGEC |
| SEQ ID NO: 43 | Morrison-H, HC | EVQLVESGGGRVQPGGSLRLSCAASGFSSSAHYMCWVRQAPGKGLEWIGCIYAGSRG STYYASWAKGRFTISKASSTTVYLQMNSLRAEDTATYYCARAAFVNRGVSWIWPYYFS LWGQGTQVTVSSASTKGPSVFPLAPCSRSTSESTAALGCLVKDYFPEPVTVSWNSGALT SGVHTFPAVLQSSGLYSLSSVVTVPSSSLGTKTYTCNVDHKPSNTKVDKRVESKYGPPC PPCPAPEFLGGPSVFLFPPKPKDTLMISRTPEVTCVVVDVSQEDPEVQFNWYVDGVEVH NAKTKPREEQFNSTYRVVSVLTVLHQDWLNGKEYKCKVSNKGLPSSIEKTISKAKGQP REPQVYTLPPSQEEMTKNQVSLTCLVKGFYPSDIAVEWESNGQPENNYKTTPPVLDSDG SFFLYSRLTVDKSRWQEGNVFSCSVMHEALHNHYTQKSLSLSLGGGGGSGGGGSAFQ LTQSPSSLSASVGDRVTITCQASQSIDSWLAWYQQKPGKPPKLLIYQASKLASGVPSRFS GSGSGTDYTLTISSLQPEDFATYYCQTYYGGGHIGWGFGTGTKVTVLGGGGGSGGGG SGGGGSGGGGSQSQLVESGGGRVQPGGSLRLSCAASGFSFSANYWICWVRQAPGKGL EWIVCIYIGSRADTYYAPWAKGRFTISKDNSKNTVYLQMNSLRAEDTATYFCARFSSGI YDLDRFFLWGQGTQVTVSS |

Throughout the text of this application, should there be a discrepancy between the text of the specification (*e. g*., Tables 1 to 4) and the sequence listing, the text of the specification shall prevail.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination. All combinations of the embodiments pertaining to the invention are specifically embraced by the present invention and are disclosed herein just as if each and every combination was individually and explicitly disclosed. In addition, all sub-combinations of the various embodiments and elements thereof are also specifically embraced by the present invention and are disclosed herein just as if each and every such sub-combination was individually and explicitly disclosed herein.

The present invention is not to be limited in scope by the specific embodiments described herein. Indeed, various modifications of the invention in addition to those described herein will become apparent to those skilled in the art from the foregoing description. Such modifications are intended to fall within the scope of the appended claims.

To the extent possible under the respective patent law, all patents, applications, publications, test methods, literature, and other materials cited herein are hereby incorporated by reference.

The following Examples illustrates the invention described above, but is not, however, intended to limit the scope of the invention in any way. Other test models known as such to the person skilled in the pertinent art can also determine the beneficial effects of the claimed invention.

### Examples

### Example 1: Generation and testing of anti-IL-4R molecules:

### Aim of the project

The goal of the project was to generate humanized monoclonal antibody variable domains that specifically bind to and neutralize the biological effect of human IL-4R. A further goal was to identify anti-IL-4R antibody variable domains that are potent and stable enough for incorporation into multispecific antibody formats.

### 1.1. Immunization

Two different immunization protocols were applied to a total of six rabbits to obtain an optimal immune response against human IL-4R. Rabbits were immunized with recombinantly produced and purified IL-4R (Sino Biological, Cat. No. 10402-H08H). Prior to immunization, the quality of recombinant human IL-4R was analyzed by the manufacturer 1) for purity by SDS-page, and 2) for biological activity by measuring the ability to bind to IL-4 in a functional ELISA. The first group of three rabbits (protocol 1) received 4 injections of 200 µg each throughout 70 days. The second group of three rabbits (protocol 2) received 5 injections of 200 µg each through a period of 112 days. During the course of the immunization, the strength of the humoral immune response against the antigen was qualitatively assessed by determining the maximal dilution (titer) for the serum of each rabbit that still results in detectable binding of the polyclonal serum antibodies to the antigen. Serum antibody titers against the immobilized antigen (recombinant human IL-4R) were assessed using an enzyme-linked immunosorbent assay (ELISA). All six rabbits immunized with purified human IL-4R showed high titers with ECs up to 3 x 10⁷.

### 1.2. Sorting

Prior to the hit identification procedure, a flow-cytometry-based sorting procedure with protein G beads was performed in the presence R-Phycoerythrin (RPE)-labelled IL-4R, which allows the specific detection and isolation of high-affinity IL-4R-binding B-cells. A total of 1.05 x 10⁷ lymphocytes derived from four rabbits were analyzed in this sorting campaign. Of these, a total of 3,520 B-cells expressing IL-4R-specific antibodies (IgG) were isolated and individually cultured as single clones for three to four weeks.

### 1.3. Hit identification

### General remarks

For hit identification, direct ELISA screens were performed to assess binding to human and mouse IL-4R whereas binding to cynomolgus IL-4R was evaluated by SPR only. 483 clones were found to bind to human IL-4R and 164 clones showed cross-reactivity to mouse IL-4R in the direct ELISA.

### Binding to human IL-4R by ELISA

For hit identification, *i*. *e*. the identification of B cell clones that produce antibodies binding to IL-4R, cell culture supernatants of the above 3,520 B cell clones were screened for the presence of antibodies that bind to human IL-4R by ELISA. This primary hit identification procedure was performed with non-purified antibodies from the cell culture supernatants, as the scale of the high-throughput culture does not allow for purification of the individual rabbit antibodies. The use of cell supernatants allows for ranking of large numbers of antibodies. The ELISA method used assesses the "quantity" of rabbit IgGs bound to recombinant human IL-4R, however it does not provide any information regarding the affinity or the concentration of the antibodies. In this assay, supernatants from 483 B cell clones produced a signal that was above background.

### Determination of binding affinity to human IL-4R

In a secondary hit identification procedure, information on the binding affinities to human IL-4R of the 483 monoclonal rabbit antibodies that were qualified as positive during the primary screening were determined by surface plasmon resonance (SPR).

For these affinity screening by SPR, an antibody specific for the Fc region of rabbit IgGs was immobilized on a sensor chip (SPR-2 Affinity Sensor, High Capacity Amine, Sierra Sensors) using a standard amine-coupling procedure. Rabbit monoclonal antibodies in B cell supernatants were captured by the immobilized anti-rabbit IgG antibody. A minimal IgG concentration in the B cell supernatants is required to allow sufficient capture. After capturing of the monoclonal antibodies, human IL-4R ECD (extracellular domain) was injected into the flow cells for 3 min at a concentration of 90 nM, and dissociation of the protein from the IgG captured on the sensor chip was allowed to proceed for 5 min. The apparent dissociation (k_{d}) and association (kₐ) rate constants and the apparent dissociation equilibrium constant (K_{D}) were calculated with the MASS-2 analysis software (Analyzer, Sierra Sensors) using the 1:1 Langmuir binding model.

For 475 anti-IL-4R antibodies binding affinities to human IL-4R could be measured. They showed dissociation constants (K_{D}) ranging from below 2.07 x 10⁻¹² M to 2.95 x 10⁻⁸ M. 32% of the antibodies analyzed had a K_{D} below 0.5 nM and 17% displayed higher affinity than dupilumab.

### Neutralization of human IL-4R in the stat-6 reporter gene cellular assay and in a competition ELISA

All 483 human IL-4R-binding supernatants were further analyzed for their potential to neutralize the biological activity of IL-4R in the cell-based stat-6 reporter gene assay and for blockade of the interaction of human IL-4 with human IL-4R in a competition ELISA.

For this purpose, a cell-based stat-6 dependent reporter gene assay using HEK-Blue cells and a receptor ligand competition-ELISA were developed and adapted for use with B cell supernatant. The cell-based assay allows to evaluate the blockade of IL-4- and IL-13-induced signaling, whereas in the competition ELISA, only the interaction of IL-4 and IL-4R is assessed. Since the assays could be performed with B cell supernatant as matrix and were sensitive and precise enough, both assays were used for screening. Mouse IL-13 present in the B cell supernatant was blocked by a commercially available anti-mouse IL-13 antibody to guarantee that signaling was induced by human IL-13 only in the cell-based assay. The inhibitory activity of each B cell supernatant was tested using single well analysis (no dose-responses were performed). Therefore, the extent of inhibition observed is not only dependent on the IgG properties, but also on the concentration of rabbit IgGs in the B cell supernatant.

122 out of 483 clones blocked the interaction of human IL-4R and human IL-4 by more than 90 % and 178 clones inhibited above 80 %. 119 and 132 clones inhibited IL-4- and IL-13-induced signaling in the HEK-Blue assay, respectively, based on a threshold of > 30 % inhibition and 86 hits inhibited both IL-4- and IL-13-induced signaling.

### Species cross-reactivity (binding to cynomolgus monkey and mouse IL-4R by SPR)

All 483 hits identified in the primary screening were analyzed for species cross-reactivity to cynomolgus monkey and mouse IL-4R by SPR. Binding affinities were determined by surface plasmon resonance (SPR) using a MASS-2 SPR device (Sierra Sensors) similarly as described above for human IL-4R with the exception that 90 nM cynomolgus monkey or murine IL-4R were used instead of human IL-4R.

423 (87.5 %) and 266 clones (55 %) showed binding to cynomolgus monkey and mouse IL-4R, respectively. The higher number of mouse IL-4R cross-reactive clones found by SPR as compared to the direct ELISA (266 versus 164) is due to the use of a more stringent cut-off in the ELISA. The anti-cynomolgus IL-4R antibodies showed equilibrium dissociation constants (K_{D}) ranging from 1.14 x 10⁻¹² M to 9.36 x 10⁻⁶ M. 11 % of all antibodies analyzed had a K_{D} below 500 pM. Rather low overall affinities were determined for the anti-mouse IL-4R antibodies with K_{D} values ranging from 1.63 x 10⁻¹¹ M to 4.57 x 10⁻⁵ M. Overall, there was no good correlation between the K_{D} values of the species, with only about 20 hits showing high affinity for both human and cynomolgus IL-4R.

### 1.4. Hit selection and hit confirmation

### Hit selection and RT-PCR

As a prerequisite for Hit Confirmation, gene sequence analysis and subsequent humanization of the rabbit antibodies, the genetic information encoding the rabbit antibody variable domain needs to be retrieved. This was achieved by reverse transcription (RT) of the respective messenger RNA into the complementary DNA (cDNA), followed by amplification of the double-stranded DNA by the polymerase chain reaction (PCR). The selection of B cell clones subjected to RT-PCR was primarily based on affinities to human IL-4R below 500 pM and on neutralizing activity in the stat-6 reporter gene assay. As additional criterion cross-reactivity to cynomolgus monkey IL-4R was considered.

94 sets of rabbit CDRs, corresponding to 94 independent clones, were selected and identified. Rabbit CDR sequences of all 94 sequenced clones were clustered into a phylogenetic tree. 75 sequences were non-redundant based on CDR regions and 26 sequences contained a free cysteine (considered to be a liability for development). All clones with identical sequence and sequences differing by less than 4 amino acids were excluded. 2 clones with affinities for human IL-4R between 400 and 500 pM and low affinity for cynomolgus IL-4R (K_{D} > 3 nM) were also removed from the selection while 8 additional clones with a free cysteine were included. Clones with free cysteines are prone to aggregation following reformatting, but considering their high affinity for cynomolgus IL-4R these clones were not discarded at this stage. As a result, a total of 60 clones were selected for expression as recombinant IgG.

### Cloning and Manufacture of Monoclonal Antibodies

Following the selection of clones for hit confirmation the rabbit antibodies were cloned, expressed and purified for further characterization. The cloning of the corresponding light and heavy chain variable domains entailed the *in vitro* ligation of the DNA fragments into a suitable mammalian expression vector. The expression vectors for the rabbit antibody heavy and light chains were transfected into a mammalian suspension cell line for transient heterologous expression. Subsequently the secreted rabbit IgGs were affinity purified and the final products were analyzed by sodium dodecyl sulfate polyacrylamide gel electrophoresis (SDS-PAGE), UV absorbance at 280 nm and size-exclusion high performance liquid chromatography (SE-HPLC) to verify identity, content and purity. 58 of the 60 clones could be cloned and manufactured in medium to very good titer (2 - 36 µg protein/ml) with a high monomeric content (95.9 to 99.6 %).

### 1.5. Pharmacological characterization of monoclonal antibodies

### Affinity to human, cynomolgus, marmoset and mouse IL-4R

Binding kinetics of 58 purified monoclonal rabbit antibodies to human IL-4R were determined by SPR (MASS-2) analysis. Each IgG was captured via an anti-rabbit IgG coupled to a carboxylmethylated dextran surface and an analyte dose response was measured. High-affinity binding to human IL-4R was confirmed for all antibodies with K_{D} values ranging from picomolar to sub-picomolar affinities except for two IgGs with nanomolar affinities. 4 of the 57 target-binding antibodies exhibited sub-picomolar K_{D} values.

Binding kinetics to cynomolgus and marmoset monkey IL-4R were determined similarly as described above. Marmoset IL-4R ECD was manufactured on demand by Sino Biological since it was not available commercially. 44 and 48 out of 58 IgGs bound to cynomolgus and marmoset IL-4R, respectively. 35 IgGs were cross-reactive to both monkey species. Higher overall affinities were determined for marmoset IL-4R as compared to human and cynomolgus IL-4R.

Binding to mouse IL-4R was assessed using a direct ELISA instead of SPR. None of the IgGs showed strong binding and EC₅₀ values were not determined since most of the curves did not reach maximum signal (top of curves not defined).

### Stat-6 reporter gene assay in HEK-Blue cells (blockade of human IL-4 and IL-13 induced signaling)

Furthermore, the effects of 58 rabbit monoclonal antibodies on IL-4 and IL-13 induced signaling via the IL-4R in the stat-6 reporter gene assay using HEK-Blue cells was assessed. The potency (IC₅₀) to neutralize signaling induced by 0.02 ng/ml IL-4 or 0.3 ng/ml IL-13 was analyzed for serial dilutions of all antibodies and compared to the potency of dupilumab. For comparison of IgGs from different assay plates, relative IC₅₀ values were used. These values were determined by calibrating the IC₅₀ value of the IgGs against the reference molecule dupilumab included on each assay plate (relative IC₅₀: IC₅₀, dupilumab/IC₅₀, test antibody).

IgGs inhibited IL-4 and IL-13 induced signaling with potencies higher than dupilumab or not more than five-fold lower. In terms of blockade of IL-4 induced signaling, 7 IgGs showed superior potency and 4 IgGs blocked both IL-4 and IL-13 induced signaling more potently than dupilumab.

### Competitive ELISA (inhibition of hIL-4 binding to hIL-4R)

The inhibition of human IL-4 binding to human IL-4R was assessed by competitive ELISA. For this purpose, IL-4 was coated on the ELISA plate. Biotinylated IL-4R was preincubated with the rabbit monoclonal antibodies and the mixture was added to the ELISA plate to allow binding to immobilized IL-4. Bound biotinylated IL-4R was then detected using streptavidin-HRP.

Given the low concentration of biotinylated IL-4R used in the assay, the competitive ELISA was sensitive enough to differentiate the rabbit antibodies in terms of potencies. In comparison with dupilumab, 12 rabbit antibodies inhibited the interaction between human IL-4 and human IL-4R more potently and 25 antibodies showed similar potencies or IC₅₀ values not more than 2.5-fold lower. For 6 antibodies, no relative IC₅₀ was determined due to lack of inhibition or due to incomplete blockade of the interaction.

### Rabbit IgG selection for generation of humanized scFvs

Based on the pharmacodynamic properties of the 58 characterized rabbit monoclonal antibodies the best performing clones were selected for the humanization and lead candidate generation. The criteria for the selection of clones were i) complete blockade of IL-4- and IL-13-induced signaling in the HEK-Blue assay, ii) high affinity to human IL-4R, iii) neutralization of the interaction of human IL-4R with human IL-4 in the competition ELISA, iv) cross-reactivity to cynomolgus and or marmoset IL-4R by SPR and v) sequence diversity. In addition to these criteria the primary sequence of all 58 clones was screened for the presence of unpaired cysteines in the complementary determining regions (CDRs). The identification of unpaired cysteines excluded the commonly occurring pair of cysteines in CDR-H1 and CDR-H2, which is coded in the rabbit's germline repertoire and is considered to form a disulfide bridge.

Since the affinity of most rabbit IgGs was close to or beyond the detection limit of the instrument, the data obtained by SPR were treated with caution. Consequently, the focus was set on the stat-6 reporter gene assay in the selection of rabbit antibodies for humanization. Antibodies showing higher potency or that were not more than 3.3 times less potent to neutralize IL-4 and IL-13 induced signaling in the stat-6 reporter gene assay were selected for humanization. In total, 18 promising clones were selected for reformatting and humanization.

### 1.6. Humanization of scFvs

For the Lead Candidate generation 18 rabbit monoclonal antibody clones were selected. The humanization of these clones comprised the transfer of the rabbit CDRs onto one of Numab's proprietary human variable domain acceptor scaffolds. In this process, the amino acid sequences of the six CDR regions were identified using Numab CDR definitions (Table 5) and grafted into Numab's proprietary and highly stable, fully human Vk1/VH3 lambda-capped acceptor framework, resulting in the constructs termed "CDR graft".

**Table 5: Numab CDR definition**

| Region | AHo numbering | Kabat numbering |
|---|---|---|
| CDR1-VL | 24-42 | 24-34 |
| CDR2-VL | 58-72 | 50-56 |
| CDR3-VL | 107-138 | 89-97 |
| CDR1-VH | 27-42 | 26-35A/35B/... |
| CDR2-VH | 57-76 | 50-65 |
| CDR3-VH | 108-138 | 94-102 |

Exclusive engraftment of rabbit CDRs onto a human acceptor framework is the most basic grafting strategy. However, occasionally particular CDR sets require mutation of particular rabbit framework residues in order to preserve their full functionality. Therefore, beside the "CDR graft", additional grafting variants containing defined patterns of rabbit framework residues were designed.

The humanized scFv constructs were designed and ordered in 1 mg scale as mammalian (CHO-S, pcDNA3.1) expression vectors from GeneUniversal (former General Biosystems). Plasmids were used for transient transfection of CHO-S cells as described below.

### 1.7. Manufacture of humanized scFv

Expression of mammalian constructs was performed in CHO-S cells using CHOgro transient transfection kit (Mirus). Cultures were harvested after 5-7 days (when cell viability <70 % was reached) expression at 37°C by centrifugation followed by filtration. Proteins were purified from clarified culture supernatants by Protein L affinity chromatography. None of the molecules needed polishing by size exclusion chromatography as all molecules exhibited at least one affinity chromatography fraction with a monomeric content >95% post capture as assessed by SE-HPLC analysis. Samples were re-buffered to final buffer (50 mM phosphate-citrate buffer with 150 mM NaCl at pH 6.4) by dialysis. For quality control of the manufactured material standard analytical methods such as SE-HPLC, UV₂₈₀ and SDS-PAGE were applied. The manufacture characteristics of 10 scFv molecules originating from five clones that were deemed to be suitable for incorporation into multispecific antibody formats are summarized in Table 6. Manufacture characteristics of the CDR graft (sc01) and the Full graft (sc04) of clone 44-18-C11 (PRO1510) and its variants (PRO1549-PRO1554) are summarized in Table 7.

**Table 6: scFv manufacture of 5 selected clones. Sc01: CDR graft, sc04: Full graft**

| **Protein ID** | **Description** | **Expression volume [ml]** | **Yield post affinity chromatography purification [mg]** | **Final yield [mg]** | **Titer [mg/l]** | **Purity SE-HPLC [% monomer]** | **Purity SDS-PAGE** | **Tₘ [°C]** | **Tₒₙₛₑₜ [°C]** |
|---|---|---|---|---|---|---|---|---|---|
| PRO1506 | 44-03-A03-sc01 | 200 | 14.6 | 11.7 | 58 | 99.0 | identity confirmed | 62.8 | 56.7 |
| PRO1524 | 44-03-A03-sc04 | 100 | 6.5 | 5.0 | 50 | 99.0 | identity confirmed | 59.6 | 54.0 |
| PRO1510 | 44-18-C11-sc01 | 200 | 12.5 | 10.0 | 50 | 98.0 | identity confirmed | 71.5 | 64.0 |
| PRO1528 | 44-18-C11-sc04 | 100 | 5.7 | 4.7 | 47 | 99.0 | identity confirmed | 59.1 | 52.3 |
| PRO1515 | 44-32-F04-sc01 | 200 | 7.3 | 1.8 | 9 | 98.8 | identity confirmed | 59.0 | 53.0 |
| PRO1533 | 44-32-F04-sc04 | 100 | 7.3 | 4.7 | 47 | 99.1 | identity confirmed | 62.2 | 56.7 |
| PRO1517 | 44-34-C10-sc01 | 50 | 1.5 | 0.3 | 7 | 97.6 | identity confirmed | 63.7 | 57.0 |
| PRO1535 | 44-34-C10-sc04 | 100 | 7.3 | 4.6 | 46 | 99.1 | identity confirmed | 67.9 | 62.7 |
| PRO1520 | 44-39-B07-sc01 | 100 | 9.5 | 3.1 | 31 | 98.2 | identity confirmed | 70.2 | 64.0 |
| PRO1538 | 44-39-B07-sc04 | 100 | 8.4 | 2.2 | 22 | 95.0 | identity confirmed | 68.3 | 63.7 |

**Table 7: scFv manufacture of all grafting variants of clone 44-18-C11. Sc01: CDR graft, sc04: Full graft, sc05 to sc10: other grafting variants**

| **Protein ID** | **Description** | **Expression volume [ml]** | **Yield post capture [mg]** | **Final yield [mg]** | **Titer [mg/l]** | **Purity SE-HPLC [% monomer]** | **Purity SDS-PAGE** | **Tₘ [°C]** | **Tₒₙₛₑₜ [°C]** |
|---|---|---|---|---|---|---|---|---|---|
| PRO1510 | 44-18-C11-sc01 | 200 | 12.5 | 10.0 | 50 | 98.0 | identity confirmed | 71.5 | 64.0 |
| PRO1528 | 44-18-C11-sc04 | 100 | 5.7 | 4.7 | 47 | 99.0 | identity confirmed | 59.1 | 52.3 |
| PRO1549 | 44-18-C11-sc05 | 100 | 8.1 | 6.8 | 68 | 99.0 | identity confirmed | 66.9 | 61.0 |
| PRO1550 | 44-18-C11-sc06 | 100 | 8.0 | 9.0 | 90 | 99.0 | identity confirmed | 69.8 | 62.0 |
| PRO1551 | 44-18-C11-sc07 | 100 | 8.1 | 6.8 | 68 | 98.0 | identity confirmed | 68.1 | 62.7 |
| PRO1552 | 44-18-C11-sc08 | 100 | 6.8 | 5.5 | 55 | 98.0 | identity confirmed | 55.6 | 47.0 |
| PRO1553 | 44-18-C11-sc09 | 100 | 7.5 | 6.7 | 67 | 99.0 | identity confirmed | 62.8 | 56.0 |
| PRO1554 | 44-18-C11-sc10 | 100 | 8.8 | 6.9 | 69 | 99.0 | identity confirmed | 69.5 | 61.3 |

### 1.8. Pharmacodynamic characterization of suitable anti-IL-4R binding domains (scFvs format)

The humanized scFv antibodies that were evaluated as suitable were characterized for primary pharmacodynamics properties.

### Affinity to human IL-4R

Affinity of the 16 humanized scFvs (derived from 5 rabbit monoclonal antibodies) to human IL-4R was determined by SPR analysis on a T200 device (Biacore, GE Healthcare). Human IL-4R-Fc was captured via an anti-human IgG-Fc coupled to a carboxylmethylated dextran surface and the scFvs were injected as analyte. After each analyte injection cycle the sensor chip was regenerated and new antigen was captured. The scFvs were measured using a dose response multi-cycle kinetic assay with seven analyte concentrations ranging from 0.12 to 90 nM diluted in running buffer. Obtained sensorgrams were fitted using a 1:1 binding model. The results are summarized in Table 8.

**Table 8: Affinity of scFvs to human IL-4R. Sc01: CDR graft, sc04: Full graft, sc05 to sc10: other grafting variants**

| **Protein ID** | **scFv** | **Affinity to human IL-4R** | | |
|---|---|---|---|---|
| | Clone ID | ka (M⁻¹ s⁻¹) | kd (s⁻¹) | K_{D} (M) |
| PRO1506 | 44-03-A03-sc01 | 2.17E+06 | 1.49E-04 | 6.89E-11 |
| PRO1524 | 44-03-A03-sc04 | 1.13E+06 | 3.40E-05 | 3.00E-11 |
| PRO1510 | 44-18-C11-sc01 | 1.67E+06 | 9.58E-04 | 5.75E-10 |
| PRO1528 | 44-18-C11-sc04 | 3.83E+06 | 2.07E-04 | 5.39E-11 |
| PRO1549 | 44-18-C11-sc05 | 1.63E+06 | 1.11E-03 | 6.77E-10 |
| PRO1550 | 44-18-C11-sc06 | 1.55E+06 | 1.32E-03 | 8.52E-10 |
| PRO1551 | 44-18-C11-sc07 | 1.84E+06 | 1.05E-03 | 5.70E-10 |
| PRO1552 | 44-18-C11-sc08 | 8.41 E+06 | 3.54E-04 | 4.21 E-11 |
| PRO1553 | 44-18-C11-sc09 | 4.24E+06 | 2.24E-04 | 5.27E-11 |
| PRO1554 | 44-18-C11-sc10 | 2.03E+06 | 1.09E-03 | 5.37E-10 |
| PRO1515 | 44-32-F04-sc01 | 4.89E+06 | 3.50E-05 | 7.15E-12 |
| PRO1533 | 44-32-F04-sc04 | 2.64E+06 | 4.66E-05 | 1.77E-11 |
| PRO1517 | 44-34-C10-sc01 | 3.55E+06 | 1.95E-05 | 5.50E-12 |
| PRO1535 | 44-34-C10-sc04 | 2.72E+06 | 5.42E-05 | 1.99E-11 |
| PRO1520 | 44-39-B07-sc01 | 6.18E+05 | 1.18E-02 | 1.91 E-08 |
| PRO1538 | 44-39-B07-sc04 | 1.67E+06 | 2.29E-04 | 1.37E-10 |

As can be seen from Table 8, the scFv antibody variable domains based on clone 44-34-C10 exhibit the best overall binding affinities to human IL-4R, closely followed by scFv antibody variable domains based on clone 44-32-F04.

### Affinity to cynomolgus monkey, marmoset monkey and mouse IL-4R

Cross-reactivity to cynomolgus IL-4R was measured using the same setup as for the analysis of human IL-4R affinities, with the difference that cynomolgus IL-4R-Fc was captured instead of human IL-4R-Fc. To determine affinities for marmoset IL-4R, a direct setup was used since only extracellular domain (ECD) of marmoset IL-4R was available. Marmoset ECD was immobilized directly onto the sensor chip and a titration series of scFvs was injected as analyte. To allow for comparison between K_{D} values obtained for cynomolgus and marmoset IL-4R, affinities to cynomolgus IL-4R ECD were also determined using a direct setup.

Only few scFvs bound to cynomolgus IL-4R. Four scFvs showed cross-reactivity in the capture setup compared to two scFvs in the direct setup, meaning that the binding of two scFvs was no longer measurable in the direct setup. This is most likely a consequence of lower K_{D} values determined in the direct setup. The difference was due to the on-rates, which were slower, possibly due to lower accessibility. Off-rates were comparable for all scFvs. Furthermore, cross-reactivity to marmoset IL-4R ECD was determined. The results are summarized in Table 9. As can be seen from Table 9, the scFv antibody variable domains based on clones 44-34-C10 and 44-32-F04 exhibit excellent cross reactivity to cynomolgus IL-4R ECD and marmoset IL-4R ECD.

Binding to mouse IL-4R was also assessed for scFvs PRO1517, PRO1535 and PRO1538 but none of them showed cross-reactivity.

### Stat-6 reporter gene assay in HEK-Blue cells (blockade of human IL-4- and IL-13-induced signaling)

The HEK-Blue assay tested the ability of the humanized scFvs to inhibit IL-4- and IL-13-induced signaling via the IL-4R. 50,000 cells per well were seeded in a 96-well plate. Serial dilution of the scFvs and of the control antibody dupilumab were added to the plates in presence of either 0.02 ng/ml IL-4 or 0.3 ng/ml IL-13. After 24 h incubation at 37°C and 5 % CO₂, supernatants were transferred to a new plate and secreted embryonic alkaline phosphatase was quantified using the Quanti-Blue substrate.

The scFvs were analyzed in this HEK-Blue assay for their ability to block IL-4R mediated signaling. The potency of the analyzed molecules was compared to dupilumab. All potency data are summarized in Table 10. Representative dose-response curves of PROL515, PRO1517, PRO1524, PRO1535 and PRO1552 for blocking IL-4-induced signaling and IL-13-induced signaling, respectively, are shown in Figures 1 and 2. Relative IC₅₀ values were calculated in mass units (ng/ml) of dupilumab and the scFvs. 10 scFvs blocked IL-4- and IL-13-induced signaling with high potencies. The scFvs based on the clones 44-34-C10 and 44-32-F04, as well as PRO1552 showed best overall blockage potencies. PROL515, PRO1517, PRO1524, PRO1533, PRO1535, PRO1538 and PRO1552 could block IL-4- and IL-13-induced signaling with a potency similar to dupilumab.

### Competitive ELISA (inhibition of hIL-4 binding to hIL-4R)

Potency of humanized scFvs was further determined using a competitive ELISA. The potency of each scFv to inhibit the interaction between human IL-4 and human IL-4R was assessed by ELISA using the same procedure as described above. Similarly, to the stat-6 reporter gene assay, individual IC₅₀ values on each plate were calibrated against the IC₅₀ of the reference molecule dupilumab.

The blockage data are summarized in Table 11. Representative dose-response curves for PROL515, PRO1517, PRO1533, PRO1535 and PRO1538 are shown in Figure 3. Six scFvs inhibited the interaction between human IL-4 and human IL-4R with good to high potency. The scFvs based on the clones 44-34-C10 and 44-32-F04 as well as PRO1552 showed the best overall blockage potencies. The blocking potencies of PROL515, PRO1517 and PRO1552 were similar to dupilumab. On the other hand, PRO1538 showed only moderate inhibition. Five scFv molecules showed no inhibition.

### Summary of pharmacological characterization of scFvs and molecule selection for developability assessment

The pharmacological characterization of 16 scFvs derived from five different B cell clones provided the basis for selection of molecules for extended biophysical characterization. Seven molecules, namely PROL515, PRO1517, PRO1533, PRO1535, PRO1524, PRO1506 and PRO1538 were selected for further extended characterization based on their overall better performance in terms of neutralization of IL-4- and IL-13-induced signaling in the stat-6 reporter gene assay and in the IL-4/IL-4R blocking ELISA. All seven molecules showed affinities better than 500 pM. The affinity to cynomolgus monkey IL-4R was lower by a factor of between 4- and 20-fold when compared to human IL-4R, except for three molecules, which showed very low affinity to cynomolgus IL-4R or no binding. In particular, the scFvs based on clone 44-18-C11, *i*. *e*. PRO1510, PRO1528 and PRO1549 to PRO1554, showed no binding to cynomolgus IL-4R. Generally, also the affinities of these scFvs to marmoset monkey IL-4R were lower when compared to human IL-4R.

As a result of the affinity studies, the scFvs based on clone 44-18-C11, *i*. *e*. PRO1510, PRO1528 and PRO1549 to PRO1554 were excluded from further evaluation due to their lack of cross-reactivity to cynomolgus IL-4R, even though PRO1552 showed vey good inhibition potencies. For the same reasons, PRO1520 was excluded as well. PRO1538 however, which is based on the same clone 44-39-B07 was not excluded, despite its weak binding affinity towards cynomolgus IL-4R.

**Table 9: Affinity of the scFvs to cynomolgus and marmoset monkey IL-4R. Sc01: CDR graft, sc04: Full graft, sc05 to sc10: other grafting variants**

| **Protein ID** | **scFv** | **Affinity to cynomolgus IL-4R capture setup** | | | **Affinity to cynomolgus IL-4R direct setup** | | | **Affinity to marmoset IL-4R direct setup** | | |
|---|---|---|---|---|---|---|---|---|---|---|
| | Clone **ID** | ka (M⁻¹ s⁻¹) | k_{d} (s⁻¹) | K_{D} (M) | ka (M⁻¹ s⁻¹) | k_{d} (s⁻¹) | K_{D} (M) | ka (M⁻¹ s⁻¹) | k_{d} (s⁻¹) | K_{D} (M) |
| PRO1506 | 44-03-A03 -sc01 | NB | NB | NB | NB | NB | NB | 3.78E+06 | 7.45E-04 | 1.97E-10 |
| PRO1524 | 44-03-A03-sc04 | 1.37E+09 | 8.07E+01 | 5.89E-08 | NB | NB | NB | 2.27E+06 | 2.06E-04 | 9.09E-11 |
| PRO1510 | 44-18-C11-sc01 | NB | NB | NB | NA | NA | NA | NA | NA | NA |
| PRO1528 | 44-18-C11-sc04 | NB | NB | NB | NB | NB | NB | 6.43E+06 | 4.39E-03 | 6.83E-10 |
| PRO1549 | 44-18-C11-sc05 | NB | NB | NB | NA | NA | NA | NA | NA | NA |
| PRO1550 | 44-18-C11-sc06 | NB | NB | NB | NA | NA | NA | NA | NA | NA |
| PRO1551 | 44-18-C11-sc07 | NB | NB | NB | NA | NA | NA | NA | NA | NA |
| PRO1552 | 44-18-C11-sc08 | NB | NB | NB | NB | NB | NB | 1.57E+07 | 1.28E-03 | 8.16E-11 |
| PRO1553 | 44-18-C11-sc09 | NB | NB | NB | NB | NB | NB | 6.64E+06 | 5.38E-03 | 8.09E-10 |
| PRO1554 | 44-18-C11-sc10 | NB | NB | NB | NA | NA | NA | NA | NA | NA |
| PRO1515 | 44-32-F04-sc01 | 4.29E+06 | 2.74E-04 | 6.39E-11 | 1.21 E+06 | 2.04E-04 | 1.69E-10 | 4.15E+06 | 5.40E-04 | 1.30E-10 |
| PRO1533 | 44-32-F04-sc04 | 2.28E+06 | 1.78E-04 | 7.81 E-11 | 7.02E+05 | 1.54E-04 | 2.19E-10 | 1.84E+06 | 1.26E-03 | 6.86E-10 |
| PRO1517 | 44-34-C10-sc01 | 1.92E+06 | 1.82E-04 | 9.46E-11 | 5.94E+05 | 4.04E-04 | 6.81E-10 | 2.67E+06 | 1.05E-03 | 3.92E-10 |
| PRO1535 | 44-34-C10-sc04 | 1.60E+06 | 2.64E-04 | 1.65E-10 | 6.34E+05 | 2.38E-04 | 3.76E-10 | 3.23E+06 | 2.54E-03 | 7.87E-10 |
| PRO1520 | 44-39-B07-sc01 | NB | NB | NB | NA | NA | NA | NA | NA | NA |
| PRO1538 | 44-39-B07-sc04 | 5.29E+04 | 8.06E-04 | 1.52E-08 | NB | NB | NB | 1.02E+06 | 1.83E-04 | 1.80E-10 |

| | | | | | | | | | | |
|---|---|---|---|---|---|---|---|---|---|---|
| NA: not analyzed; NB: no binding | | | | | | | | | | |

**Table 10: Potencies of the scFvs to neutralize IL-4- and IL-13-induced signaling in the stat-6 reporter gene assay. Sc01: CDR graft, sc04: Full graft, sc05 to sc10: other grafting variants**

| **Protein ID** | **scFv** | **Neutralization of hIL-4-induced signaling in reporter gene assay** | | **Neutralization of hIL-13-induced signaling in reporter gene assay** | |
|---|---|---|---|---|---|
| | | IC₅₀ [ng/ml] | rel. IC₅₀* | IC₅₀ [ng/ml] | rel. IC₅₀* |
| PRO1506 | 44-03-A03 -sc01 | 0.93 | 0.78 | 2.06 | 0.98 |
| PRO1524 | 44-03-A03-sc04 | 0.95 | 0.80 | 1.81 | 1.05 |
| PRO1510 | 44-18-C11-sc01 | no inhibition | n/a | no inhibition | n/a |
| PRO1528 | 44-18-C11-sc04 | 1.49 | 0.28 | 2.29 | 0.59 |
| PRO1549 | 44-18-C11-sc05 | no inhibition | n/a | no inhibition | n/a |
| PRO1550 | 44-18-C11-sc06 | no inhibition | n/a | no inhibition | n/a |
| PRO1551 | 44-18-C11-sc07 | no inhibition | n/a | no inhibition | n/a |
| PRO1552 | 44-18-C11-sc08 | 0.43 | 1.30 | 1.02 | 1.41 |
| PRO1553 | 44-18-C11-sc09 | 1.19 | 0.47 | 6.05 | 0.24 |
| PRO1554 | 44-18-C11-sc10 | no inhibition | n/a | no inhibition | n/a |
| PRO1515 | 44-32-F04-sc01 | 1.36 | 0.42 | 2.03 | 0.52 |
| PRO1533 | 44-32-F04-sc04 | 1.27 | 0.70 | 2.35 | 0.95 |
| PRO1517 | 44-34-C10-sc01 | 1.44 | 0.39 | 1.83 | 0.57 |
| PRO1535 | 44-34-C10-sc04 | 1.15 | 1.02 | 2.30 | 0.99 |
| PRO1520 | 44-39-B07-sc01 | NA | NA | NA | NA |
| PRO1538 | 44-39-B07-sc04 | 0.64 | 1.83 | 1.69 | 1.35 |

| | | | | | |
|---|---|---|---|---|---|
| NA: not analyzed * IC₅₀, dupilumab/IC₅₀, test sample n/a: relative IC₅₀ not calculated (no inhibition or IC₅₀ not determined) | | | | | |

**Table 11: Potencies of scFvs to inhibit the interaction between IL-4R and IL-4. Sc01: CDR graft, sc04: Full graft, sc05 to sc10: other grafting variants**

| **Protein ID** | **scFv** | **Potency in IL-4/IL-4R ELISA** | |
|---|---|---|---|
| | Clone ID | IC₅₀ [ng/ml] | reL.IC50* |
| PRO1506 | 44-03-A03 -sc01 | 1.60 | 2.71 |
| PRO1524 | 44-03-A03-sc04 | 1.25 | 1.87 |
| PRO1510 | 44-18-C11-sc01 | 18.90 | 0.10 |
| PRO1528 | 44-18-C11-sc04 | 0.79 | 2.28 |
| PRO1549 | 44-18-C11-sc05 | no inhibition | n/a |
| PRO1550 | 44-18-C11-sc06 | no inhibition | n/a |
| PRO1551 | 44-18-C11-sc07 | no inhibition | n/a |
| PRO1552 | 44-18-C11-sc08 | 0.58 | 2.91 |
| PRO1553 | 44-18-C11-sc09 | 2.36 | 0.72 |
| PRO1554 | 44-18-C11-sc10 | no inhibition | n/a |
| PRO1515 | 44-32-F04-sc01 | 0.68 | 2.87 |
| PRO1533 | 44-32-F04-sc04 | 1.74 | 1.07 |
| PRO1517 | 44-34-C10-sc01 | 0.54 | 3.46 |
| PRO1535 | 44-34-C10-sc04 | 1.92 | 0.98 |
| PRO1520 | 44-39-B07-sc01 | no inhibition | n/a |
| PRO1538 | 44-39-B07-sc04 | 10.71 | 0.26 |

| | | | |
|---|---|---|---|
| *: IC₅₀, dupilumab/IC₅₀ test sample n/a: relative IC₅₀ not calculated (no inhibition or IC₅₀ not determined) | | | |

### 1.9. Biophysical characterization of suitable anti-IL-4R binding domains (scFv format)

### Manufacture of material for stability measurements

PROL515, PRO1517 and PRO1538 were produced again using the same manufacturing process as described above at slightly larger scale (0.25 l expression volume) to generate sufficient material for stability assessment. For other proteins which were selected for stability assessment, previously produced material amount was sufficient to perform stability assessment. Samples were formulated in 50 mM phosphate-citrate buffer with 150 mM NaCl at pH 6.4 (50 mM NaCiP, pH 6.4) after purification. After purification and dialysis, protein samples were concentrated to ≥10 mg/ml using centrifugal concentration tubes with 5 MWCO as this is the desired concentration to perform storage stability assessment.

Monomer loss upon concentration to 10 mg/ml was between 0 and 12 % depending on the molecule as shown in Table 12. Monomeric content of PRO1506, PROL515, PRO1524 and PRO1533 dropped below 95 % which is a critical value for molecules entering storage stability assessment and, therefore, a reason to exclude them from the study. Nevertheless, as the material was already prepared and everything was set up for the study, stability of those molecules was assessed alongside with remaining molecules which exhibited a monomeric content >95 % post concentration. The scFvs based on clone 44-34-C10, *i*. *e*. PRO1517 and PRO1535, as well as PRO1538 showed virtually no loss of the monomeric content upon concentration.

**Table 12: % monomer loss (SE-HPLC) upon concentration to 10 mg/ml. Sc01: CDR-graft, sc04: Full graft**

| **Protein ID** | **Clone ID** | **Initial monomer content [%]** | **Monomer content post concentration to 10mg/ml [%]** | **% loss upon concentration to 10 mg/ml** |
|---|---|---|---|---|
| PRO1506 | 44-03-A03 -sc01 | 99.0 | 92.5 | 6.6 |
| PRO1515 | 44-32-F04 -sc01 | 98.8 | 93.3 | 5.6 |
| PRO1517 | 44-34-C10 -sc01 | 97.6 | 97.4 | 0.2 |
| PRO1524 | 44-03-A03-sc04 | 99.0 | 87.3 | 11.8 |
| PRO1533 | 44-32-F04-sc04 | 99.1 | 88.3 | 10.9 |
| PRO1535 | 44-34-C10-sc04 | 99.1 | 99.0 | 0.1 |
| PRO1538 | 44-39-B07-sc04 | 95.0 | 98.2 | -3.4 |

### Storage stability study

Humanized scFvs were subjected to stability studies such as a four-week stability study, in which the scFvs were formulated in an aqueous buffer (50 mM NaCiP, 150 mM NaCl, pH 6.4) at 10 mg/ml and stored at < -80°C, 4°C and 40°C for four weeks. At the minimum, the fractions of monomers and oligomers in the formulation were evaluated by integration of SE-HPLC peak areas after one week, two weeks and at the end of each study. Additional time points were recorded for most of the molecules. Table 13 compares d14 (day 14) and endpoint measurements obtained at d28 (day 28) of the study.

As can be depicted from Table 13, the scFvs based on clone 44-34-C10, *i*. *e*. PRO1517 and PRO1535, showed a good storage stability at 4°C. The loss of monomeric content after storage at 4°C for d28 was less than 5 %. Also PRO1538 showed a good storage stability at least for 14d. On the other hand, PRO1515 exhibits a loss in monomeric content of >10 % for storage at 4°C, which reprersents a clear reason for exclusion. However, none of the stable molecules showed considerable loss of protein content at any temperature.

### Freeze-thaw stability

In addition to the storage stability study described above, the compatibility of the top performing scFv molecules with respect to freeze-thawing (F/T) cycles (colloidal stability) was assessed. As in the early development stages the drug substance is usually stored frozen, formulation and fill/finish processing will require some freezing and thawing operations.

For the F/T stability assessment the same analytical methods (SE-HPLC, SDS-PAGE) and parameters (% monomer content and % monomer loss) as for the storage stability study were applied to monitor the quality of the molecules over five F/T cycles. Table 14 illustrates the course of % monomer content loss over five F/T cycles. As no dedicated freeze-thaw study was performed, freeze-thaw data obtained with the -80°C samples of storage stability study, which was acquired over 28 days, is shown. As only four time points could be recorded for some of the proteins, also only four F/T-cycles were done with those proteins. As can be seen from Table 14, apart from PRO1515 and PRO1533, none of the other tested scFvs showed a considerable loss in monomeric content.

### Thermal unfolding

Thermal unfolding measurements of the five selected scFv constructs were performed using Differential Scanning Fluorimetry (DSF). Resulting midpoint of thermal unfolding (Tₘ) and onset temperature of unfolding calculated at 10 % of maximal signal (Tonset 10 %) values were determined by fitting of data to a Boltzmann equation. Table 15 summarizes calculated melting temperatures measured by DSF. As can be deduced from Table 15, the scFvs based on the clone 44-34-C10 exhibit high melting temperatures of above 63°C. Also, PRO1538 showed a high melting temperature.

**Table 13: 28d storage stability study at 10 mg/ml and temperatures of -80°C, 4°C and 40°C. Sc01: CDR graft, sc04: Full graft**

| **Protein ID** | **Clone ID** | **Temp. [°C]** | **monomer content [%]** | | | **% monomer change** | | **protein concentration [mg/ml]** | | | **% protein content change** | |
|---|---|---|---|---|---|---|---|---|---|---|---|---|
| | | | **d0** | **d14** | **d28** | **d14** | **d28** | **d0** | **d14** | **d28** | **d14** | **d28** |
| PRO1506 | 44-03-A03-sc01 | -80 | 92.5 | 91.9 | 94.1 | -0.6 | 1.7 | 10.1 | 9.8 | NA | -3.0 | NA |
| | | 4 | 92.5 | 92.3 | 92.2 | -0.3 | -0.3 | 10.1 | 9.4 | NA | -6.7 | NA |
| | | 40 | 92.5 | 91.7 | 92.1 | -0.9 | -0.5 | 10.1 | 9.7 | 10.3 | -3.7 | 2.0 |
| PRO1515 | 44-32-F04 -sc01 | -80 | 93.3 | 90.7 | 88.0 | -2.8 | -5.7 | 10.4 | 11.3 | 11.9 | 8.5 | 14.8 |
| | | 4 | 93.3 | 83.3 | 80.5 | -10.8 | -13.8 | 10.4 | 9.7 | 12.0 | -6.9 | 15.1 |
| | | 40 | 93.3 | 85.9 | 84.7 | -7.9 | -9.3 | 10.4 | 11.3 | 11.8 | 8.8 | 13.2 |
| PRO1517 | 44-34-C10-sc01 | -80 | 97.4 | 97.1 | 96.8 | -0.4 | -0.7 | 10.0 | 9.9 | 10.9 | -1.5 | 8.4 |
| | | 4 | 97.4 | 96.4 | 95.2 | -1.1 | -2.3 | 10.0 | 7.9 | 11.9 | -21.7 | 18.8 |
| | | 40 | 97.4 | 87.2 | 86.6 | -10.5 | -11.2 | 10.0 | 10.2 | 10.7 | 2.0 | 6.9 |
| PRO1524 | 44-03-A03-sc04 | -80 | 87.3 | 88.7 | 88.9 | 1.6 | 1.8 | 10.5 | 10.3 | 9.6 | -1.9 | -8.1 |
| | | 4 | 87.3 | 89.2 | 88.2 | 2.2 | 1.0 | 10.5 | 10.2 | 9.6 | -2.4 | -8.8 |
| | | 40 | 87.3 | 88.4 | 88.4 | 1.2 | 1.3 | 10.5 | 11.4 | 13.5 | 8.4 | 28.5 |
| PRO1533 | 44-32-F04-sc04 | -80 | 88.3 | 86.4 | 87.0 | -2.2 | -1.4 | 9.8 | NA | 7.0 | NA | -28.2 |
| | | 4 | 88.3 | 86.6 | 86.7 | -2.0 | -1.9 | 9.8 | NA | 10.4 | NA | 6.0 |
| | | 40 | 88.3 | 87.7 | 86.5 | -0.6 | -2.1 | 9.8 | NA | 11.6 | NA | 18.8 |
| PRO1535 | 44-34-C10-sc04 | -80 | 99.0 | 98.5 | 98.1 | -0.5 | -0.9 | 10.5 | 10.9 | 9.6 | 3.3 | -9.1 |
| | | 4 | 99.0 | 96.9 | 95.0 | -2.1 | -4.1 | 10.5 | 10.4 | 7.8 | -1.4 | -25.6 |
| | | 40 | 99.0 | 90.3 | 88.6 | -8.8 | -10.5 | 10.5 | 10.8 | 10.8 | 2.6 | 3.1 |
| PRO1538 | 44-39-B07-sc04 | -80 | 98.2 | 97.8 | NA | -0.3 | NA | 10.4 | 10.5 | NA | 0.6 | NA |
| | | 4 | 98.2 | 96.0 | NA | -2.2 | NA | 10.4 | 10.2 | NA | -2.0 | NA |
| | | 40 | 98.2 | 88.3 | NA | -10.0 | NA | 10.4 | 12.0 | NA | 15.2 | NA |

| | | | | | | | | | | | | |
|---|---|---|---|---|---|---|---|---|---|---|---|---|
| NA: not analyzed | | | | | | | | | | | | |

**Table 14: F/T stability - monomer content in % and % monomeric change upon repetitive freeze thawing. Sc01: CDR graft, sc04: Full graft**

| **Protein ID** | | **monomer content [%]** | | | | | | % monomer change | | | | |
|---|---|---|---|---|---|---|---|---|---|---|---|---|
| | | **F/T 0** | **F/T 1** | **F/T 2** | **F/T 3** | **F/T 4** | **F/T 5** | **F/T 1** | **F/T 2** | **F/T 3** | **F/T 4** | **F/T 5** |
| PRO1506 | 44-03-A03-sc01 | 92.51 | 92.4 | 92.6 | 91.9 | 94.1 | NA* | -0.1 | 0.1 | -0.6 | 1.7 | NA* |
| PRO1515 | 44-32-F04 -sc01 | 93.34 | 92.1 | 92.4 | 90.7 | 88.9 | 88.0 | -1.3 | -1.0 | -2.8 | -4.7 | -5.7 |
| PRO1517 | 44-34-C10-sc01 | 97.44 | 97.7 | 97.7 | 97.1 | 96.9 | 96.8 | 0.2 | 0.3 | -0.4 | -0.6 | -0.7 |
| PRO1524 | 44-03-A03-sc04 | 87.31 | 85.5 | 85.6 | 88.7 | 88.9 | NA* | -2.1 | -2.0 | 1.6 | 1.8 | NA* |
| PRO1533 | 44-32-F04-sc04 | 88.3 | 87.6 | 86.2 | 86.4 | 87.0 | NA* | -0.8 | -2.4 | -2.2 | -1.4 | NA* |
| PRO1535 | 44-34-C10-sc04 | 98.98 | 99.0 | 98.8 | 98.5 | 98.1 | NA* | 0.0 | -0.2 | -0.5 | -0.9 | NA* |
| PRO1538 | 44-39-B07-sc04 | 98.17 | 98.2 | 98.2 | 97.8 | 97.8 | NA* | 0.0 | 0.0 | -0.3 | -0.4 | NA* |

| | | | | | | | | | | | | |
|---|---|---|---|---|---|---|---|---|---|---|---|---|
| *not applicable as only 4 time points could be measured | | | | | | | | | | | | |

**Table 15: DSF of selected domains. Sc01: CDR graft, sc04: Full graft**

| **Protein ID** | **Description** | **Purity SE-HPLC [% monomer]** | **Tₘ [°C]** | **Tₒₙₛₑₜ [°C]** |
|---|---|---|---|---|
| PRO1506 | 44-03-A03-sc01 | 99.0 | 62.8 | 56.7 |
| PRO1515 | 44-32-F04 -sc01 | 98.8 | 59.0 | 53.0 |
| PRO1517 | 44-34-C10-sc01 | 97.6 | 63.7 | 57.0 |
| PRO1524 | 44-03-A03-sc04 | 99.0 | 59.6 | 54.0 |
| PRO1533 | 44-32-F04-sc04 | 99.1 | 62.2 | 56.7 |
| PRO1535 | 44-34-C10-sc04 | 99.1 | 67.9 | 62.7 |
| PRO1538 | 44-39-B07-sc04 | 95.0 | 68.3 | 63.7 |

### Example 2: Selection and optimization of anti-IL-4R molecules for multispecific format:

### 2.1. General remarks on the selection of anti-IL-4R domains for optimization

The anti-IL-4R domains PRO1517 (44-34-C10-sc01) and PRO1535 (44-34-C10-sc04) were selected in the first place, since they exhibit very good pharmacodynamic properties and a acceptable to good stability. PRO1538 (44-39-B07-sc04) was selected as well, albeit having improvable pharmacological properties, since it showed the best stability. PRO1517 (44-34-C10-sc01) was subjected to a stability optimization and PRO1538 (44-39-B07-sc04) was subjected to pl balance optimization, as shown below in 2.2.

The anti-IL-4R binding domains applied in the final multispecific antibodies showed exclusive cross-reactivity to cynomolgus and marmoset monkey.

### 2.2. Optimization of anti-IL-4R domains

The anti-IL-4R domains PRO1538 (44-39-B07-sc03) and PRO1517 (44-34-C10-sc01) were further optimized for assembly into multispecific formats.

### Optimization of anti-IL-4R domain 44-39-B07-sc04 (PRO1538)

PRO1538 (44-39-B07-sc03) has a pl imbalance between VL and VH (pl_{VL}=8.2 and pL_{VH}=5.0). Imbalance in pl correlates with high viscosity and could negatively influence stability. Therefore, two new variants of this molecule were designed. These variants are described below. Briefly, all residues in VH have been analyzed in detail with the aim to design molecules with removed pl imbalance and without compromising binding affinity and without introducing critical sequence liabilities (chemical and post-translational modifications), T cell epitopes *etc.*

Two variants were designed, which are summarized in Table 16.

**Table 16: Optimization variants for 44-39-B07-sc03**

| **Protein ID** | **Construct** | **Optimization Aim** | **Mutations VL** | **Mutations VH** |
|---|---|---|---|---|
| PRO2097 | 44-39-B07-sc05 | pl balance | I2V, A51P, P75S, G82R, F89Y | V2E, A25R, R82S, Y105F, T143R |
| PRO2098 | 44-39-B07-sc06 | pl balance | I2V, A51P, P75S, G82R, F89Y | V2E, A24R, R82S, Y105F, T143R |

These variants of PRO1538 (44-39-B07-sc03) did, however, not show the desired improvement of stability. Since PRO1538 is significantly less potent in inhibiting IL-4- and IL-13-induced signaling, and has a more than 10-fold lower potency in blocking the interaction of IL-4 to IL-4R than PRO1517, PRO1538 and its variants were excluded from further consideration and efforts were focused on the improvement of RPO1517 (44-34-C10-sc01).

### Optimization of anti-IL-4R domain 44-34-C10-sc01 (PRO1517)

PRO1517 (44-34-C10-sc01) is an anti-IL-4R scFv with favorable binding characteristics. During biophysical characterization the scFv PRO1517 exhibited good but yet still improvable solubility properties. Efforts were made to further improve PRO1517 with respect to solubility, long-term stability and concentration behavior. Therefore, four new variants of this molecule were designed. These variants are described below. Briefly, hydrophobic patches in CDRs or in the former VH-CH interface have been analyzed in detail and pl imbalance has been reduced with the aim to design molecules with removed hydrophobic patches without compromising binding affinity and without introducing critical sequence liabilities (chemical and post-translational modifications), T cell epitopes *etc.*

Four variants were designed, which are summarized in Table 17.

**Table 17: Optimization variants for 44-34-C10-sc01**

| **Protein ID** | **Construct** | **Optimization Aim** | **Mutations VL** | **Mutations VH** |
|---|---|---|---|---|
| PRO1896 | 44-34-C10-sc06 | Solubility, stability | | L12R, V103T, L144Q |
| PRO1897 | 44-34-C10-sc07 | Solubility, stability | Y72S | |
| PRO1898 | 44-34-C10-sc08 | Solubility, stability | S14R, T87R | |
| PRO1899 | 44-34-C10-sc09 | Solubility, stability, affinity | | L12R, V103T, L144Q + RDNSKN changed to KASST (AHo 82-87) |

### 2.3. Biophysical characterization of optimized anti-IL-4R binding domains (scFv format)

### Storage stability study

PRO1897, PRO1898 and PRO1899 were subjected to storage stability studies as decribed above in section 1.9. The results are summarized in Table 18.

As can be depicted from Table 19, the scFvs PRO1898 and PRO1899 exhibits excellent storage stability. The loss of monomeric content after storage at 4°C for d28 was less than 5 %. Also, none of these molecules showed considerable loss of protein content at any temperature.

### Thermal unfolding

Thermal unfolding measurements for PRO1898 and PRO1899 have been performed as decribed above in section 1.9. The results are summarized in Table 19. As can be deduced from Table 19, PRO1898 and PRO1899 exhibit high melting temperatures.

**Table 18: 28d storage stability study at 10 mg/ml and temperatures of -80°C, 4°C and 40°C.**

| **Protein ID** | **Clone ID** | **Temp. [°C]** | **monomer content [%]** | | | **% monomer loss** | | **protein concentration [mg/ml]** | | | **% protein content loss** | |
|---|---|---|---|---|---|---|---|---|---|---|---|---|
| | | | **d0** | **d1** | **d28** | **d1** | **d28** | **d0** | **d1** | **d28** | **d1** | **d28** |
| PRO1897 | 44-34-C10-sc07 | -80 | 97.7 | 97.7 | NA | -0.1 | NA | 10.2 | 10.5 | NA | 2.2 | NA |
| | | 4 | 97.7 | 98 | 97 | -0.1 | -0.3 | 10.2 | 10 | 11 | 0.3 | 3.6 |
| | | 40 | 97.7 | 95 | 87 | -2.6 | -10.8 | 10.2 | 11 | 13 | 2.6 | 30.0 |
| PRO1898 | 44-34-C10-sc08 | -80 | 97.4 | 97.3 | NA | -0.1 | NA | 10.2 | 10.4 | NA | 1.8 | NA |
| | | 4 | 97.4 | 97 | 96 | -0.1 | -1.1 | 10.2 | 10 | 10 | -0.3 | -1.7 |
| | | 40 | 97.4 | 93 | 86 | -4.4 | -11.8 | 10.2 | 10 | 11 | -1.7 | 9.7 |
| PRO1899 | 44-34-C10-sc09 | -80 | 98.1 | 98.0 | NA | 0.0 | NA | 10.5 | 10.7 | NA | 1.9 | NA |
| | | 4 | 98.1 | 98 | 98 | -0.1 | 0.0 | 10.5 | 10 | 11 | -0.2 | 1.8 |
| | | 40 | 98.1 | 97 | 84 | -0.6 | -14.3 | 10.5 | 11 | 13 | 2.9 | 28.8 |

| | | | | | | | | | | | | |
|---|---|---|---|---|---|---|---|---|---|---|---|---|
| NA: not analyzed | | | | | | | | | | | | |

**Table 19: DSF of optimized domains.**

| **Protein ID** | **Description** | **Purity SE-HPLC [% monomer]** | **Tₘ [°C]** | **Tₒₙₛₑₜ [°C]** |
|---|---|---|---|---|
| PRO1898 | 44-34-C10-sc08 | 97.4 | 73.1 | 65.5 |
| PRO1899 | 44-34-C10-sc09 | 98.1 | 72.3 | 64.1 |

### Example 3: Anti-IL-4R x IL-31 bispecific antibodies based on Morrison-H IgG4

It was then further tested whether the anti-IL-4R antibody variable domains of the present invention also provides advantageous biological and biophysical properties when incorporated into multispecific antibody formats. Therefore, multispecific antibodies were designed based on Morrison-H IgG4 format comprising two anti-IL-4R antibody variable domains, as defined herein. For the binding domains, which specifically binds to a target different from IL-4R, two IL-31 binding domains (IL31-BDs) were selected.

### 3.1. Morrison format design

A series of anti-IL-4R x IL-31 bispecific antibodies were designed having a Morrison-H format, wherein the Fc region is derived from the IgG subclass IgG4.

Due to their cross-reactivity to cynomolgus monkey IL-4R, their excellent potency in blocking IL-4R-mediated signalling and their excellent biophysical properties, the anti-IL-4R scFv variable domains 44-34-C10-sc08 and 44-34-C10-sc09 were selected for the Fab-arm binding domains of the Morrison-H constructs. Likewise, due to their excellent biological and biophysical properties, the anti IL-31 scFv 50-09-D07-sc04 was selected for the scFv-domains that are fused to the C-terminus of the heavy chain of the Morrison-H antibodies.

The identification, humanization, production and final selection of the humanized anti-IL-31 binding domain 50-09-D07-sc04 was performed using the same methodology as described above for the anti-IL-4R antibody variable domains.

Two IgG4-(scFv)₂ Morrison-H molecules were designed, *i*. *e*. PRO2198 and PRO2199, as shown in Table 20 (Morrison-H).

**Table 20: IgG4 Morrison-H constructs**

| **PRO ID** | **Fab-arm domain** | **Constant** | **scFv-domain** |
|---|---|---|---|
| PRO2198 | 44-34-C10-sc08 | IgG4 (S228P) | 50-09-D07-sc04 |
| PRO2199 | 44-34-C10-sc09 | IgG4 (S228P) | 50-09-D07-sc04 |

Expression of multispecific antibodies PRO2198 and PRO2199 was performed in FreeStyle CHO-S cells using the transient CHOgro expression system (Mirus). The genes of interest were optimized for mammalian expression, synthesized and cloned into a standard pcDNA3.1 vector. Expression cultures were cultivated in batch using shaking flasks for 6 to 7 days (cell viability < 70 %) at 37°C. The culture supernatants were separated from cells by centrifugation followed by a 0.22 µm sterile filtration. The target proteins were captured from the clarified culture supernatants by Protein L or A affinity chromatography followed by a size-exclusion chromatography polishing step (in case no fractions with a suitable monomeric content as assessed by SE-HPLC were available post capture already). For the quality control of the manufactured material standard analytical methods, such as SE-HPLC, SDS-PAGE, and UV₂₈₀ were used.

### 3.2. Affinity to human and cyno IL-4R

Binding kinetics (including affinity) of the bispecific Morrison-H antibodies PRO2198, PRO2199 to recombinant human IL-4R protein (ECD with His Tag, Sino Biological) were determined by SPR analysis on a T200 device (Biacore, Cytiva). In this SPR experiment, Morrison molecules were captured via an anti-human IgG-Fc antibody (Human Antibody Capture Kit; Biacore, Cytiva) covalently immobilized to a carboxylmethylated dextran surface (CM5 sensorchip; Biacore, Cytiva) and a titration series of human IL-4R ECD was injected as analyte. After each analyte injection cycle, the sensor chip was regenerated (MgCl₂), and new Morrison antibody was subsequently re-captured. The binding kinetics to human IL-4R was measured using a multi-cycle kinetic assay, with nine analyte concentrations ranging from 0.175 to 45 nM diluted in running buffer (HEPES buffered saline, 0.05 % Tween-20, pH 7.5). The apparent dissociation (k_{d}) and association (kₐ) rate constants and the apparent dissociation equilibrium constant (K_{D}) were calculated with the Biacore analysis software (Biacore Evaluation software Version 3.2, Cytiva) using one-to-one Langmuir binding model and quality of the fits was monitored based on Chi2 and U-value, which are measures for the quality of the curve fitting. The binding level was calculated as the maximum stability binding achieved normalized to the theoretical Rmax. Cross-reactivity to cynomolgus monkey IL-4R was measured using the same setup as for the analysis of human IL-4R kinetics, with the difference that recombinant cynomolgus monkey IL-4R (ECD with His-tag, Acro Biosystems) protein was used as analyte instead of human IL-4R.

As shown in Table 21 high affinity binding to human IL-4R and cynomolgus IL-4R was demonstrated for the Morrison-H antibodies PRO2198 and PRO2199.

**Table 21: Binding kinetics of Morrison antibodies to human IL-4R and cynomolgus monkey IL-4R**

| **Protein ID** | **Fab arm** | **scFv domain** | **Affinity by SPR measurement** | | | |
|---|---|---|---|---|---|---|
| | Clone ID | Clone ID | Antigen | ka (M⁻¹ s⁻¹) | k_{d} (s⁻¹) | K_{D} (M) |
| PRO2198 | 44-34-C10-sc08 | 50-09-D07-sc04 | human IL-4R | 3.52E+05 | 1.01 E-04 | 2.91 E-1 0 |
| PRO2199 | 44-34-C10-sc09 | 50-09-D07-sc04 | human IL-4R | 2.93E+05 | 5.76E-05 | 1.97E-10 |
| PRO2198 | 44-34-C10-sc08 | 50-09-D07-sc04 | cynomolgus IL-4R | 3.70E+05 | 4.90E-05 | 1.44E-10 |
| PRO2199 | 44-34-C10-sc09 | 50-09-D07-sc04 | cynomolgus IL-4R | 9.80E+05 | 3.44E-05 | 3.51 E-11 |

### 3.3. Potency assessment to inhibit human IL-4 and IL-13 signaling (HEK-Blue^{™} IL-4/IL-13 Cells stat-6 reporter gene assay)

The HEK-Blue assay has been used to test the ability of the Morrison-H antibodies PRO2198 and PRO2199 to inhibit IL-4- and IL-13-induced signaling via IL-4R. 50,000 cells per well were seeded in a 96-well plate. Three-fold serial dilutions of the Morrison molecules and of the reference antibody dupilumab at concentrations ranging from 100 to 0.002 ng/ml were added to the plates in presence of either 0.05 ng/ml IL-4 or 0.3 ng/ml IL-13. After 24 h incubation at 37°C and 5% CO₂, supernatants were transferred to a new plate and secreted embryonic alkaline phosphatase was quantified using the Quanti-Blue substrate. The Morrison-H antibodies PRO2198 and PRO2199 were also tested for inhibition of IL-4-induced signaling with an excess of IL-31 (10 nM) in the assay medium.

All potency data are summarized in Table 22 (IL-4/IL-4R inhibition) and Table 23 (IL-13/IL-4R inhibition). Mean relative IC₅₀ values from repeated analyses are also indicated in pM. Morrison-H antibodies PRO2198 and PRO2199 efficiently blocked the interaction between IL-4 or IL-13 and IL-4R, with comparable potency to dupilumab.

**Table 22: Potencies of the Morrison-H antibodies to neutralize human IL-4-induced signaling in the stat-6 reporter gene assay without and with IL-31 in the assay medium. n= number of repeated analyses.**

| | | | **Mean IC₅₀** | **Mean rel. IC₅₀** | **Retained IC₅₀ with and without IL-31 excess (n=1)** |
|---|---|---|---|---|---|
| **PRO ID** | **Fab-arm domain** | **scFv-domain** | **[pM]** | **[IC₅₀ dupilumab/ IC₅₀ sample]** | **[IC₅₀, no IL-31/IC₅₀, with IL-31] ng/ml values** |
| PRO2198 | 44-34-C10-sc08 | 50-09-D07-sc04 | 23.9 | **0.42** | 0.71 |
| PRO2199 | 44-34-C10-sc09 | 50-09-D07-sc04 | 11.5 | **0.72** | 1.25 |
| * IC₅₀ or relative IC₅₀ (single analysis) | | | | | |

**Table 23: Potencies of the Morrison-H molecules to neutralize human IL-13 induced signaling in the stat-6 reporter gene assay. n= number of repeated analyses.**

| | | | **Mean IC₅₀** | **Mean rel. IC₅₀** |
|---|---|---|---|---|
| **PRO ID** | **Fab-arm domain** | **scFv-domain** | **[pM]** | **[ICso dupilumab/ IC₅₀ sample]** |
| PRO2198 | 44-34-C10-sc08 | 50-09-D07-sc04 | 22.1 | **0.42** |
| PRO2199* | 44-34-C10-sc09 | 50-09-D07-sc04 | 11.3 | **0.69** |
| * **IC₅₀** or relative **IC₅₀** (single analysis) | | | | |

### 3.4. Biophysical characterization of PRO2198 and PRO2199:

### 3.4.1. Thermal stability

PRO2198 and PRO2199 were analyzed for their thermal stability between pH 5 and pH 8.5. Both thermal unfolding as well as the onset of thermal aggregation were determined. During thermal unfolding, all molecules showed more than one transition due to the multi-domain architecture of the molecules. For simplicity, only the first melting midpoint is shown. Table 24 summarizes the results.

**Table 24: Thermal stability**

| **pH** | **PRO2198** | **PRO2199** |
|---|---|---|
| Onset of unfolding (Tₒₙ) by nDSF [°C] | | |
| 5 | 55.4 | 55.1 |
| 7 | 60.5 | 59.3 |
| 8.5 | 59.7 | 58.3 |

| Midpoint of the first unfolding transition (Tₘ₁) by nDSF [°C] | | |
|---|---|---|
| 5 | 62.9 | 62.8 |
| 7 | 66.9 | 66.7 |
| 8.5 | 66.5 | 66.2 |

| Onset of aggregation (T_{scattering}) by nDSF [°C] | | |
|---|---|---|
| 5 | No aggregation | observed |
| 7 | 73.5 | 71.6 |
| 8.5 | 71.4 | 68.4 |

The onset of unfolding (Tₒₙ) as well as the first melting point (Tₘ₁) was highest at pH 7 for all molecules. A change to pH 8.5 decreased the thermal stability only slightly. Towards acidic pH, the thermal stability decreased. However, at pH 5 all onsets of unfolding were above 55°C.

Thermal aggregation was only observed at pH 7 and pH 8.5. At pH 5.0, both Morrison antibodies did not form larger aggregates, even in the unfolded state. At neutral and basic pH, the aggregation onset was above the first melting point, suggesting a non-native aggregation mechanism.

### 3.4.2. High concentration stability study

For the high concentration stability tests PRO2198 was formulated in two formulation buffers:
Formulation buffer F1: 20 mM acetate at pH 5.5;
Formulation buffer F2: 20 mM citrate with 50 mM NaCl at pH 5.5.

### Solubility - Protein concentration

PRO2198 could be concentrated above 100 mg/ml, without signs of precipitation or reaching the limit of solubility. Furthermore, PRO2198 showed no detectable decrease in protein concentration, *i*. *e*. PRO2198 was sufficiently soluble to reach and keep a target concentration of above 100 mg/ml for at least four weeks at 4°C and 25°C.

### Monomer stability at different temperatures

PRO2198 was formulated in F1 and F2 and concentrated to >100 mg/ml. The concentrated samples were stored at 4°C, 25°C and 40°C for up to 4 weeks. At different time points, the monomeric content was analyzed by means of SE-HPLC. The results are summarized in Table 25.

**Table 25: Monomer stability for PRO2198 at a concentration of >100 mg/ml**

| **Storage temperature [°C]** | **time [weeks]** | **Formulation F1** | | **Formulation F2** | |
|---|---|---|---|---|---|
| | | **Monomer [rel. %]** | | **Monomer [rel. %]** | |
| | | **Absolut** | **Delta** | **Absolut** | **Delta** |
| N/A | 0 | 99.0 | 0.0 | 99.1 | 0.0 |
| 4.0 | 4 | 98.1 | -0.9 | 98.3 | -0.8 |
| 25.0 | 4 | 96.1 | -2.9 | 96.2 | -2.9 |
| 40.0 | 2 | 83.1 | -15.9 | 82.0 | -17.1 |
| | 4 | 72.4 | -26.6 | 70.9 | -28.2 |

### 3.5. General methods used in the biophysical characterization of PRO2198 and PRO2199

### Buffer exchange

Buffer exchange was carried out by dialysis. The antibodies were dialyzed in Spectra Pro 3 dialysis membranes (Spectrum Laboratories) with at least a 200-fold excess of dialysis buffer.

### Concentrating of the antibodies

Antibodies were concentrated using centrifugal concentrators with a molecular weight cut-off (MWCO) of 10 kDa or 30 kDa. Samples were centrifuged in 5 min steps at 22°C until target concentration was reached. In between steps, the samples are resuspended.

### Determination of protein concentration

The concentration of the protein samples was determined using a Tecan plate reader and a NanoQuant Plate. The buffer was used as blank to be subtracted from the absorbance measured at 280 nm. Each measurement was corrected for scattering caused by visible particles determined at 310 nm. The corrected value was normalized to a path length of 1 cm and the protein concentration calculated using the theoretical extinction coefficient of the corresponding protein. For protein samples with a concentration higher than 10 mg/ml, the sample was diluted in the corresponding buffer at least 10 times or to 1 mg/ml nominal concentration.

### Storage

To assess the protein stability at different temperatures, samples were incubated at 4°C, 25°C and 40°C. 4°C storage was carried out in a fridge at a nominal temperature of 4°C. For storage at 25°C and 40°C samples were placed in cabinets with controlled humidity of 65 % rH and 75 % rH, respectively.

### Dynamic Light Scattering

Dynamic Light Scattering is used to determine the diffusion coefficient of molecules and particles in solution. It allows to calculate the hydrodynamic radius (Rh) of the molecule in solution as well as provides a sensitive method to detect the formation of higher order oligomers.

Within the high concentration stability study, Rh and the polydispersity were determined at the target concentration. This yielded information on self-association, oligomerization as well as potential increase in viscosity. Measurements were not corrected for buffer or sample viscosity, instead the viscosity of water was used to calculate the Rh of the samples.

### Thermal unfolding by nanoDSF

Thermal unfolding using TSA was obtained by the change in fluorescence intensity of Sypro Orange. The fluorescence of the dye is sensitive to hydrophobic interactions. As the protein unfolds, hydrophobic amino acid are exposed to the solvent leading to an increased fluorescence of Sypro Orange. The resulting midpoint of thermal unfolding (Tₘ) as well as the onset temperature calculated at 10 % of the maximal signal (Tₒₙₛₑₜ is the temperature at: min. signal + 0.1*(max. signal - min. signal)) was determined by fitting the data to a Boltzmann equation.

### Monomeric content by SE-HPLC

The monomeric content was determined by analytical size-exclusion chromatography using a Shodex KW403-4F column running in 50 mM sodium phosphate, 300 mM NaCl, pH 6.5. For analysis, 5 µg of sample were injected and the absorption recorded at 280 nm. The quality of the sample is stated as relative percentages of monomer, HMWS, and LMWS.

## Claims

1. An antibody variable domain, which specifically binds to IL-4R, comprising:
a) a VH sequence selected from SEQ ID NOs: 4, 5, 6, 7, 8 and 9, and
b) a VL sequence selected from SEQ ID NOs: 14, 15, 16, 17 and 18.

2. The antibody variable domain of any one of the preceding claims, wherein said antibody variable domain comprises:
a) a VH sequence of SEQ ID NO: 4 and a VL sequence of SEQ ID NO: 14; or
b) a VH sequence of SEQ ID NO: 4 and a VL sequence of SEQ ID NO: 17; or
c) a VH sequence of SEQ ID NO: 4 and a VL sequence of SEQ ID NO: 18; or
d) a VH sequence of SEQ ID NO: 5 and a VL sequence of SEQ ID NO: 15; or
e) a VH sequence of SEQ ID NO: 6 and a VL sequence of SEQ ID NO: 16; or
f) a VH sequence of SEQ ID NO: 7 and a VL sequence of SEQ ID NO: 14; or
g) a VH sequence of SEQ ID NO: 8 and a VL sequence of SEQ ID NO: 18; or
h) a VH sequence of SEQ ID NO: 9 and a VL sequence of SEQ ID NO: 14.

3. The antibody variable domain of claim 1 or 2, wherein said antibody variable domain is selected from a FAB, an Fv, an scFv and a dsFv.

4. The antibody variable domain of claim 3, which is selected from the scFv antibodies of SEQ ID NOs: 33 to 39.

5. A multispecific antibody comprising:
a) one or two antibody variable domains as defined in any one of claims 1 to 4;
b) at least one binding domain, which specifically binds to a target different from IL-4R.

6. The multispecific antibody of claim 5, wherein the multispecific antibody does not comprise an immunoglobulin Fc region.

7. The multispecific antibody of claim 6, wherein the multispecific antibody is in a format selected from the group consisting of: a tandem scDb (Tandab), a linear dimeric scDb (LD-scDb), a circular dimeric scDb (CD-scDb), a tandem tri-scFv, a tribody (Fab-(scFv)₂), a Fab-Fv₂, atriabody, an scDb-scFv, a tetrabody, a di-diabody, a tandem-di-scFv and a MATCH.

8. The multispecific antibody of claim 5, wherein the multispecific antibody comprises an immunoglobulin Fc region that is selected from IgG subclasses IgG1 and IgG4, particularly from IgG4.

9. The multispecific antibody of claim 8, wherein the format of said multispecific antibody is selected from KiH-based IgGs; DVD-Ig; CODV-IgG and Morrison (IgG CH₃-scFv fusion (Morrison H) or IgG CL-scFv fusion (Morrison L)), in particular from Morrison H and Morrison L.

10. The multispecific antibody of any one of claims 5 to 9, wherein said multispecific antibody comprises two antibody variable domains as defined in any one of claims 1 to 4 and two binding domains, which specifically bind to a second target different from IL-4R.

11. A nucleic acid sequence or two nucleic acid sequences encoding the antibody variable domain of any one of claims 1 to 4 or the multispecific antibody of any one of claims 5 to 10.

12. A vector or two vectors comprising the nucleic acid sequence or the two nucleic acid sequences of claim 11.

13. A host cell or host cells comprising the vector or the two vectors of claim 12.

14. A pharmaceutical composition comprising the multispecific antibody of any one of claims 5 to 10 and a pharmaceutically acceptable carrier.

15. The multispecific antibody of any one of claims 5 to 10 for use as a medicament.

16. The multispecific antibody of any one of claims 5 to 10 for use in the treatment of a disease, particularly a human disease, more particularly a human disease selected from allergic, inflammatory and autoimmune diseases, particularly from inflammatory and autoimmune diseases.


SEQUENCE LISTING

<110>  Numab Therapeutics AG

<120>  ANTIBODY VARIABLE DOMAINS THAT BIND IL-4R

<130>  117126P877EP

<160>  45

<170>  PatentIn version 3.5

<210>  1
<211>  11
<212>  PRT
<213>  Artificial sequence

<220>
<223>  artificial antibody-related sequence

<400>  1

Gly Phe Ser Ser Ser Ser Ala His Tyr Met Cys
1               5                   10

<210>  2
<211>  18
<212>  PRT
<213>  Artificial sequence

<220>
<223>  artificial antibody-related sequence

<400>  2

Cys Ile Tyr Ala Gly Ser Arg Gly Ser Thr Tyr Tyr Ala Ser Trp Ala
1               5                   10                  15

Lys Gly

<210>  3
<211>  19
<212>  PRT
<213>  Artificial sequence

<220>
<223>  artificial antibody-related sequence

<400>  3

Arg Ala Ala Phe Val Asn Arg Gly Val Ser Trp Ile Trp Pro Tyr Tyr
1               5                   10                  15

Phe Ser Leu

<210>  4

<211>  129
<212>  PRT
<213>  Artificial sequence

<220>
<223>  artificial antibody-related sequence

<400>  4

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Ser Ser Ser Ser Ala
            20                  25                  30

His Tyr Met Cys Trp Val Arg Gln Ala Pro Gly Lys Gly Leu Glu Trp
        35                  40                  45

Ile Gly Cys Ile Tyr Ala Gly Ser Arg Gly Ser Thr Tyr Tyr Ala Ser
    50                  55                  60

Trp Ala Lys Gly Arg Phe Thr Ile Ser Arg Asp Asn Ser Lys Asn Thr
65                  70                  75                  80

Val Tyr Leu Gln Met Asn Ser Leu Arg Ala Glu Asp Thr Ala Val Tyr
                85                  90                  95

Tyr Cys Ala Arg Ala Ala Phe Val Asn Arg Gly Val Ser Trp Ile Trp
            100                 105                 110

Pro Tyr Tyr Phe Ser Leu Trp Gly Gln Gly Thr Leu Val Thr Val Ser
        115                 120                 125

Ser

<210>  5
<211>  129
<212>  PRT
<213>  Artificial sequence

<220>
<223>  artificial antibody-related sequence

<400>  5

Glu Ser Gln Leu Val Glu Ser Gly Gly Gly Leu Val Gln Pro Gly Gly
1               5                   10                  15

Ser Leu Arg Leu Ser Cys Ala Ala Ser Gly Phe Ser Ser Ser Ser Ala
            20                  25                  30

His Tyr Met Cys Trp Val Arg Gln Ala Pro Gly Lys Gly Leu Glu Trp
        35                  40                  45

Ile Ala Cys Ile Tyr Ala Gly Ser Arg Gly Ser Thr Tyr Tyr Ala Ser
    50                  55                  60

Trp Ala Lys Gly Arg Phe Thr Ile Ser Lys Asp Asn Ser Lys Asn Thr
65                  70                  75                  80

Val Tyr Leu Gln Met Asn Ser Leu Arg Ala Glu Asp Thr Ala Val Tyr
                85                  90                  95

Phe Cys Ala Arg Ala Ala Phe Val Asn Arg Gly Val Ser Trp Ile Trp
            100                 105                 110

Pro Tyr Tyr Phe Ser Leu Trp Gly Gln Gly Thr Leu Val Thr Val Ser
        115                 120                 125

Ser

<210>  6
<211>  129
<212>  PRT
<213>  Artificial sequence

<220>
<223>  artificial antibody-related sequence

<400>  6

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Ser Ser Ser Ser Ala
            20                  25                  30

His Tyr Met Cys Trp Val Arg Gln Ala Pro Gly Lys Cys Leu Glu Trp
        35                  40                  45

Ile Gly Cys Ile Tyr Ala Gly Ser Arg Gly Ser Thr Tyr Tyr Ala Ser
    50                  55                  60

Trp Ala Lys Gly Arg Phe Thr Ile Ser Arg Asp Asn Ser Lys Asn Thr
65                  70                  75                  80

Val Tyr Leu Gln Met Asn Ser Leu Arg Ala Glu Asp Thr Ala Val Tyr
                85                  90                  95

Tyr Cys Ala Arg Ala Ala Phe Val Asn Arg Gly Val Ser Trp Ile Trp

            100                 105                 110

Pro Tyr Tyr Phe Ser Leu Trp Gly Gln Gly Thr Leu Val Thr Val Ser
        115                 120                 125

Ser

<210> 7
<211> 129
<212> PRT
<213> Artificial sequence

<220>
<223> artificial antibody-related sequence

<400> 7

Glu Val Gln Leu Val Glu Ser Gly Gly Gly Arg Val Gln Pro Gly Gly
1               5                   10                  15

Ser Leu Arg Leu Ser Cys Ala Ala Ser Gly Phe Ser Ser Ser Ser Ala
            20                  25                  30

His Tyr Met Cys Trp Val Arg Gln Ala Pro Gly Lys Gly Leu Glu Trp
        35                  40                  45

Ile Gly Cys Ile Tyr Ala Gly Ser Arg Gly Ser Thr Tyr Tyr Ala Ser
    50                  55                  60

Trp Ala Lys Gly Arg Phe Thr Ile Ser Arg Asp Asn Ser Lys Asn Thr
65                  70                  75                  80

Val Tyr Leu Gln Met Asn Ser Leu Arg Ala Glu Asp Thr Ala Thr Tyr
                85                  90                  95

Tyr Cys Ala Arg Ala Ala Phe Val Asn Arg Gly Val Ser Trp Ile Trp
            100                 105                 110

Pro Tyr Tyr Phe Ser Leu Trp Gly Gln Gly Thr Gln Val Thr Val Ser
        115                 120                 125

Ser

<210> 8
<211> 128
<212> PRT
<213> Artificial sequence

<220>

<223>  artificial antibody-related sequence

<400>  8

Glu Val Gln Leu Val Glu Ser Gly Gly Gly Arg Val Gln Pro Gly Gly
1               5                   10                  15

Ser Leu Arg Leu Ser Cys Ala Ala Ser Gly Phe Ser Ser Ser Ser Ala
            20                  25                  30

His Tyr Met Cys Trp Val Arg Gln Ala Pro Gly Lys Gly Leu Glu Trp
        35                  40                  45

Ile Gly Cys Ile Tyr Ala Gly Ser Arg Gly Ser Thr Tyr Tyr Ala Ser
    50                  55                  60

Trp Ala Lys Gly Arg Phe Thr Ile Ser Lys Ala Ser Ser Thr Thr Val
65                  70                  75                  80

Tyr Leu Gln Met Asn Ser Leu Arg Ala Glu Asp Thr Ala Thr Tyr Tyr
                85                  90                  95

Cys Ala Arg Ala Ala Phe Val Asn Arg Gly Val Ser Trp Ile Trp Pro
            100                 105                 110

Tyr Tyr Phe Ser Leu Trp Gly Gln Gly Thr Gln Val Thr Val Ser Ser
        115                 120                 125

<210>  9
<211>  128
<212>  PRT
<213>  Artificial sequence

<220>
<223>  artificial antibody-related sequence

<400>  9

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Ser Ser Ser Ser Ala
            20                  25                  30

His Tyr Met Cys Trp Val Arg Gln Ala Pro Gly Lys Gly Leu Glu Trp
        35                  40                  45

Ile Gly Cys Ile Tyr Ala Gly Ser Arg Gly Ser Thr Tyr Tyr Ala Ser
    50                  55                  60

Trp Ala Lys Gly Arg Phe Thr Ile Ser Lys Ala Ser Ser Thr Thr Val

65                  70                  75                  80

Tyr Leu Gln Met Asn Ser Leu Arg Ala Glu Asp Thr Ala Val Tyr Tyr
                85                  90                  95

Cys Ala Arg Ala Ala Phe Val Asn Arg Gly Val Ser Trp Ile Trp Pro
            100                 105                 110

Tyr Tyr Phe Ser Leu Trp Gly Gln Gly Thr Leu Val Thr Val Ser Ser
        115                 120                 125

<210>  10
<211>  11
<212>  PRT
<213>  Artificial sequence

<220>
<223>  artificial antibody-related sequence

<400>  10

Gln Ala Ser Glu Asp Ile Glu Ser Tyr Leu Ala
1               5                   10

<210>  11
<211>  7
<212>  PRT
<213>  Artificial sequence

<220>
<223>  artificial antibody-related sequence

<400>  11

Arg Ala Ser Thr Leu Ala Tyr
1               5

<210>  12
<211>  7
<212>  PRT
<213>  Artificial sequence

<220>
<223>  artificial antibody-related sequence

<400>  12

Arg Ala Ser Thr Leu Ala Ser
1               5

<210>  13
<211>  11
<212>  PRT
<213>  Artificial sequence

<220>

<223>  artificial antibody-related sequence

<400>  13

Leu Gly Ser Ser Tyr Ser Thr Gly Ser Asn Ile
1               5                   10


<210>  14
<211>  99
<212>  PRT
<213>  Artificial sequence

<220>
<223>  artificial antibody-related sequence

<400>  14

Asp Ile Gln Met Thr Gln Ser Pro Ser Ser Leu Ser Ala Ser Val Gly
1               5                   10                  15


Asp Arg Val Thr Ile Thr Cys Gln Ala Ser Glu Asp Ile Glu Ser Tyr
            20                  25                  30


Leu Ala Trp Tyr Gln Gln Lys Pro Gly Lys Ala Pro Lys Leu Leu Ile
        35                  40                  45


Tyr Arg Ala Ser Thr Leu Ala Tyr Gly Val Pro Ser Arg Phe Ser Gly
    50                  55                  60


Ser Gly Ser Gly Thr Asp Phe Thr Leu Thr Ile Ser Ser Leu Gln Pro
65                  70                  75                  80


Glu Asp Phe Ala Thr Tyr Tyr Cys Leu Gly Ser Ser Tyr Ser Thr Gly
                85                  90                  95


Ser Asn Ile




<210>  15
<211>  99
<212>  PRT
<213>  Artificial sequence

<220>
<223>  artificial antibody-related sequence

<400>  15

Asp Ile Gln Met Thr Gln Ser Pro Ser Ser Leu Ser Ala Ser Val Gly
1               5                   10                  15


Asp Arg Val Thr Ile Thr Cys Gln Ala Ser Glu Asp Ile Glu Ser Tyr
            20                  25                  30

Leu Ala Trp Tyr Gln Gln Lys Pro Gly Lys Pro Pro Lys Leu Leu Ile
        35                  40                  45

Tyr Arg Ala Ser Thr Leu Ala Tyr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Leu Gly Ser Ser Tyr Ser Thr Gly
                85                  90                  95

Ser Asn Ile

<210>  16
<211>  99
<212>  PRT
<213>  Artificial sequence

<220>
<223>  artificial antibody-related sequence

<400>  16

Asp Ile Gln Met Thr Gln Ser Pro Ser Ser Leu Ser Ala Ser Val Gly
1               5                   10                  15

Asp Arg Val Thr Ile Thr Cys Gln Ala Ser Glu Asp Ile Glu Ser Tyr
            20                  25                  30

Leu Ala Trp Tyr Gln Gln Lys Pro Gly Lys Ala Pro Lys Leu Leu Ile
        35                  40                  45

Tyr Arg Ala Ser Thr Leu Ala Tyr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Leu Gly Ser Ser Tyr Ser Thr Gly
                85                  90                  95

Ser Asn Ile

<210>  17
<211>  99
<212>  PRT
<213>  Artificial sequence

<220>
<223>  artificial antibody-related sequence

<400>  17

Asp Ile Gln Met Thr Gln Ser Pro Ser Ser Leu Ser Ala Ser Val Gly
1               5                   10                  15

Asp Arg Val Thr Ile Thr Cys Gln Ala Ser Glu Asp Ile Glu Ser Tyr
            20                  25                  30

Leu Ala Trp Tyr Gln Gln Lys Pro Gly Lys Ala Pro Lys Leu Leu Ile
        35                  40                  45

Tyr Arg Ala Ser Thr Leu Ala Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Leu Gly Ser Ser Tyr Ser Thr Gly
                85                  90                  95

Ser Asn Ile

<210>  18
<211>  99
<212>  PRT
<213>  Artificial sequence

<220>
<223>  artificial antibody-related sequence

<400>  18

Asp Ile Gln Met Thr Gln Ser Pro Ser Ser Leu Ser Ala Arg Val Gly
1               5                   10                  15

Asp Arg Val Thr Ile Thr Cys Gln Ala Ser Glu Asp Ile Glu Ser Tyr
            20                  25                  30

Leu Ala Trp Tyr Gln Gln Lys Pro Gly Lys Ala Pro Lys Leu Leu Ile
        35                  40                  45

Tyr Arg Ala Ser Thr Leu Ala Tyr Gly Val Pro Ser Arg Phe Ser Gly
    50                  55                  60

Ser Gly Ser Gly Arg Asp Phe Thr Leu Thr Ile Ser Ser Leu Gln Pro
65                  70                  75                  80

Glu Asp Phe Ala Thr Tyr Tyr Cys Leu Gly Ser Ser Tyr Ser Thr Gly
                85                  90                  95

Ser Asn Ile

<210>  19
<211>  122
<212>  PRT
<213>  Artificial sequence

<220>
<223>  artificial antibody-related sequence

<400>  19

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Ser Phe Ser Ala Asn
            20                  25                  30

Tyr Tyr Pro Cys Trp Val Arg Gln Ala Pro Gly Lys Gly Leu Glu Trp
        35                  40                  45

Ile Gly Cys Ile Tyr Gly Gly Ser Ser Asp Ile Thr Tyr Asp Ala Asn
    50                  55                  60

Trp Thr Lys Gly Arg Phe Thr Ile Ser Arg Asp Asn Ser Lys Asn Thr
65                  70                  75                  80

Val Tyr Leu Gln Met Asn Ser Leu Arg Ala Glu Asp Thr Ala Val Tyr
                85                  90                  95

Tyr Cys Ala Arg Ser Ala Trp Tyr Ser Gly Trp Gly Gly Asp Leu Trp
            100                 105                 110

Gly Gln Gly Thr Leu Val Thr Val Ser Ser
        115                 120

<210>  20
<211>  124
<212>  PRT
<213>  Artificial sequence

<220>
<223>  artificial antibody-related sequence

<400>  20

Gln Val Gln Leu Gln Glu Ser Gly Pro Gly Leu Val Lys Pro Ser Glu
1               5                   10                  15

Thr Leu Ser Leu Thr Cys Lys Val Ser Gly Phe Ser Phe Ser Asn Ser
            20                  25                  30

Tyr Trp Ile Cys Trp Ile Arg Gln Pro Pro Gly Lys Gly Leu Glu Trp
        35                  40                  45

Ile Gly Cys Thr Phe Val Gly Ser Ser Asp Ser Thr Tyr Tyr Ala Asn
    50                  55                  60

Trp Ala Lys Gly Arg Val Thr Ile Ser Val Asp Ser Ser Lys Asn Gln
65                  70                  75                  80

Phe Ser Leu Lys Leu Ser Ser Val Thr Ala Ala Asp Thr Ala Val Tyr
                85                  90                  95

Tyr Cys Ala Arg His Pro Ser Asp Ala Val Tyr Gly Tyr Ala Asn Asn
            100                 105                 110

Leu Trp Gly Gln Gly Thr Leu Val Thr Val Ser Ser
        115                 120

<210>  21
<211>  108
<212>  PRT
<213>  Artificial sequence

<220>
<223>  artificial antibody-related sequence

<400>  21

Asp Ile Gln Met Thr Gln Ser Pro Ser Ser Leu Ser Ala Ser Val Gly
1               5                   10                  15

Asp Arg Val Thr Ile Thr Cys Gln Ala Ser Gln Ser Ile Asn Asn Val
            20                  25                  30

Leu Ala Trp Tyr Gln Gln Lys Pro Gly Lys Ala Pro Lys Leu Leu Ile
        35                  40                  45

Tyr Arg Ala Ser Thr Leu Ala Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Ser Ser Tyr Gly Asn Tyr Gly
                85                  90                  95

Asp Phe Gly Thr Gly Thr Lys Val Thr Val Leu Gly
            100                 105

<210>  22
<211>  11
<212>  PRT
<213>  Artificial sequence

<220>
<223>  artificial antibody-related sequence

<400>  22

Phe Gly Thr Gly Thr Lys Val Thr Val Leu Gly
1               5                   10

<210>  23
<211>  11
<212>  PRT
<213>  Artificial sequence

<220>
<223>  artificial antibody-related sequence

<400>  23

Phe Gly Gly Gly Thr Lys Leu Thr Val Leu Gly
1               5                   10

<210>  24
<211>  11
<212>  PRT
<213>  Artificial sequence

<220>
<223>  artificial antibody-related sequence

<400>  24

Phe Gly Gly Gly Thr Gln Leu Ile Ile Leu Gly
1               5                   10

<210>  25
<211>  11
<212>  PRT
<213>  Artificial sequence

<220>
<223>  artificial antibody-related sequence

<400>  25

Phe Gly Glu Gly Thr Glu Leu Thr Val Leu Gly
1               5                   10

<210>  26
<211>  11

<212>  PRT
<213>  Artificial sequence

<220>
<223>  artificial antibody-related sequence

<400>  26

Phe Gly Ser Gly Thr Lys Val Thr Val Leu Gly
1               5                   10


<210>  27
<211>  11
<212>  PRT
<213>  Artificial sequence

<220>
<223>  artificial antibody-related sequence

<400>  27

Phe Gly Gly Gly Thr Gln Leu Thr Val Leu Gly
1               5                   10


<210>  28
<211>  11
<212>  PRT
<213>  Artificial sequence

<220>
<223>  artificial antibody-related sequence

<400>  28

Phe Gly Gly Gly Thr Gln Leu Thr Ala Leu Gly
1               5                   10


<210>  29
<211>  11
<212>  PRT
<213>  Artificial sequence

<220>
<223>  artificial antibody-related sequence

<400>  29

Phe Gly Cys Gly Thr Lys Val Thr Val Leu Gly
1               5                   10


<210>  30
<211>  20
<212>  PRT
<213>  Artificial sequence

<220>
<223>  artificial antibody-related sequence

<400>  30

Gly Gly Gly Gly Ser Gly Gly Gly Gly Ser Gly Gly Gly Gly Ser Gly
1               5                   10                  15

Gly Gly Gly Ser
            20

<210>  31
<211>  5
<212>  PRT
<213>  Artificial sequence

<220>
<223>  artificial antibody-related sequence

<400>  31

Gly Gly Gly Gly Ser
1               5

<210>  32
<211>  10
<212>  PRT
<213>  Artificial sequence

<220>
<223>  artificial antibody-related sequence

<400>  32

Gly Gly Gly Gly Ser Gly Gly Gly Gly Ser
1               5                   10

<210>  33
<211>  259
<212>  PRT
<213>  Artificial sequence

<220>
<223>  artificial antibody-related sequence

<400>  33

Asp Ile Gln Met Thr Gln Ser Pro Ser Ser Leu Ser Ala Ser Val Gly
1               5                   10                  15

Asp Arg Val Thr Ile Thr Cys Gln Ala Ser Glu Asp Ile Glu Ser Tyr
            20                  25                  30

Leu Ala Trp Tyr Gln Gln Lys Pro Gly Lys Ala Pro Lys Leu Leu Ile
        35                  40                  45

Tyr Arg Ala Ser Thr Leu Ala Tyr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Leu Gly Ser Ser Tyr Ser Thr Gly
                85                  90                  95

Ser Asn Ile Phe Gly Thr Gly Thr Lys Val Thr Val Leu Gly Gly Gly
            100                 105                 110

Gly Gly Ser Gly Gly Gly Gly Ser Gly Gly Gly Gly Ser Gly Gly Gly
        115                 120                 125

Gly Ser Glu Val Gln Leu Val Glu Ser Gly Gly Gly Leu Val Gln Pro
    130                 135                 140

Gly Gly Ser Leu Arg Leu Ser Cys Ala Ala Ser Gly Phe Ser Ser Ser
145                 150                 155                 160

Ser Ala His Tyr Met Cys Trp Val Arg Gln Ala Pro Gly Lys Gly Leu
                165                 170                 175

Glu Trp Ile Gly Cys Ile Tyr Ala Gly Ser Arg Gly Ser Thr Tyr Tyr
            180                 185                 190

Ala Ser Trp Ala Lys Gly Arg Phe Thr Ile Ser Arg Asp Asn Ser Lys
        195                 200                 205

Asn Thr Val Tyr Leu Gln Met Asn Ser Leu Arg Ala Glu Asp Thr Ala
    210                 215                 220

Val Tyr Tyr Cys Ala Arg Ala Ala Phe Val Asn Arg Gly Val Ser Trp
225                 230                 235                 240

Ile Trp Pro Tyr Tyr Phe Ser Leu Trp Gly Gln Gly Thr Leu Val Thr
                245                 250                 255

Val Ser Ser

<210>  34
<211>  259
<212>  PRT
<213>  Artificial sequence

<220>
<223>  artificial antibody-related sequence

<400>  34

Asp Ile Gln Met Thr Gln Ser Pro Ser Ser Leu Ser Ala Ser Val Gly

1               5                   10                  15

Asp Arg Val Thr Ile Thr Cys Gln Ala Ser Glu Asp Ile Glu Ser Tyr
            20                  25                  30

Leu Ala Trp Tyr Gln Gln Lys Pro Gly Lys Pro Pro Lys Leu Leu Ile
        35                  40                  45

Tyr Arg Ala Ser Thr Leu Ala Tyr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Leu Gly Ser Ser Tyr Ser Thr Gly
                85                  90                  95

Ser Asn Ile Phe Gly Thr Gly Thr Lys Val Thr Val Leu Gly Gly Gly
            100                 105                 110

Gly Gly Ser Gly Gly Gly Gly Ser Gly Gly Gly Gly Ser Gly Gly Gly
        115                 120                 125

Gly Ser Glu Ser Gln Leu Val Glu Ser Gly Gly Gly Leu Val Gln Pro
    130                 135                 140

Gly Gly Ser Leu Arg Leu Ser Cys Ala Ala Ser Gly Phe Ser Ser Ser
145                 150                 155                 160

Ser Ala His Tyr Met Cys Trp Val Arg Gln Ala Pro Gly Lys Gly Leu
                165                 170                 175

Glu Trp Ile Ala Cys Ile Tyr Ala Gly Ser Arg Gly Ser Thr Tyr Tyr
            180                 185                 190

Ala Ser Trp Ala Lys Gly Arg Phe Thr Ile Ser Lys Asp Asn Ser Lys
        195                 200                 205

Asn Thr Val Tyr Leu Gln Met Asn Ser Leu Arg Ala Glu Asp Thr Ala
    210                 215                 220

Val Tyr Phe Cys Ala Arg Ala Ala Phe Val Asn Arg Gly Val Ser Trp
225                 230                 235                 240

Ile Trp Pro Tyr Tyr Phe Ser Leu Trp Gly Gln Gly Thr Leu Val Thr
                245                 250                 255

Val Ser Ser

<210> 35
<211> 259
<212> PRT
<213> Artificial sequence

<220>
<223> artificial antibody-related sequence

<400> 35

Asp Ile Gln Met Thr Gln Ser Pro Ser Ser Leu Ser Ala Ser Val Gly
1               5                   10                  15

Asp Arg Val Thr Ile Thr Cys Gln Ala Ser Glu Asp Ile Glu Ser Tyr
            20                  25                  30

Leu Ala Trp Tyr Gln Gln Lys Pro Gly Lys Ala Pro Lys Leu Leu Ile
        35                  40                  45

Tyr Arg Ala Ser Thr Leu Ala Tyr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Leu Gly Ser Ser Tyr Ser Thr Gly
                85                  90                  95

Ser Asn Ile Phe Gly Cys Gly Thr Lys Val Thr Val Leu Gly Gly Gly
            100                 105                 110

Gly Gly Ser Gly Gly Gly Gly Ser Gly Gly Gly Gly Ser Gly Gly Gly
        115                 120                 125

Gly Ser Glu Val Gln Leu Val Glu Ser Gly Gly Gly Leu Val Gln Pro
    130                 135                 140

Gly Gly Ser Leu Arg Leu Ser Cys Ala Ala Ser Gly Phe Ser Ser Ser
145                 150                 155                 160

Ser Ala His Tyr Met Cys Trp Val Arg Gln Ala Pro Gly Lys Cys Leu
                165                 170                 175

Glu Trp Ile Gly Cys Ile Tyr Ala Gly Ser Arg Gly Ser Thr Tyr Tyr
            180                 185                 190

Ala Ser Trp Ala Lys Gly Arg Phe Thr Ile Ser Arg Asp Asn Ser Lys

195                 200                 205

Asn Thr Val Tyr Leu Gln Met Asn Ser Leu Arg Ala Glu Asp Thr Ala
    210                 215                 220

Val Tyr Tyr Cys Ala Arg Ala Ala Phe Val Asn Arg Gly Val Ser Trp
225                 230                 235                 240

Ile Trp Pro Tyr Tyr Phe Ser Leu Trp Gly Gln Gly Thr Leu Val Thr
                245                 250                 255

Val Ser Ser

<210>  36
<211>  259
<212>  PRT
<213>  Artificial sequence

<220>
<223>  artificial antibody-related sequence

<400>  36

Asp Ile Gln Met Thr Gln Ser Pro Ser Ser Leu Ser Ala Ser Val Gly
1               5                   10                  15

Asp Arg Val Thr Ile Thr Cys Gln Ala Ser Glu Asp Ile Glu Ser Tyr
            20                  25                  30

Leu Ala Trp Tyr Gln Gln Lys Pro Gly Lys Ala Pro Lys Leu Leu Ile
        35                  40                  45

Tyr Arg Ala Ser Thr Leu Ala Tyr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Leu Gly Ser Ser Tyr Ser Thr Gly
                85                  90                  95

Ser Asn Ile Phe Gly Thr Gly Thr Lys Val Thr Val Leu Gly Gly Gly
            100                 105                 110

Gly Gly Ser Gly Gly Gly Gly Ser Gly Gly Gly Gly Ser Gly Gly Gly
        115                 120                 125

Gly Ser Glu Val Gln Leu Val Glu Ser Gly Gly Gly Arg Val Gln Pro
    130                 135                 140

Gly Gly Ser Leu Arg Leu Ser Cys Ala Ala Ser Gly Phe Ser Ser Ser
145                 150                 155                 160

Ser Ala His Tyr Met Cys Trp Val Arg Gln Ala Pro Gly Lys Gly Leu
                165                 170                 175

Glu Trp Ile Gly Cys Ile Tyr Ala Gly Ser Arg Gly Ser Thr Tyr Tyr
            180                 185                 190

Ala Ser Trp Ala Lys Gly Arg Phe Thr Ile Ser Arg Asp Asn Ser Lys
        195                 200                 205

Asn Thr Val Tyr Leu Gln Met Asn Ser Leu Arg Ala Glu Asp Thr Ala
    210                 215                 220

Thr Tyr Tyr Cys Ala Arg Ala Ala Phe Val Asn Arg Gly Val Ser Trp
225                 230                 235                 240

Ile Trp Pro Tyr Tyr Phe Ser Leu Trp Gly Gln Gly Thr Gln Val Thr
                245                 250                 255

Val Ser Ser

<210>  37
<211>  259
<212>  PRT
<213>  Artificial sequence

<220>
<223>  artificial antibody-related sequence

<400>  37

Asp Ile Gln Met Thr Gln Ser Pro Ser Ser Leu Ser Ala Ser Val Gly
1               5                   10                  15

Asp Arg Val Thr Ile Thr Cys Gln Ala Ser Glu Asp Ile Glu Ser Tyr
            20                  25                  30

Leu Ala Trp Tyr Gln Gln Lys Pro Gly Lys Ala Pro Lys Leu Leu Ile
        35                  40                  45

Tyr Arg Ala Ser Thr Leu Ala Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Leu Gly Ser Ser Tyr Ser Thr Gly
                85                  90                  95

Ser Asn Ile Phe Gly Thr Gly Thr Lys Val Thr Val Leu Gly Gly Gly
            100                 105                 110

Gly Gly Ser Gly Gly Gly Gly Ser Gly Gly Gly Gly Ser Gly Gly Gly
        115                 120                 125

Gly Ser Glu Val Gln Leu Val Glu Ser Gly Gly Gly Leu Val Gln Pro
    130                 135                 140

Gly Gly Ser Leu Arg Leu Ser Cys Ala Ala Ser Gly Phe Ser Ser Ser
145                 150                 155                 160

Ser Ala His Tyr Met Cys Trp Val Arg Gln Ala Pro Gly Lys Gly Leu
                165                 170                 175

Glu Trp Ile Gly Cys Ile Tyr Ala Gly Ser Arg Gly Ser Thr Tyr Tyr
            180                 185                 190

Ala Ser Trp Ala Lys Gly Arg Phe Thr Ile Ser Arg Asp Asn Ser Lys
        195                 200                 205

Asn Thr Val Tyr Leu Gln Met Asn Ser Leu Arg Ala Glu Asp Thr Ala
    210                 215                 220

Val Tyr Tyr Cys Ala Arg Ala Ala Phe Val Asn Arg Gly Val Ser Trp
225                 230                 235                 240

Ile Trp Pro Tyr Tyr Phe Ser Leu Trp Gly Gln Gly Thr Leu Val Thr
                245                 250                 255

Val Ser Ser

<210>  38
<211>  259
<212>  PRT
<213>  Artificial sequence

<220>
<223>  artificial antibody-related sequence

<400>  38

Asp Ile Gln Met Thr Gln Ser Pro Ser Ser Leu Ser Ala Arg Val Gly
1               5                   10                  15

Asp Arg Val Thr Ile Thr Cys Gln Ala Ser Glu Asp Ile Glu Ser Tyr
            20                  25                  30

Leu Ala Trp Tyr Gln Gln Lys Pro Gly Lys Ala Pro Lys Leu Leu Ile
        35                  40                  45

Tyr Arg Ala Ser Thr Leu Ala Tyr Gly Val Pro Ser Arg Phe Ser Gly
    50                  55                  60

Ser Gly Ser Gly Arg Asp Phe Thr Leu Thr Ile Ser Ser Leu Gln Pro
65                  70                  75                  80

Glu Asp Phe Ala Thr Tyr Tyr Cys Leu Gly Ser Ser Tyr Ser Thr Gly
                85                  90                  95

Ser Asn Ile Phe Gly Thr Gly Thr Lys Val Thr Val Leu Gly Gly Gly
            100                 105                 110

Gly Gly Ser Gly Gly Gly Gly Ser Gly Gly Gly Gly Ser Gly Gly Gly
        115                 120                 125

Gly Ser Glu Val Gln Leu Val Glu Ser Gly Gly Gly Leu Val Gln Pro
    130                 135                 140

Gly Gly Ser Leu Arg Leu Ser Cys Ala Ala Ser Gly Phe Ser Ser Ser
145                 150                 155                 160

Ser Ala His Tyr Met Cys Trp Val Arg Gln Ala Pro Gly Lys Gly Leu
                165                 170                 175

Glu Trp Ile Gly Cys Ile Tyr Ala Gly Ser Arg Gly Ser Thr Tyr Tyr
            180                 185                 190

Ala Ser Trp Ala Lys Gly Arg Phe Thr Ile Ser Arg Asp Asn Ser Lys
        195                 200                 205

Asn Thr Val Tyr Leu Gln Met Asn Ser Leu Arg Ala Glu Asp Thr Ala
    210                 215                 220

Val Tyr Tyr Cys Ala Arg Ala Ala Phe Val Asn Arg Gly Val Ser Trp
225                 230                 235                 240

Ile Trp Pro Tyr Tyr Phe Ser Leu Trp Gly Gln Gly Thr Leu Val Thr
                245                 250                 255

Val Ser Ser

<210>  39
<211>  258
<212>  PRT
<213>  Artificial sequence

<220>
<223>  artificial antibody-related sequence

<400>  39

Asp Ile Gln Met Thr Gln Ser Pro Ser Ser Leu Ser Ala Ser Val Gly
1               5                   10                  15

Asp Arg Val Thr Ile Thr Cys Gln Ala Ser Glu Asp Ile Glu Ser Tyr
            20                  25                  30

Leu Ala Trp Tyr Gln Gln Lys Pro Gly Lys Ala Pro Lys Leu Leu Ile
        35                  40                  45

Tyr Arg Ala Ser Thr Leu Ala Tyr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Leu Gly Ser Ser Tyr Ser Thr Gly
                85                  90                  95

Ser Asn Ile Phe Gly Thr Gly Thr Lys Val Thr Val Leu Gly Gly Gly
            100                 105                 110

Gly Gly Ser Gly Gly Gly Gly Ser Gly Gly Gly Gly Ser Gly Gly Gly
        115                 120                 125

Gly Ser Glu Val Gln Leu Val Glu Ser Gly Gly Gly Arg Val Gln Pro
    130                 135                 140

Gly Gly Ser Leu Arg Leu Ser Cys Ala Ala Ser Gly Phe Ser Ser Ser
145                 150                 155                 160

Ser Ala His Tyr Met Cys Trp Val Arg Gln Ala Pro Gly Lys Gly Leu
                165                 170                 175

Glu Trp Ile Gly Cys Ile Tyr Ala Gly Ser Arg Gly Ser Thr Tyr Tyr
            180                 185                 190

Ala Ser Trp Ala Lys Gly Arg Phe Thr Ile Ser Lys Ala Ser Ser Thr
        195                 200                 205

Thr Val Tyr Leu Gln Met Asn Ser Leu Arg Ala Glu Asp Thr Ala Thr
    210                 215                 220

Tyr Tyr Cys Ala Arg Ala Ala Phe Val Asn Arg Gly Val Ser Trp Ile
225                 230                 235                 240

Trp Pro Tyr Tyr Phe Ser Leu Trp Gly Gln Gly Thr Gln Val Thr Val
                245                 250                 255

Ser Ser

<210>  40
<211>  216
<212>  PRT
<213>  Artificial sequence

<220>
<223>  artificial antibody-related sequence

<400>  40

Asp Ile Gln Met Thr Gln Ser Pro Ser Ser Leu Ser Ala Arg Val Gly
1               5                   10                  15

Asp Arg Val Thr Ile Thr Cys Gln Ala Ser Glu Asp Ile Glu Ser Tyr
            20                  25                  30

Leu Ala Trp Tyr Gln Gln Lys Pro Gly Lys Ala Pro Lys Leu Leu Ile
        35                  40                  45

Tyr Arg Ala Ser Thr Leu Ala Tyr Gly Val Pro Ser Arg Phe Ser Gly
    50                  55                  60

Ser Gly Ser Gly Arg Asp Phe Thr Leu Thr Ile Ser Ser Leu Gln Pro
65                  70                  75                  80

Glu Asp Phe Ala Thr Tyr Tyr Cys Leu Gly Ser Ser Tyr Ser Thr Gly
                85                  90                  95

Ser Asn Ile Phe Gly Gln Gly Thr Lys Val Glu Ile Lys Arg Thr Val
            100                 105                 110

Ala Ala Pro Ser Val Phe Ile Phe Pro Pro Ser Asp Glu Gln Leu Lys
        115                 120                 125

Ser Gly Thr Ala Ser Val Val Cys Leu Leu Asn Asn Phe Tyr Pro Arg
    130                 135                 140

Glu Ala Lys Val Gln Trp Lys Val Asp Asn Ala Leu Gln Ser Gly Asn

145                 150                 155                 160

Ser Gln Glu Ser Val Thr Glu Gln Asp Ser Lys Asp Ser Thr Tyr Ser
                165                 170                 175

Leu Ser Ser Thr Leu Thr Leu Ser Lys Ala Asp Tyr Glu Lys His Lys
            180                 185                 190

Val Tyr Ala Cys Glu Val Thr His Gln Gly Leu Ser Ser Pro Val Thr
        195                 200                 205

Lys Ser Phe Asn Arg Gly Glu Cys
    210                 215

<210>  41
<211>  720
<212>  PRT
<213>  Artificial sequence

<220>
<223>  artificial antibody-related sequence

<400>  41

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Ser Ser Ser Ser Ala
            20                  25                  30

His Tyr Met Cys Trp Val Arg Gln Ala Pro Gly Lys Gly Leu Glu Trp
        35                  40                  45

Ile Gly Cys Ile Tyr Ala Gly Ser Arg Gly Ser Thr Tyr Tyr Ala Ser
    50                  55                  60

Trp Ala Lys Gly Arg Phe Thr Ile Ser Arg Asp Asn Ser Lys Asn Thr
65                  70                  75                  80

Val Tyr Leu Gln Met Asn Ser Leu Arg Ala Glu Asp Thr Ala Val Tyr
                85                  90                  95

Tyr Cys Ala Arg Ala Ala Phe Val Asn Arg Gly Val Ser Trp Ile Trp
            100                 105                 110

Pro Tyr Tyr Phe Ser Leu Trp Gly Gln Gly Thr Leu Val Thr Val Ser
        115                 120                 125

Ser Ala Ser Thr Lys Gly Pro Ser Val Phe Pro Leu Ala Pro Cys Ser
    130                 135                 140

Arg Ser Thr Ser Glu Ser Thr Ala Ala Leu Gly Cys Leu Val Lys Asp
145                 150                 155                 160

Tyr Phe Pro Glu Pro Val Thr Val Ser Trp Asn Ser Gly Ala Leu Thr
                165                 170                 175

Ser Gly Val His Thr Phe Pro Ala Val Leu Gln Ser Ser Gly Leu Tyr
            180                 185                 190

Ser Leu Ser Ser Val Val Thr Val Pro Ser Ser Ser Leu Gly Thr Lys
        195                 200                 205

Thr Tyr Thr Cys Asn Val Asp His Lys Pro Ser Asn Thr Lys Val Asp
    210                 215                 220

Lys Arg Val Glu Ser Lys Tyr Gly Pro Pro Cys Pro Pro Cys Pro Ala
225                 230                 235                 240

Pro Glu Phe Leu Gly Gly Pro Ser Val Phe Leu Phe Pro Pro Lys Pro
                245                 250                 255

Lys Asp Thr Leu Met Ile Ser Arg Thr Pro Glu Val Thr Cys Val Val
            260                 265                 270

Val Asp Val Ser Gln Glu Asp Pro Glu Val Gln Phe Asn Trp Tyr Val
        275                 280                 285

Asp Gly Val Glu Val His Asn Ala Lys Thr Lys Pro Arg Glu Glu Gln
    290                 295                 300

Phe Asn Ser Thr Tyr Arg Val Val Ser Val Leu Thr Val Leu His Gln
305                 310                 315                 320

Asp Trp Leu Asn Gly Lys Glu Tyr Lys Cys Lys Val Ser Asn Lys Gly
                325                 330                 335

Leu Pro Ser Ser Ile Glu Lys Thr Ile Ser Lys Ala Lys Gly Gln Pro
            340                 345                 350

Arg Glu Pro Gln Val Tyr Thr Leu Pro Pro Ser Gln Glu Glu Met Thr
        355                 360                 365

Lys Asn Gln Val Ser Leu Thr Cys Leu Val Lys Gly Phe Tyr Pro Ser
    370                 375                 380

Asp Ile Ala Val Glu Trp Glu Ser Asn Gly Gln Pro Glu Asn Asn Tyr

385                 390                 395                 400

Lys Thr Thr Pro Pro Val Leu Asp Ser Asp Gly Ser Phe Phe Leu Tyr
                405                 410                 415

Ser Arg Leu Thr Val Asp Lys Ser Arg Trp Gln Glu Gly Asn Val Phe
            420                 425                 430

Ser Cys Ser Val Met His Glu Ala Leu His Asn His Tyr Thr Gln Lys
        435                 440                 445

Ser Leu Ser Leu Ser Leu Gly Gly Gly Gly Gly Ser Gly Gly Gly Gly
    450                 455                 460

Ser Ala Phe Gln Leu Thr Gln Ser Pro Ser Ser Leu Ser Ala Ser Val
465                 470                 475                 480

Gly Asp Arg Val Thr Ile Thr Cys Gln Ala Ser Gln Ser Ile Asp Ser
                485                 490                 495

Trp Leu Ala Trp Tyr Gln Gln Lys Pro Gly Lys Pro Pro Lys Leu Leu
            500                 505                 510

Ile Tyr Gln Ala Ser Lys Leu Ala Ser Gly Val Pro Ser Arg Phe Ser
        515                 520                 525

Gly Ser Gly Ser Gly Thr Asp Tyr Thr Leu Thr Ile Ser Ser Leu Gln
    530                 535                 540

Pro Glu Asp Phe Ala Thr Tyr Tyr Cys Gln Thr Tyr Tyr Gly Gly Gly
545                 550                 555                 560

His Ile Gly Trp Gly Phe Gly Thr Gly Thr Lys Val Thr Val Leu Gly
                565                 570                 575

Gly Gly Gly Gly Ser Gly Gly Gly Gly Ser Gly Gly Gly Gly Ser Gly
            580                 585                 590

Gly Gly Gly Ser Gln Ser Gln Leu Val Glu Ser Gly Gly Gly Arg Val
        595                 600                 605

Gln Pro Gly Gly Ser Leu Arg Leu Ser Cys Ala Ala Ser Gly Phe Ser
    610                 615                 620

Phe Ser Ala Asn Tyr Trp Ile Cys Trp Val Arg Gln Ala Pro Gly Lys
625                 630                 635                 640

Gly Leu Glu Trp Ile Val Cys Ile Tyr Ile Gly Ser Arg Ala Asp Thr
                645                 650                 655

Tyr Tyr Ala Pro Trp Ala Lys Gly Arg Phe Thr Ile Ser Lys Asp Asn
            660                 665                 670

Ser Lys Asn Thr Val Tyr Leu Gln Met Asn Ser Leu Arg Ala Glu Asp
        675                 680                 685

Thr Ala Thr Tyr Phe Cys Ala Arg Phe Ser Ser Gly Ile Tyr Asp Leu
    690                 695                 700

Asp Arg Phe Phe Leu Trp Gly Gln Gly Thr Gln Val Thr Val Ser Ser
705                 710                 715                 720

<210>  42
<211>  216
<212>  PRT
<213>  Artificial sequence

<220>
<223>  artificial antibody-related sequence

<400>  42

Asp Ile Gln Met Thr Gln Ser Pro Ser Ser Leu Ser Ala Ser Val Gly
1               5                   10                  15

Asp Arg Val Thr Ile Thr Cys Gln Ala Ser Glu Asp Ile Glu Ser Tyr
            20                  25                  30

Leu Ala Trp Tyr Gln Gln Lys Pro Gly Lys Ala Pro Lys Leu Leu Ile
        35                  40                  45

Tyr Arg Ala Ser Thr Leu Ala Tyr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Leu Gly Ser Ser Tyr Ser Thr Gly
                85                  90                  95

Ser Asn Ile Phe Gly Gln Gly Thr Lys Val Glu Ile Lys Arg Thr Val
            100                 105                 110

Ala Ala Pro Ser Val Phe Ile Phe Pro Pro Ser Asp Glu Gln Leu Lys
        115                 120                 125

Ser Gly Thr Ala Ser Val Val Cys Leu Leu Asn Asn Phe Tyr Pro Arg

130                 135                 140

Glu Ala Lys Val Gln Trp Lys Val Asp Asn Ala Leu Gln Ser Gly Asn
145                 150                 155                 160

Ser Gln Glu Ser Val Thr Glu Gln Asp Ser Lys Asp Ser Thr Tyr Ser
                165                 170                 175

Leu Ser Ser Thr Leu Thr Leu Ser Lys Ala Asp Tyr Glu Lys His Lys
            180                 185                 190

Val Tyr Ala Cys Glu Val Thr His Gln Gly Leu Ser Ser Pro Val Thr
        195                 200                 205

Lys Ser Phe Asn Arg Gly Glu Cys
    210                 215

<210>  43
<211>  719
<212>  PRT
<213>  Artificial sequence

<220>
<223>  artificial antibody-related sequence

<400>  43

Glu Val Gln Leu Val Glu Ser Gly Gly Gly Arg Val Gln Pro Gly Gly
1               5                   10                  15

Ser Leu Arg Leu Ser Cys Ala Ala Ser Gly Phe Ser Ser Ser Ser Ala
            20                  25                  30

His Tyr Met Cys Trp Val Arg Gln Ala Pro Gly Lys Gly Leu Glu Trp
        35                  40                  45

Ile Gly Cys Ile Tyr Ala Gly Ser Arg Gly Ser Thr Tyr Tyr Ala Ser
    50                  55                  60

Trp Ala Lys Gly Arg Phe Thr Ile Ser Lys Ala Ser Ser Thr Thr Val
65                  70                  75                  80

Tyr Leu Gln Met Asn Ser Leu Arg Ala Glu Asp Thr Ala Thr Tyr Tyr
                85                  90                  95

Cys Ala Arg Ala Ala Phe Val Asn Arg Gly Val Ser Trp Ile Trp Pro
            100                 105                 110

Tyr Tyr Phe Ser Leu Trp Gly Gln Gly Thr Gln Val Thr Val Ser Ser
        115                 120                 125

Ala Ser Thr Lys Gly Pro Ser Val Phe Pro Leu Ala Pro Cys Ser Arg
    130                 135                 140

Ser Thr Ser Glu Ser Thr Ala Ala Leu Gly Cys Leu Val Lys Asp Tyr
145                 150                 155                 160

Phe Pro Glu Pro Val Thr Val Ser Trp Asn Ser Gly Ala Leu Thr Ser
                165                 170                 175

Gly Val His Thr Phe Pro Ala Val Leu Gln Ser Ser Gly Leu Tyr Ser
            180                 185                 190

Leu Ser Ser Val Val Thr Val Pro Ser Ser Ser Leu Gly Thr Lys Thr
        195                 200                 205

Tyr Thr Cys Asn Val Asp His Lys Pro Ser Asn Thr Lys Val Asp Lys
    210                 215                 220

Arg Val Glu Ser Lys Tyr Gly Pro Pro Cys Pro Pro Cys Pro Ala Pro
225                 230                 235                 240

Glu Phe Leu Gly Gly Pro Ser Val Phe Leu Phe Pro Pro Lys Pro Lys
                245                 250                 255

Asp Thr Leu Met Ile Ser Arg Thr Pro Glu Val Thr Cys Val Val Val
            260                 265                 270

Asp Val Ser Gln Glu Asp Pro Glu Val Gln Phe Asn Trp Tyr Val Asp
        275                 280                 285

Gly Val Glu Val His Asn Ala Lys Thr Lys Pro Arg Glu Glu Gln Phe
    290                 295                 300

Asn Ser Thr Tyr Arg Val Val Ser Val Leu Thr Val Leu His Gln Asp
305                 310                 315                 320

Trp Leu Asn Gly Lys Glu Tyr Lys Cys Lys Val Ser Asn Lys Gly Leu
                325                 330                 335

Pro Ser Ser Ile Glu Lys Thr Ile Ser Lys Ala Lys Gly Gln Pro Arg
            340                 345                 350

Glu Pro Gln Val Tyr Thr Leu Pro Pro Ser Gln Glu Glu Met Thr Lys
        355                 360                 365

Asn Gln Val Ser Leu Thr Cys Leu Val Lys Gly Phe Tyr Pro Ser Asp

370                 375                 380

Ile Ala Val Glu Trp Glu Ser Asn Gly Gln Pro Glu Asn Asn Tyr Lys
385                 390                 395                 400

Thr Thr Pro Pro Val Leu Asp Ser Asp Gly Ser Phe Phe Leu Tyr Ser
                405                 410                 415

Arg Leu Thr Val Asp Lys Ser Arg Trp Gln Glu Gly Asn Val Phe Ser
            420                 425                 430

Cys Ser Val Met His Glu Ala Leu His Asn His Tyr Thr Gln Lys Ser
        435                 440                 445

Leu Ser Leu Ser Leu Gly Gly Gly Gly Gly Ser Gly Gly Gly Gly Ser
    450                 455                 460

Ala Phe Gln Leu Thr Gln Ser Pro Ser Ser Leu Ser Ala Ser Val Gly
465                 470                 475                 480

Asp Arg Val Thr Ile Thr Cys Gln Ala Ser Gln Ser Ile Asp Ser Trp
                485                 490                 495

Leu Ala Trp Tyr Gln Gln Lys Pro Gly Lys Pro Pro Lys Leu Leu Ile
            500                 505                 510

Tyr Gln Ala Ser Lys Leu Ala Ser Gly Val Pro Ser Arg Phe Ser Gly
        515                 520                 525

Ser Gly Ser Gly Thr Asp Tyr Thr Leu Thr Ile Ser Ser Leu Gln Pro
    530                 535                 540

Glu Asp Phe Ala Thr Tyr Tyr Cys Gln Thr Tyr Tyr Gly Gly Gly His
545                 550                 555                 560

Ile Gly Trp Gly Phe Gly Thr Gly Thr Lys Val Thr Val Leu Gly Gly
                565                 570                 575

Gly Gly Gly Ser Gly Gly Gly Gly Ser Gly Gly Gly Gly Ser Gly Gly
            580                 585                 590

Gly Gly Ser Gln Ser Gln Leu Val Glu Ser Gly Gly Gly Arg Val Gln
        595                 600                 605

Pro Gly Gly Ser Leu Arg Leu Ser Cys Ala Ala Ser Gly Phe Ser Phe
    610                 615                 620

Ser Ala Asn Tyr Trp Ile Cys Trp Val Arg Gln Ala Pro Gly Lys Gly
625                 630                 635                 640

Leu Glu Trp Ile Val Cys Ile Tyr Ile Gly Ser Arg Ala Asp Thr Tyr
                645                 650                 655

Tyr Ala Pro Trp Ala Lys Gly Arg Phe Thr Ile Ser Lys Asp Asn Ser
            660                 665                 670

Lys Asn Thr Val Tyr Leu Gln Met Asn Ser Leu Arg Ala Glu Asp Thr
        675                 680                 685

Ala Thr Tyr Phe Cys Ala Arg Phe Ser Ser Gly Ile Tyr Asp Leu Asp
    690                 695                 700

Arg Phe Phe Leu Trp Gly Gln Gly Thr Gln Val Thr Val Ser Ser
705                 710                 715

<210>  44
<211>  5
<212>  PRT
<213>  Artificial sequence

<220>
<223>  artificial antibody-related sequence

<400>  44

Gly Ser Gly Gly Ser
1               5

<210>  45
<211>  4
<212>  PRT
<213>  Artificial sequence

<220>
<223>  artificial antibody-related sequence

<400>  45

Gly Gly Gly Ser
1